# EUROPEAN PATENT APPLICATION

(11) **EP 4 435 060 A1**
(43) Date of publication of application: **25.09.2024**
(21) Application number: 23163708.3
(22) Date of filing: 23.03.2023
(51) Int. Cl.: C09B 47/18, C09B 47/30, A61P 35/00

(54) **SILICON PHTHALOCYANINE AND TRIBENZOPHTHALOCYANINE DYES AND THEIR USE IN DIAGNOSIS AND THERAPY**

(71) Applicant: Albert-Ludwigs-Universität Freiburg, 79098 Freiburg (DE)
(72) Inventor: WOLF, Philipp, 79350 Sexau (DE); BRÜCKNER, Reinhard, 79186 Bad Krozingen (DE); STORZ, Jonas, 79114 Freiburg (DE); WOLF, Isis, 79312 Emmendingen (DE)
(74) Representative: Kraus & Lederer PartGmbB

(57) **Abstract**

The present application relates to new A₃B-type silicon phthalocyanine including new silicon tribenzophthalocyanine dyes.

## Description

### Background of the invention

The present invention relates (1) to new A₃B-type silicon phthalocyanine dyes *including* new silicon tribenzophthalocyanine (from here "3:1 naphthalo-/phthalocyanine hybrid") dyes and (2) to derivatives of these dyes wherein they bind covalently to molecules (e.g. antibodies) forming bioconjugates that specifically bind non-covalently to a biological structure (e.g. an antigen). The A₃B-type silicon phthalocyanines including the silicon 3:1 naphthalo-/phthalocyanine hybrids, both as shown in formula I, according to the present invention can be advantageously used in diagnosis and therapy, in particular in targeted photodynamic therapy (from here: "TPDT") for which purpose a silicon phthalocyanine core or a 3:1 naphthalo-/phthalocyanine hybrid core is connected via a linker "L" to a reactive or activable group "Q" which may form a covalent bond to certain molecules, which in turn bind to a welldefined biological structure. The silicon phthalocyanine dyes including 3:1 naphthalo-/phthalocyanine hybrid dyes according to the invention (from here for simplicity's sake referred to as "silicon phthalocyanine dyes according to the present invention") absorb light (alone or when covalently bound to molecules, such as antibodies) from the red to the near-infrared (from here: "NIR") parts of the electromagnetic spectrum. This property may be used for diagnosis in order to indicate the location of the above-mentioned biological structure or for therapy since the energy of the red or NIR light is directed to the above-mentioned biological structures which may be destroyed thereby. The silicon phthalocyanine dyes according to the present invention are dyes, not pigments. They are water-soluble (because they contain multiple charges introduced via their twice occurring substituents OR), which is important since these compounds are used in a biological (aqueous) environment.

The activation of many kinds of dyes by light for destroying e.g. tumor cells involves, as one believes, the formation of oxygen in its singlet excited-state (from here "singlet oxygen") via the following steps:
1) Absorption of light exclusively by a singlet ground-state dye molecule; as consequence thereof an electron is promoted from an occupied to an unoccupied molecular orbital; the dye molecule thus assumes a singlet excited-state.
2) Spin inversion of one electron in such an excited dye molecule; this provides a longer-lived triplet excited-state dye molecule in a process known as intersystem crossing.
3) Energy transfer between such a triplet dye molecule and molecular oxygen - omnipresent in the aqueous solutions which make up biological systems - which has a triplet ground-state; this regenerates a singlet ground-state dye molecule (ready to re-participate in step 1) and singlet oxygen.
4) Other than triplet oxygen such singlet oxygen attacks constituents of the surrounding biological systems and eventually destroys the latter (see step 5). In the absence of dye(s) with the mentioned properties, (visible) light cannot convert triplet oxygen (cell-compatible) into singlet oxygen (cell-damaging). That insight led to the notion of dyes "sensitizing" triplet oxygen towards the uptake of energy from light; accordingly, a dye for photodynamic therapy (from here "PDT"), TPDT, or photoimmunotherapy (from here "PIT") is often referred to as a "photosensitizer" (from here: "PS").
5) Singlet oxygen can directly kill tumor cells by the induction of cell death within the light focus, including immunogenic cell death (ICD); it can cause destruction of tumor vasculature and it can produce an acute inflammatory response that induces a host immune response against released antigens from the dying tumor cells that amplifies the therapeutic effects. Such mechanisms result in the eradication of tumor cells even outside of the light focus, in particular circulating tumor cells or clusters of metastatic tumor cells.

An A₃B-type silicon phthalocyanine dye of the prior art which is deemed useful for binding covalently to molecules (e.g. antibodies) binding non-covalently to biological structures is disclosed in US Patent 7,005,518 as shown as structure Li-Cor-A below; it has an absorption maximum at λ = 680 nm (which corresponds to red light). The dye may be bound covalently to proteins, in particular to antibodies, by an aminolysis of an *N-*hydroxysuccinimide ester moiety. The silicon phthalocyanine dyes of the prior art claimed in this US Patent 7,005,518 have a silicon atom located in the center of the phthalocyanine core and thereby within an octahedral coordination sphere such that it binds to 4 nitrogen atoms equatorially and to 2 silyloxy (from here: "siloxy") groups axially. Furthermore, WO 2021/207691 discloses similar phthalocyanine dye compounds of the prior art as shown as structure **Rakuten-A** below; they were developed, inter alia, for deriving "therapeutic conjugates as the photosensitizing agents in light-based therapies including photoimmunotherapy":

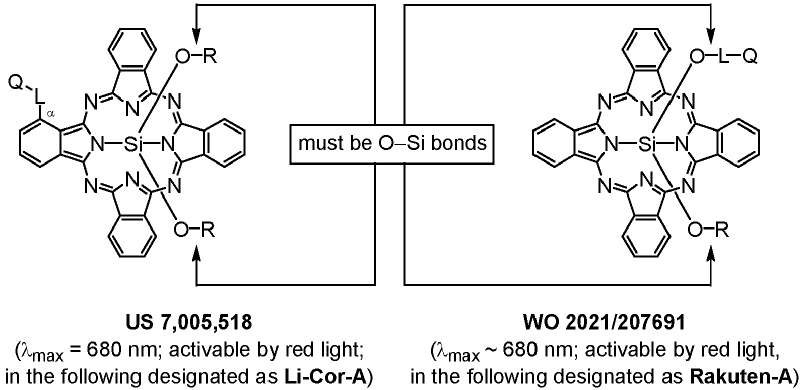

A₃B-type silicon phthalocyanine-type dyes of the present invention differ from structures like **Li-Cor-A** or analogs of **Rakuten-A** as disclosed in the prior art. The newly designed silicon phthalocyanine dyes according to the present invention have improved properties when used in diagnosis or therapy. The silicon phthalocyanine dyes according to the present invention can preferably be used in TPDT of tumors.

### Summary of the invention

The present invention relates to A₃B-type silicon phthalocyanines having any of structures **B** and ***iso*-B** in Formula I including 3:1 naphthalo-/phthalocyanine hybrid dyes as represented by structures **C** and ***iso*-C** in the same

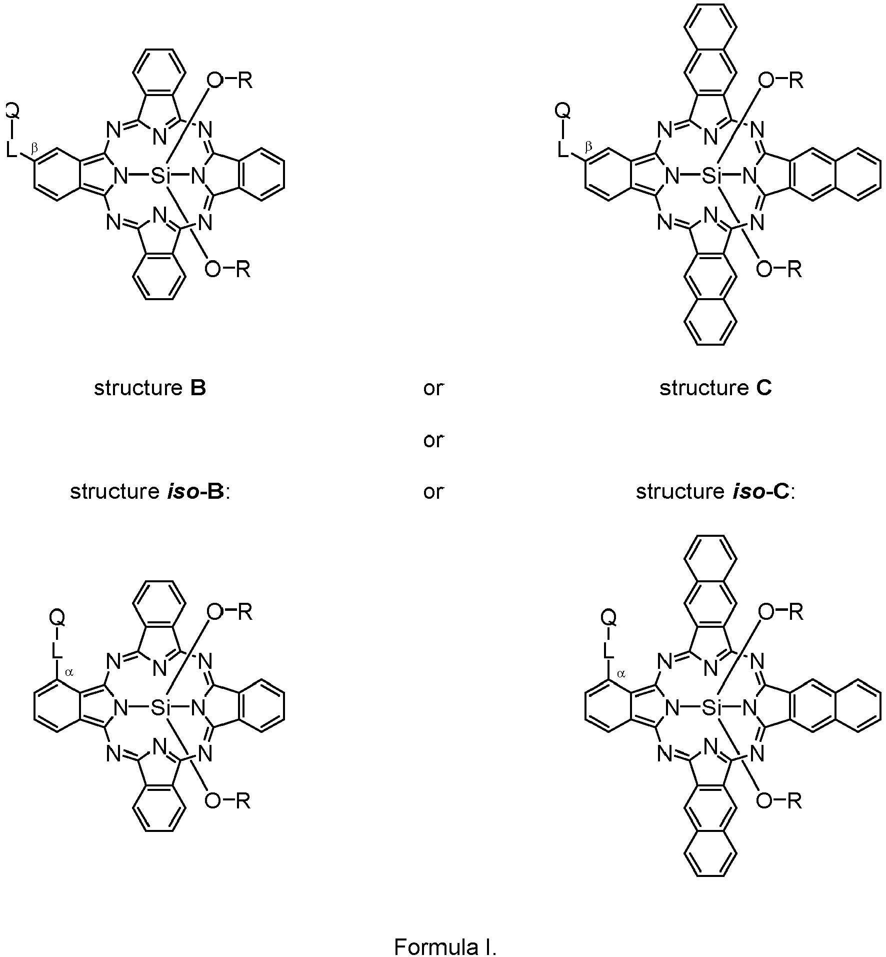

Therein:
-O-R is -O(-R¹)ₚ(-O)_{q}-R²(-R³)ₛ
R¹ is selected from C₁-C₃ alkyl and C(O),
p is 0 or 1,
q is 0 or 1, with the proviso that if p is 0, then q is 0,
R² is selected from alkyl, aryl, arylalkyl, (heteroaryl)alkyl, alkoxyalkyl, and heteroaryl,
R³ is -A¹-A²,
A¹ is selected from (heterocyclyl)alkyl-(O)ᵣ,
r is 0 or 1
A² is selected from di-, tri- and tetrasaccharides and -C₁-C₆ alkyl, which is substituted with at least one group selected from sulfonic acid, phosphonic acid, carboxylic acid, and salts thereof,
   and
s is 1, 2 or 3,
L is a linker unit -O-L¹-L² being bound via the oxygen either to C^{β} of structures **B and C** to form an ether motif C^{β}-O-L¹-L² or to C^{α} of structures ***iso*-B** and ***iso*-C** to form an ether motif C^{α}-O-L¹-L²,))
L¹ is selected from aryl, arylaryl, (arylalkyl)aryl, (arylalkoxy)aryl, [(arylalkyl)amino]aryl, aroylaryl, (arylsulfanyl)aryl, and arylsulfonylaryl
L² is selected from ureido(C₁-C₆ alkyl), ureido(C₂-C₆ alkenyl), ureido(C₂-C₆ alkynyl), carbamoyloxy(C₁-C₆ alkyl), carbamoyloxy(C₂-C₆ alkenyl), carbamoyloxy(C₂-C₆ alkynyl), (alkoxycarbonyl)amino(C₁-C₆ alkyl), (alkoxycarbonyl)amino(C₂-C₆ alkenyl), (alkoxycarbonyl)amino(C₂-C₆ alkynyl), [ureido(C₁-C₆ alkyl)]amino, [carbamoyloxy(C₁-C₆ alkyl)]amino, [(alkoxycarbonyl)amino(C₁-C₆ alkyl)]amino, ureido(C₁-C₆ alkoxy), carbamoyloxy(C₁-C₆ alkoxy), (alkoxycarbonyl)amino(C₁-C₆ alkoxy), [ureido(C₁-C₆ alkyl)]thio, [carbamoyloxy(C₁-C₆ alkyl)]thio, [(alkoxycarbonyl)amino(C₁-C₆ alkyl)]thio, {[ureido(C₁-C₆ alkyl)]amino}carbonyl, {[carbamoyloxy(C₁-C₆ alkyl)]amino}carbonyl, {[(alkoxycarbonyl)amino(C₁-C₆ alkyl)]amino}carbonyl, [ureido(C₁-C₆ alkoxy)]carbonyl, [carbamoyloxy(C₁-C₆ alkoxy)]carbonyl, [(alkoxycarbonyl)am ino(C₁-C₆ alkoxy)]carbonyl, [ureido(C₁-C₆ alkyl)]carbonyl, [ureido(C₂-C₆ alkenyl)]carbonyl, [ureido(C₂-C₆ alkynyl)]carbonyl, [carbamoyloxy(C₁-C₆ alkyl)]carbonyl, [carbamoyloxy(C₂-C₆ alkenyl)]carbonyl, [carbamoyloxy(C₂-C₆ alkynyl)]carbonyl, [(alkoxycarbonyl)amino(C₁-C₆ alkyl)]carbonyl, [(alkoxycarbonyl)amino(C₂-C₆ alkenyl)]carbonyl, and [(alkoxycarbonyl)amino(C₂-C₆ alkynyl)]carbonyl,
Q is -Q¹(-Q²)ₜ-Q³
Q¹ is a linear or branched, preferably linear, C₁-C₆ alkyl group,
Q² is selected from aryl and alkylaryl,
t is 0 or 1, and
Q³ is selected from the group consisting of
   a) a carboxylic acid, a corresponding acyl halide, a corresponding mixed anhydride, preferably aza-analogs resulting from additions to carbodiimides or condensations with uronium salts, a corresponding activated ester,
   b) an electrophilic C,C double bonds, preferably α,β-unsaturated esters, α,β-unsaturated lactones (including α-alkylidene lactones), α,β-unsaturated amides, α,β-unsaturated imides (including halogenated ones), α,β-unsaturated nitriles, α,β-unsaturated ketones, α,β-unsaturated sulfones, α,β-unsaturated sulfoxides, alk-1-enylphosphonates, nitroolefins,
   c) an alkyl halide, preferably α-haloketones and (haloacet)amides, an alkyl sulfonate,
   d) an isocyanate, isothiocyanate,
   e) a disulfide, a thiosulfonic acid ester, a trisulfide-S,S-dioxide,
   f) a phosphoramidite and
   g) an /V-aryl-1,2,4-triazolin-3,5-dione.

The silicon phthalocyanine dyes according to the present invention differ structurally from the A₃B-type silicon phthalocyanine dyes **Li-Cor-A** claimed in the US Patent 7,005,518 and particularly from the only dye of that kind whose synthesis is specifically described in this patent in various aspects. Most fundamentally by the silicon atom in the center of the molecules not binding axially to 2 siloxy groups but to 2 alkoxy groups, preferably to 2 arylalkoxy groups, or to 2 aryloxy groups or to 2 acyloxy groups or to 2 alkylcarbonato groups or 2 arylcarbonato groups. Bathochromic shifts of over 100 nm were achieved by another fundamental difference of the dye structures of the present invention compared to the structures **Li-Cor-A** claimed in US Patent 7,005,518, namely by extending the unsaturated core of the **B**-type silicon phthalocyanines to the unsaturated core of the C-type and ***iso*-C-**type silicon 3:1 naphthalo-/phthalocyanine hybrids. *Any* bathochromic shift of dyes for (T)PDT and PIT is beneficial because the light required for using dyes as PSs is known to penetrate the deeper into tissue the longer the absorption wavelength of the dye employed; as a consequence, e.g. more targets cells, can be affected by singlet oxygen in tissues than if this infrared shift was absent.

In another embodiment, the invention relates to the silicon phthalocyanines according to the present invention for use in therapy of tumor associated diseases.

In an embodiment, the invention relates to the silicon phthalocyanines according to the present invention for use in theranostics whereby the dye is activated by a light source in the red / near infrared wavelength for diagnosis and therapy.

In an embodiment, the invention relates to the silicon phthalocyanines according to the present invention for use in ex vivo diagnosis.

In an embodiment, the invention relates to the use of a compound of formula HO-L¹-Hal in the preparation of the silicon phthalocyanines according to the present invention, wherein L¹ is defined as in claim 1 and Hal is a halogen selected from I, Br, and Cl.

### Detailed description

The following definitions are relevant in connection with the embodiments of the present invention.

The prefix "C_{y}-Cₓ" as used herein refers to the number of carbon atoms of the respective group. For heteroaryl groups, the prefix "C_{y}-Cₓ" refers to the number of atoms (including heteroatoms) in the aromatic system.

As used herein, singular forms, such as "a", "an", and "the", include both singular and plural referents unless the context clearly dictates otherwise. By way of example, "silicon phthalocyanine" means one silicon phthalocyanine or more than one silicon phthalocyanine.

The term "silicon phthalocyanine dyes according to the present invention" or "silicon phthalocyanines according to the present invention" refers to silicon phthalocyanine dyes as represented by structures **B and *iso*-B** in Formula I including 3:1 naphthalo-/phthalocyanine hybrid dyes as represented by structures **C** and ***iso*-C** in Formula I.

The term "aryl" as used herein by itself or as part of another group refers to a monocyclic, bicyclic or tricyclic aromatic ring system having from six to fourteen carbon atoms (i.e., C₆-C₁₄ aryl). Non-limiting exemplary aryl groups include phenyl (abbreviated as "Ph"), naphthyl, phenanthryl, anthracenyl, indenyl, biphenylyl including fluorenyl. The term "aryl" as used herein encompasses phenyls, naphthyls, phenanthryls, anthracenyls, and biphenylyls including fluorenyls that are substituted by one or more substituents, e.g. halo, nitro, cyano, hydroxy, alkoxy, and alkyl.

The term "alkyl" as used herein by itself or as part of another group refers to a straight- or branched-chain aliphatic hydrocarbon containing one to twelve carbon atoms (i.e., C₁-C₁₂ alkyl).

In one embodiment, the alkyl group is chosen from a straight chain C₁-C₁₀ alkyl group. In another embodiment, the alkyl group is chosen from a branched chain C₃-C₁₀ alkyl group. In another embodiment, the alkyl group is chosen from a straight chain C₁-C₆ alkyl group. In another embodiment, the alkyl group is chosen from a branched chain C₃-C₆ alkyl group. In another embodiment, the alkyl group is chosen from a straight chain C₁-C₄ alkyl group. In another embodiment, the alkyl group is chosen from a branched chain C₃-C₄ alkyl group. In another embodiment, the alkyl group is chosen from a straight or branched chain C₃-C₄ alkyl group. Non-limiting exemplary C₁-C₁₀ alkyl groups include methyl, ethyl, propyl, isopropyl, butyl, sec-butyl, tert-butyl, iso-butyl, 3-pentyl, hexyl, heptyl, octyl, nonyl, decyl, and the like. Non-limiting exemplary C₁-C₄ alkyl groups include methyl, ethyl, propyl, isopropyl, butyl, sec-butyl, tert-butyl, and iso-butyl.

The skilled person will understand that the term "alkyl" as used herein also encompasses "alkylene", including as non-limiting examples e.g. methylene (-CH₂-), ethylene (-CH₂CH₂-), or propylene (-CH₂CH₂CH₂-), when the alkyl group is not a terminal group, but is part of a chain, such as e.g. R¹ and R² in -O(-R¹)ₚ(-O)_{q}-R²(-R³)ₛ. The same considerations also apply to other groups or substituents as defined in the present application when being part of a chain, and not a terminal group. For example, the term (hetero)aryl also encompasses "(hetero)arylene".

Furthermore, the term "alkyl" also includes branched alkyls such as "alkan-1,ω-diyl", "alkan-1,ω,ω-triyl" and "alkan-1,ω,ω,ω -tetrayl", e.g. when -R²(-R³)ₛ, R² is "alkyl" and s is 1, 2 or 3.

The term "heteroaryl" as used herein by itself or as part of another group refers to a monocyclic or bicyclic heteroaromatic ring system having from five to ten atoms (including 1, 2, 3, or 4 heteroatoms independently chosen from oxygen, nitrogen and sulfur, i.e. C₅-C₁₀ heteroaryl). In one embodiment, the heteroaryl is a C₅ heteroaryl. In another embodiment, the heteroaryl is a C₆ heteroaryl. Non-limiting exemplary heteroaryl groups include but are not limited to furyl, pyrrolyl, pyrazolyl, imidazolyl, triazolyl, oxazolyl, thienyl, pyridyl, pyridazinyl, pyrimidinyl, pyrazinyl, 1,2,3-triazinyl, 1,2,4,5-tetrazinyl, benzofuryl, indolyl, benzothienyl, quinolinyl, isoquinolinyl, cinnolinyl, quinaziolinyl, quinoxalinyl, and phthalazinyl. The term "heteroaryl" as used herein encompasses heteroaryls that are substituted by one or more substituents, e.g. halo, nitro, cyano, hydroxy, and alkyl.

The term "alkenyl" as used herein by itself or as part of another group refers to an alkyl group as defined above containing one, two or three carbon-to-carbon double bonds. In one embodiment, the alkenyl group is chosen from a C₂-C₆ alkenyl group having 2, 3, 4, 5 or 6 carbon atoms. In another embodiment, the alkenyl group is chosen from a C₂-C₄ alkenyl group having 2, 3, or 4 carbon atoms. Non-limiting exemplary alkenyl groups include ethenyl, propenyl, isopropenyl, butenyl, sec-butenyl, pentenyl, and hexenyl.

The term "alkynyl" as used herein by itself or as part of another group refers to an alkyl group as defined above containing one to three carbon-to-carbon triple bonds. In one embodiment, the alkynyl has one carbon-to-carbon triple bond. In one embodiment, the alkynyl group is chosen from a C₂-C₆ alkynyl group having 2, 3, 4, 5 or 6 carbon atoms. In another embodiment, the alkynyl group is chosen from a C₂-C₄ alkynyl group having 2, 3 or 4 carbon atoms. Non-limiting exemplary alkynyl groups include ethynyl, propynyl, butynyl, 2-butynyl, pentynyl, and hexynyl groups.

The term "alkoxy" as used herein by itself or as part of another group refers to an optionally substituted alkyl, optionally substituted alkenyl or optionally substituted alkynyl attached to a terminal oxygen atom. In one embodiment, the alkoxy group is chosen from a C₁-C₆ alkoxy group. In another embodiment, the alkoxy group is chosen from a C₁-C₄ alkyl attached to a terminal oxygen atom, e.g., methoxy, ethoxy, tert-butoxy, pentoxy and hexoxy. It is preferred that the term "alkoxy" refers to a linear C₁-C₆ alkyl group attached to a terminal oxygen atom.

The term "arylalkoxy" as used by itself or as part of another group refers to an aralkyl group attached to a terminal oxygen atom. A non-limiting exemplary arylalkoxy group is PhCH₂O-.

The term "heterocyclyl" as used by itself or as part of another group refers to saturated and partially unsaturated (e.g., containing one or two double bonds) cyclic groups containing one, two, or three rings having from three to fourteen ring members (i.e., a 3-, 4-, 5-, 6-, 7-, 8-, 9-, 10-, 11-, 12-, 13- or 14-membered heterocyclyl) and at least one heteroatom. Each heteroatom is independently selected from the group consisting of oxygen, sulfur, including sulfoxide and sulfone, and/or nitrogen atoms. The term "heterocyclyl" encompasses heteroaryl groups. The heterocycle may be unsubstituted or optionally substituted with one to four substituents independently selected from halo, nitro, cyano, hydroxy, amino, alkoxy, alkyl, alkenyl, alkynyl, aryl, heteroaryl, heterocyclyl, alkoxyalkyl, and (heteroaryl)alkyl. Substitution may occur on any available carbon or nitrogen atom, and may form a spirocycle. Non-limiting exemplary heterocyclyl groups include [1,2,3]triazolyl (D), tetrahydrocycloocta[1,2,3]triazolyl (E), 3,4,5-triazatricyclo[5.2.1.0]decyl (F), 3,4,5-triazatricyclo[5.2.1.0]dec-3-enyl (G), and 1,6-dihydropyridazinyl (H).

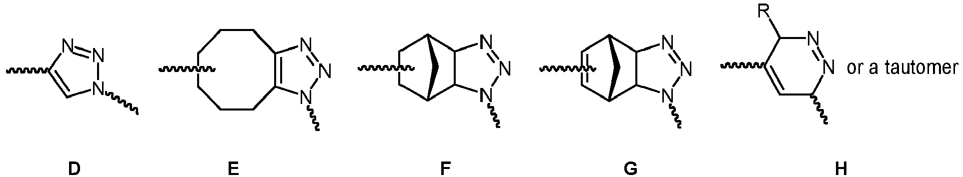

The term "alkylcarbonato" as used herein by itself or as part of another group refers to a carbonato group (-O-C(=O)-O-) that is linked to an alkyl group. Non-limiting examples of alkylcarbonato include methylcarbonato (-O-C(=O)-O-CH₃). Likewise, the term "arylcarbonato" as used herein by itself or as part of another group refers to a carbonato group (-O-C(=O)-O-) that is linked to an aryl group. Non-limiting examples of arylcarbonato include phenylcarbonato (-O-C(=O)-O-C₆H₅).

The term "arylalkyl" as used herein by itself or as part of another group refers to an alkyl group substituted with one, two, or three, preferably one, aryl groups. In one embodiment, the arylalkyl group is a C1-C4 alkyl substituted with one optionally substituted aryl group. Non-limiting exemplary aralkyl groups include benzyl and phenethyl.

The term "ureido" as used herein by itself or as part of another group refers to a radical of the formula -NH-C(=O)-NH₂.

The term "carbamoyloxy" as used herein by itself or as part of another group refers to a radical of the formula -O-C(=O)-NH₂.

The term "alkoxycarbonyl)amino" as used herein by itself or as part of another group refers to a radical of the formula -NH-C(=O)-O-alkyl.

The term "ureido(C₁-C₆ alkyl)" as used herein refers to a group of formula -C₁-C₆ alkyl-NH-C(=O)-NH₂. The term "ureido(C₂-C₆ alkenyl)" as used herein refers to a group of formula -C₂-C₆ alkenyl-NH-C(=O)-NH₂. The term "ureido(C₂-C₆ alkynyl)" as used herein refers to a group of formula -C₂-C₆ alkynyl-NH-C(=O)-NH₂.

The term "carbamoyloxy(C₁-C₆ alkyl)" as used herein refers to a group of formula -C₁-C₆ alkyl-O-C(=O)-NH₂. The term "carbamoyloxy(C₂-C₆ alkenyl)" as used herein refers to a group of formula -C₂-C₆ alkenyl-O-C(=O)-NH₂. The term "carbamoyloxy(C₂-C₆ alkynyl)" as used herein refers to a group of formula -C₂-C₆ alkynyl-O-C(=O)-NH₂.

The term "(alkoxycarbonyl)amino(C₁-C₆ alkyl)" as used herein by itself or as part of another group refers to a group of formula -(C₁-C₆ alkyl)-NH-C(=O)-O-alkyl. The term "(alkoxycarbonyl)amino(C₂-C₆ alkenyl)" as used herein by itself or as part of another group refers to a group of formula -(C₂-C₆ alkenyl)-NH-C(=O)-O-alkyl. The term "(alkoxycarbonyl)amino(C₂-C₆ alkynyl)" as used herein by itself or as part of another group refers to a group of formula -(C₂-C₆ alkynyl)-NH-C(=O)-O-alkyl.

The term "salt" used herein refers to but is not limited to, alkaline metal salts such as a sodium salt, potassium salt, cesium salt; alkaline earth metals such as a magnesium salt, calcium salt, and ammonium salts.

Composite terms as used herein, such as "heteroarylalkoxy", are to be understood in their broadest technically sensible way and may refer to a connectivity of -heteroarylalkoxy or heteroarylalkoxy-. However, it is preferred that composite terms are interpreted in that the last-mentioned group is connected "first", e.g. -O-alkyl-heteroaryl. In case the composite term has more than one bond/connection, it is preferred that the last-mentioned group is connected first and the first mentioned group connects the group to the next substituent. For example, for -O-L¹-L²-Q and L² being ureido(C₂-C₆ alkynyl), it is preferred that the connectivity is -O-L¹-(C₂-C₆ alkynyl)-NH-C(=O)-NH-Q.

The term "aroyl" as used herein by itself or as part of another group refers to a carbonyl group substituted with one aryl group. Non limiting exemplary aroyl groups include -C(=O)-C₆H₅.

The term "arylsulfonyl" as used herein by itself or as part of another group refers to a sulfonyl (SO₂) group substituted with one aryl group. Non limiting exemplary arylsulfonyl groups include -SO₂-C₆H₅.

Non-limiting examples for "di-, tri- or tetrasaccharides" as used herein are maltose, cellobiose, isomaltose, gentiobiose, mannobiose, xylobiose, lactose, maltotriose, panose, 2'-fucosyllactose, 3-fucosyllactose, and maltotetraose.

Preferred embodiments according to the invention are defined hereinafter. The preferred embodiments are preferred alone or in combination. Further, it is to be understood that the following preferred embodiments refer to all aspects of the present invention, i.e. the silicon phthalocyanine, silicon phthalocyanine for use, and the use of a compound of formula HO-L¹-Hal.

The naphthalo-/phthalocyanine hybrid dyes as represented by structures C and iso-C in Formula I are preferred. It has been found that these dyes as represented by structures C and ***iso*-C** in Formula I can be prepared, in particular, purified more easily than the silicon phthalocyanine dyes of the prior art and even the silicon phthalocyanine dyes as represented by structures **B** and ***iso*-B** in Formula I.

In a preferred embodiment, p is 1 and R¹ is C₁-C₃ alkyl. In a preferred embodiment, q is 0.

In a preferred embodiment, R² is selected from a linear or branched, more preferably linear, C₁-C₆ alkyl group, phenyl, naphthyl, phenanthryl, anthracyl, biphenylyl, pyridyl, pyrimidinyl, and pyridazinyl. In a more preferred embodiment, R² is selected from C₁-C₆ alkyl, phenyl, and pyridyl.

In case s is 2 or 3, each R³ may be selected independently. It is, however, preferred that each R³ is identical.

In a preferred embodiment, A' is ([1,2,3]triazolyl)alkoxy, tetrahydrocycloocta[1,2,3]triazolyl)alkoxy, (3,4,5-triazatricyclo[5.2.1.0]decyl)alkoxy, (3,4,5-triazatricyclo[5.2.1.0]dec-3-enyl)alkoxy or 1,6-dihydropyridazinyl)alkoxy.

In a preferred embodiment, A² is a C₁-C₆ alkyl substituted with a group from sulfonic acid, phosphonic acid, and salts thereof.

The linker unit L (-O-L¹-L²) is described below.
L = 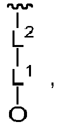 which equals 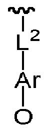 and wherein 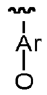 is preferably any one of

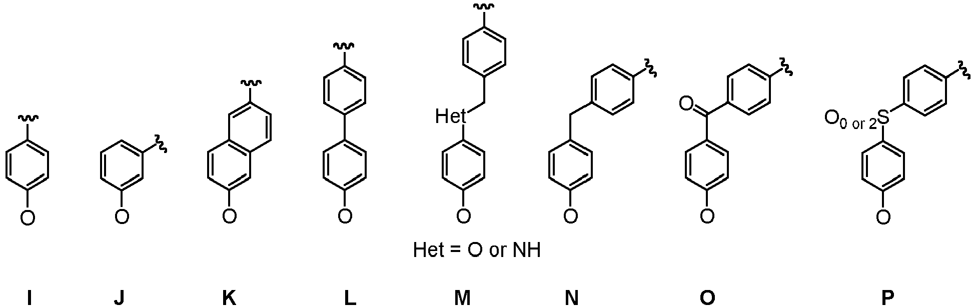

In a preferred embodiment, L² has the following structure.
L = 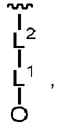 which equals 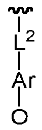 and wherein 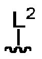 is preferably any one of 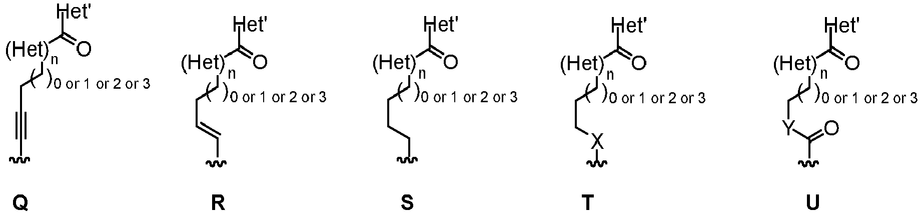 , wherein n is 1 or 0,
X is selected from aryl, NH and O,
Y is selected from alkyl, aryl, NH, N_{without H} and O, and
Het and Het' are independently selected from NH, O and S.

It is particularly preferred that
Het is NH and Het' is NH,
Het is O and Het' is NH, or
Het is NH and Het' is O.

In a preferred embodiment, L¹ is aryl, more preferably phenyl or naphthyl.

In a preferred embodiment, L² is ureido(C₂-C₆ alkynyl), carbamoyloxy(C₂-C₆ alkynyl) or (alkoxycarbonyl)amino(C₂-C₆ alkynyl).

In an a particularly preferred embodiment, the linker L has a structure selected from the group consisting of any one of

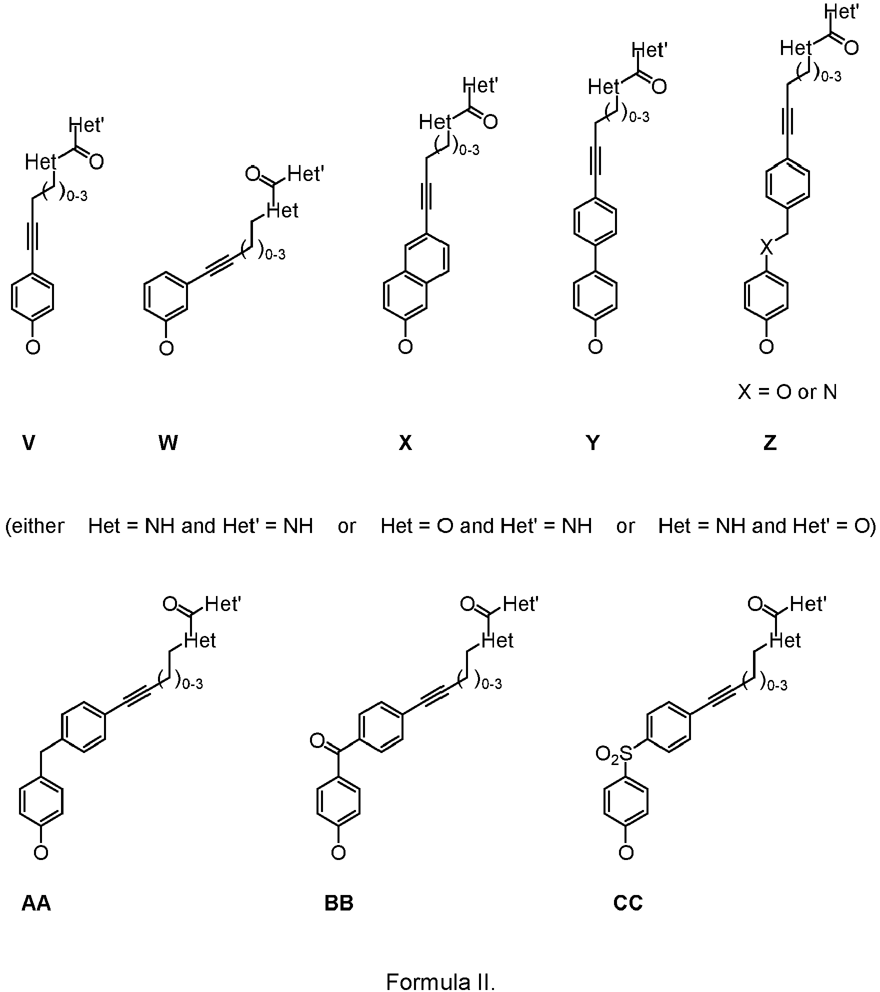

Q is a reactive or an activable group which can form a covalent bond to a molecule specifically, whereupon the resulting entity, a bioconjugate, binds non-covalently to a biological structure.

In particularly preferred embodiments the linker L is one of the (alk-1-ynylaryl)oxy structures DD or EE of

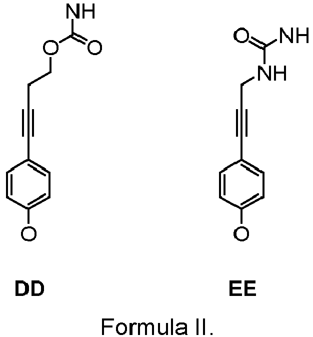

The structure of Q -is -Q¹(-Q²)ₜ-Q³ is depicted below.

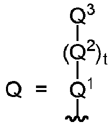

In a preferred embodiment 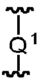 is selected from the following structure:

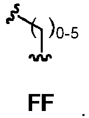

In a preferred embodiment 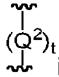 is selected from the following structure:

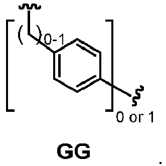

In a preferred embodiment, 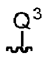 is selected from any one of the following structures:

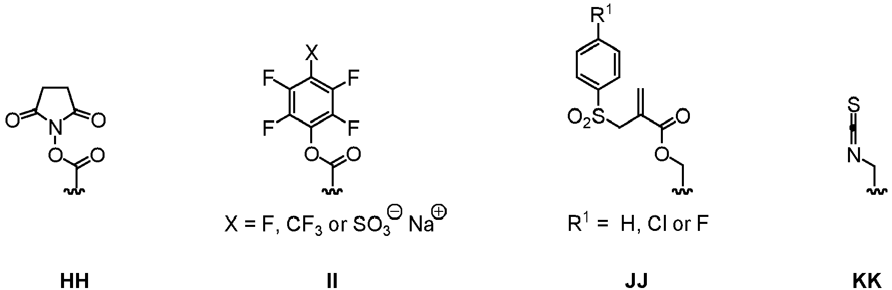

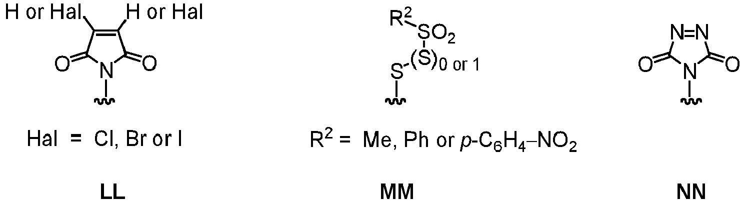

In a preferred embodiment, accordingly, Q is selected from any one of the following structures:

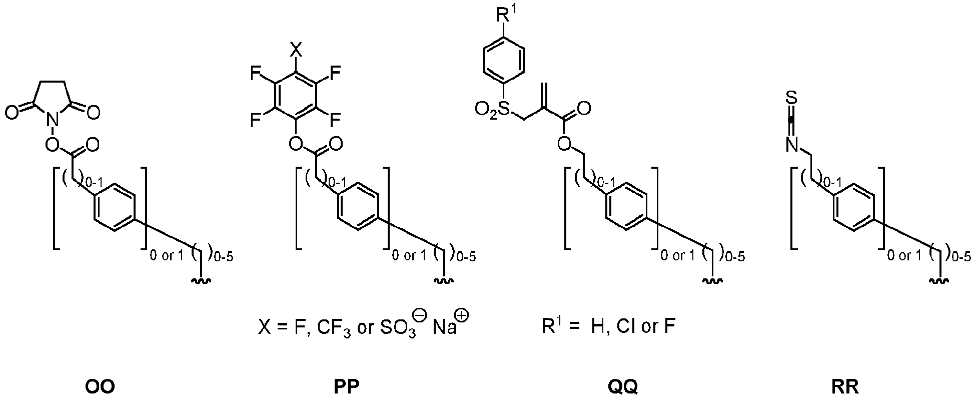

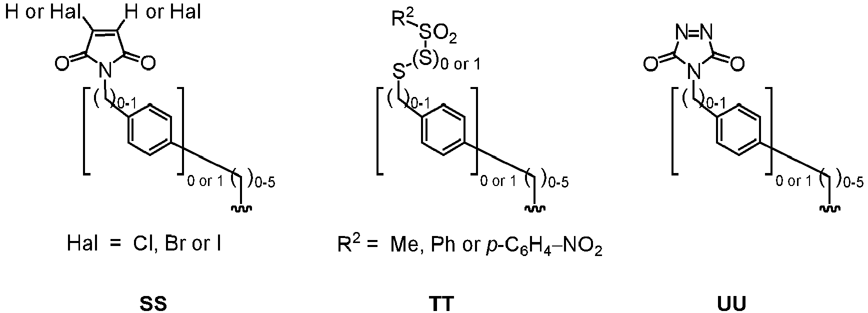

In a more preferred embodiment, Q is selected from any one of the structures **W**, **WW**, **XX** and **YY** of 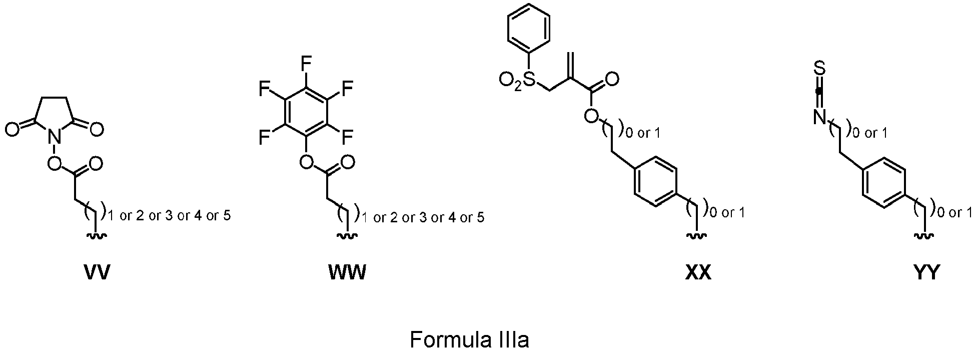 or from any one of the structures **ZZ**, **AAA**, **BBB** and **CCC** of

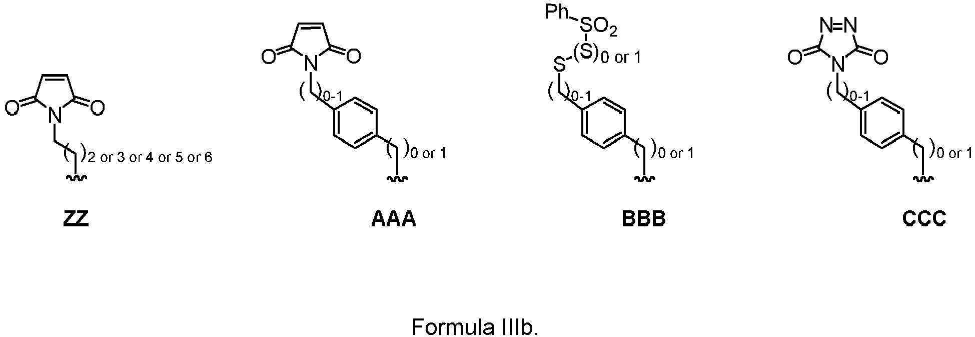

In a particularly preferred embodiment, Q is selected from any of the structures **DDD**, **EEE** and **FFF** of 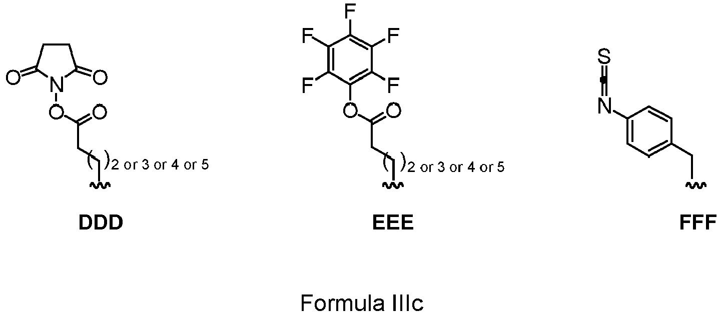 or from any one of the structures **GGG, HHH** and **III** of

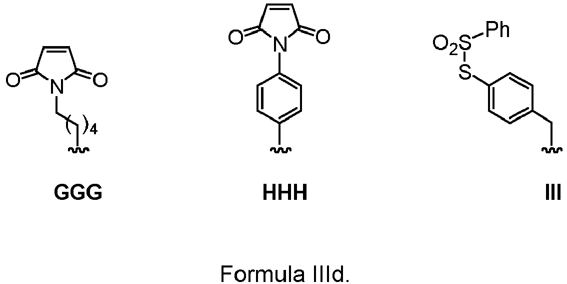

In the silicon phthalocyanine of the present invention, the residue Q according to Formula III is designed such as to form a covalent bond specifically or designed to be activable to form a covalent bond specifically to molecules binding non-covalently to biological structures. The mentioned molecules are preferably selected from but not limited to the group of antibodies, antibody fragments, nanobodies, antigens, ligands, proteins, peptides, small molecules, inhibitors, aptamers, cells, phages, viruses, RNA, DNA, lipids, carbohydrates, polysaccharides, nanomaterials or chemical drugs.

An example of a preferred molecule-dye conjugate comprises therefore the following elements: a silicon phthalocyanine dye of the present invention which is conjugated (i.e. covalently bound) to a molecule (e.g. antibody) which can bind to a biological structure (e.g. antigen). Another example of such specific biological interaction is the binding of a ligand to its receptor.

The β-substituent in the B-part of the A₃B-type silicon phthalocyanines **B** or **C** or the α-substituent in the B-part of the A₃B-type silicon phthalocyanines *iso*-**B** or *iso*-**C**, all according to the present invention, contains β- or α-bound oxygen, a linker L, and a reactive or activable grouping Q as a -O-L-Q moiety. The -O-L- part of said -O-L-Q moiety may be synthesized advantageously from bifunctional reactants containing both a hydroxyarene moiety and a haloarene moiety - including halohydroxyarenes, but not limited to them. The aryloxy moiety of these reactants allows to synthesize A₃B-type silicon phthalocyanines, which display an O-Ar group at C-β or at C-α, in a straightforward manner. The haloarene moiety of the same reactant acts as a re-functionalization site because it allows to vary the then-present A₃B-type silicon phthalocyanine into stucturally different A₃B-type silicon phthalocyanines in a straightforward manner, namely by Cu-catalyzed, Pd-catalyzed or Cu-/Pd-cocatalyzed C,C or C,heteroatom coupling reactions. These re-functionalizations play a key-role in the synthesis of the silicon phthalocyanines of the invention. This is, firstly, because of their versatility, and secondly, because of making our phthalocyanine syntheses convergent and thereby shorter and higher-yielding than syntheses performed according to the prior art.

Such incorporations of combined hydroxyarene/haloarene precursors by forming an aryloxygen bond in conjunction with subsequent cross-couplings of the resulting haloarene moieties add up to an unprecedented strategy in the synthesis of A₃B-type phthalocyanines of whatever kind. The HO-C_{aryl} bond and the Hal-C_{aryl} bond can reside in one and the same mono- or multinuclear benzenoid aromatic of any kind or in two different mono- or multinuclear benzenoid aromatics of any kind which need not be annulated to each other yet can have a different connectivity with one another, Hal being I, Br or Cl. Thus, the use of a compound of formula HO-L¹-Hal in the preparation of the silicon phthalocyanines according to the present invention results in a unique and unprecedented synthetic strategy.

In preferred embodiments of the hydroxyarene/haloarene precursors (HO-L¹-Hal) of the 4-substituted or 3-substituted phthalodinitrile from which the B-part of the A₃B-type silicon phthalocyanines according to the present invention and thus their substituent -L-Q originate synthetically, the haloarene moiety of the respective A₃B-type silicon phthalocyanine intermediate is coupled by Pd-catalyzed or Cu-/Pd-cocatalyzed C,C coupling reactions. In a preferred embodiment, said coupling reaction is a Sonogashira coupling reaction.

In a preferred embodiment, the compound of formula HO-L¹-Hal is selected from any one of the structures **JJJ, KKK, LLL, MMM, NNN, OOO, PPP** and **QQQ** of

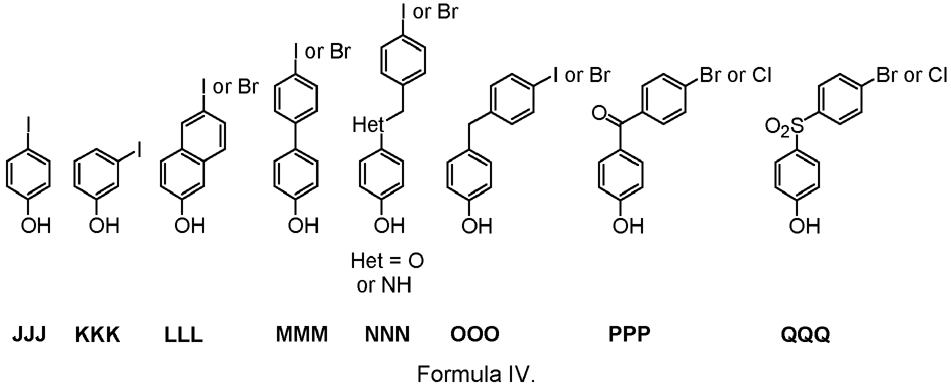

In the silicon phthalocyanines according to the present invention, the silicon atom located in the center of the molecule binds 2 identical groups OR axially; these groups may contain charged groups in the form of ion pairs (i.e. a salt) or di-, tri-, or tetrasaccharides. These groups increase the solubility of the silicon phthalocyanines according to the present invention in water.

In a preferred embodiment, the substituent R has any of the following structures of

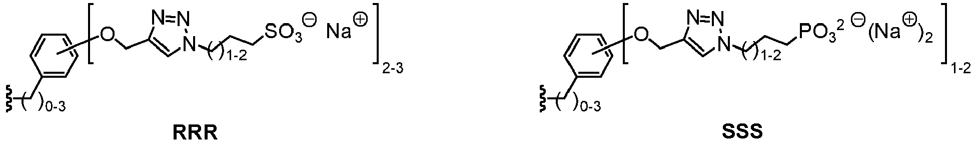

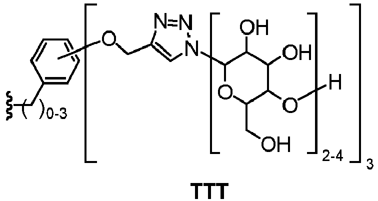

In a more preferred embodiment, the substituent R of the silicon phthalocyanines according to the present invention has any of the structures **UUU** and **VVV** of

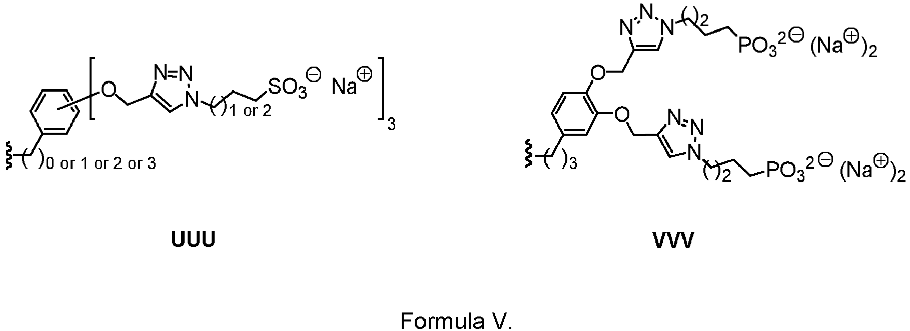

In particularly preferred embodiments, the substituent R has the structure **WWW** of

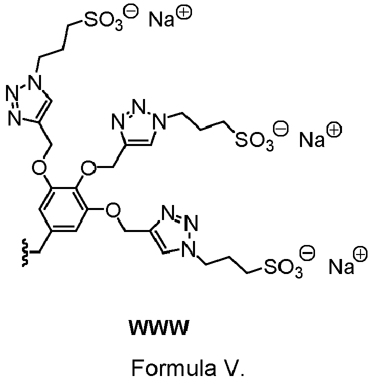

Introducing the water-solubility conferring ion-pair moieties of all preferred silicon phthalocyanines according to the present invention may include "click reactions" between alkyne-containing silicon phthalocyanines and azide-containing sulfonates or azide-containing anions of other strong organic acids.

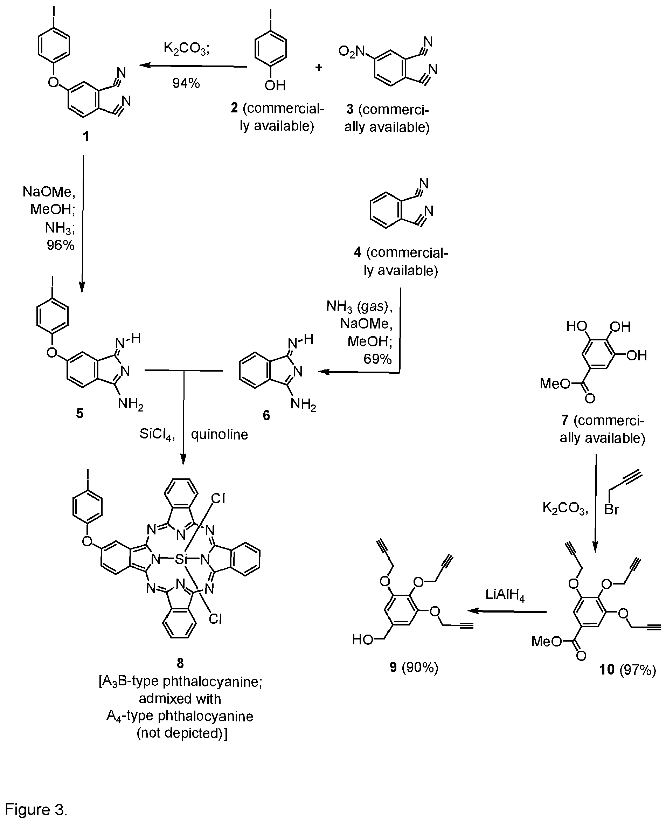

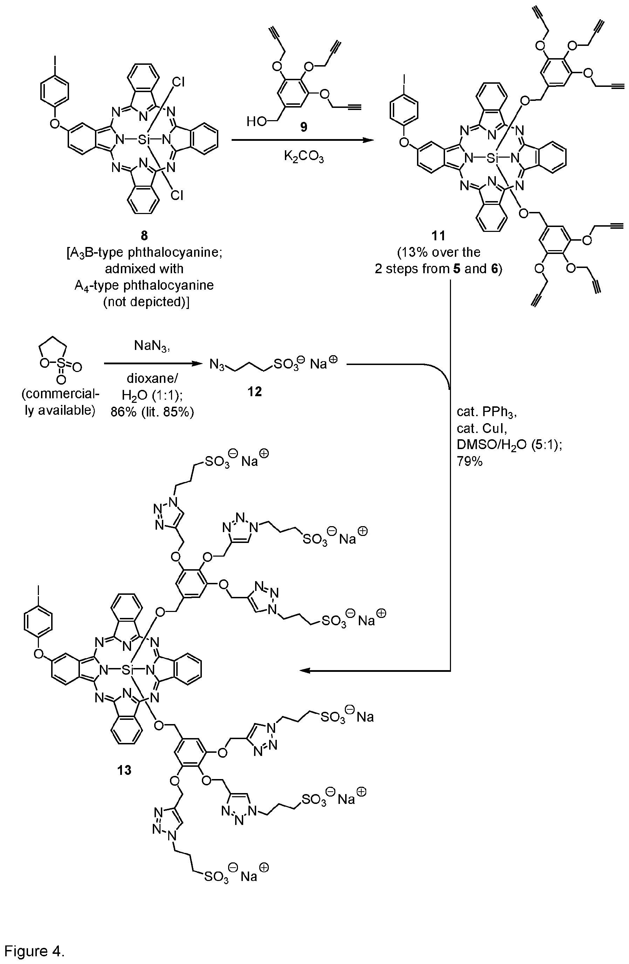

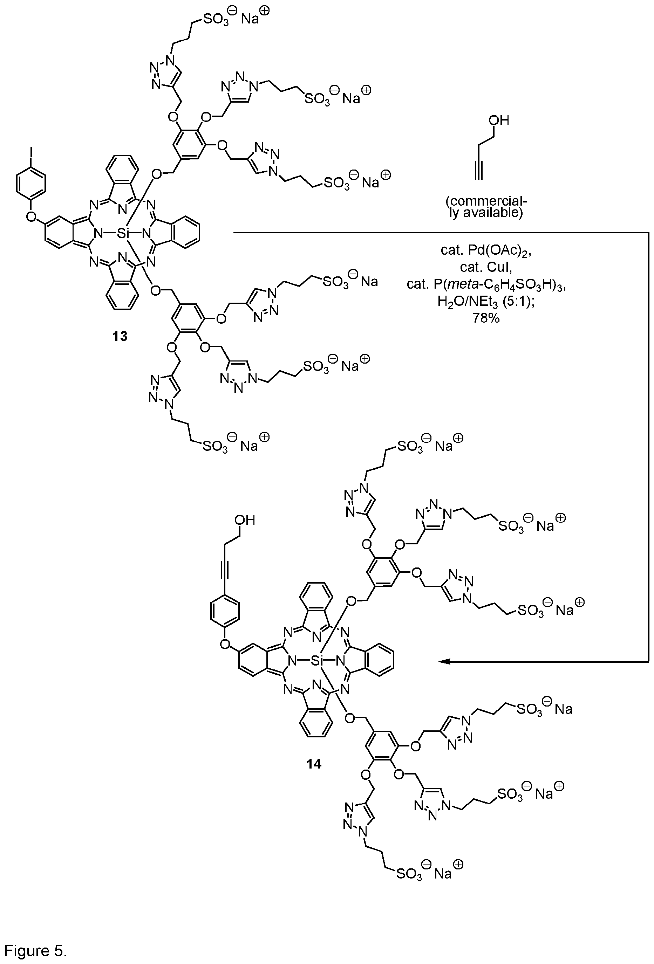

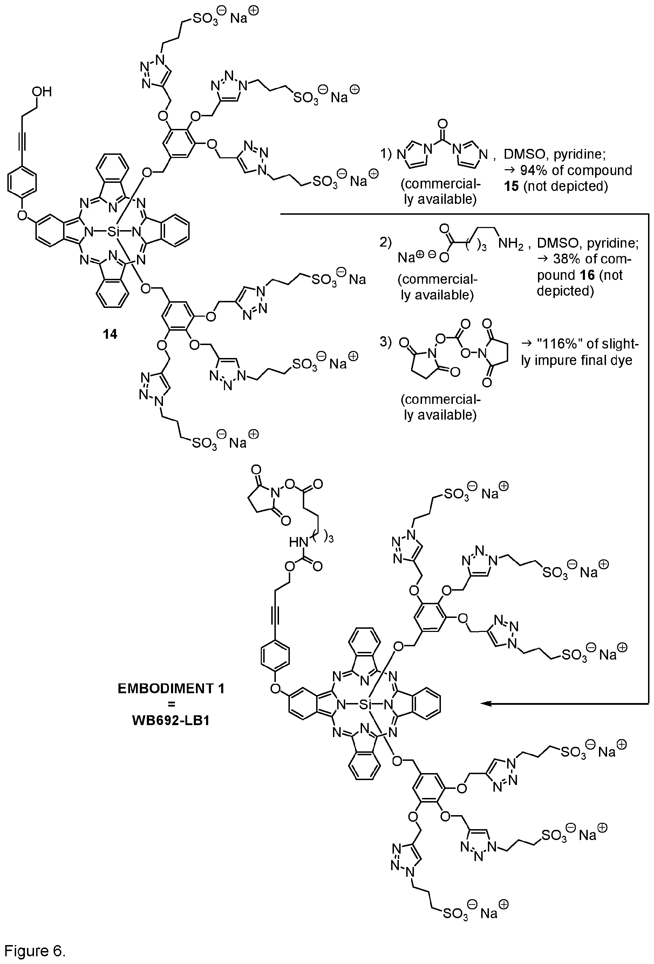

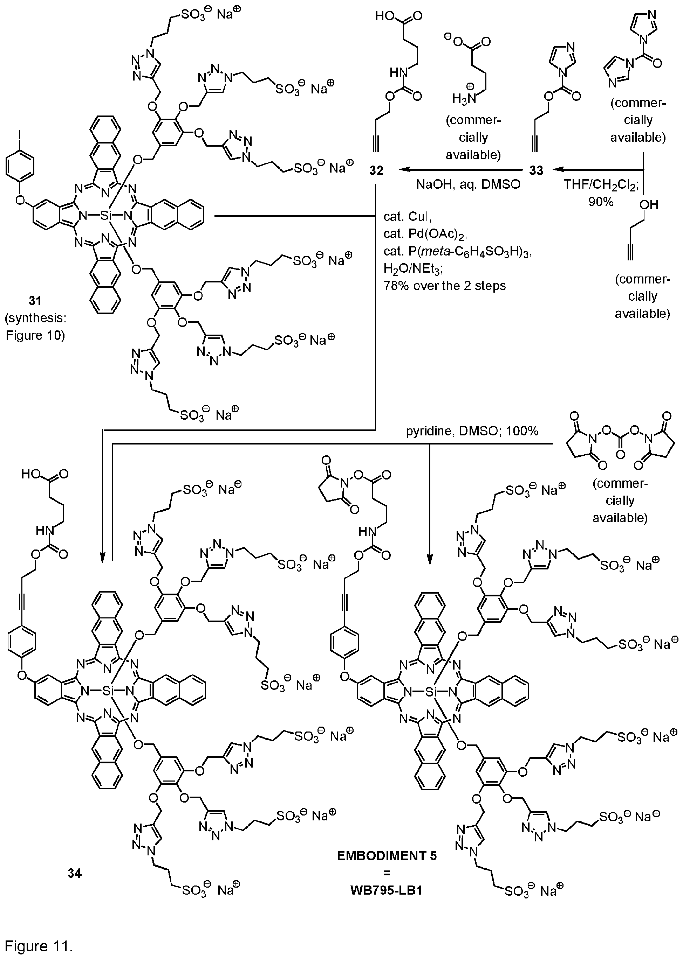

Each synthesis according to Figures 3-12 of the silicon phthalocyanine according to the present invention requires relatively few steps altogether. Moreover, each synthesis is very convergent. This convergency is a significant advantage compared to the synthesis of the silicon phthalocyanine dye **Li-Cor-A** of the prior art known from the US Patent 7,005,518.

Screening the most recent review articles in this regard makes more than clear that the vast majority of silicon phthalocyanine studies concerns silicon phthalocyanines of the A₄-type which cannot form bioconjugates. The A₄-type silicon phthalocyanines **Rakuten-A** from patent application WO 2021/207691, which can form bioconjugates, are extremely exceptional in that regard. A₃B-type silicon phthalocyanines of the prior art can form bioconjugates also only in the very few cases where they were appropriately designed. Till date, syntheses of A₃B-type silicon phthalocyanines need not be longer than syntheses of A₄-type silicon phthalocyanines but current synthetic methodologies imply that the maximum theoretical yields of A₃B-type silicon phthalocyanine syntheses are lower than the maximum theoretical yields of A₄-type silicon phthalocyanine syntheses. It is therefore a particular advantage of the present invention that it furnishes A₃B-type silicon phthalocyanines according to the present invention very convergently and hence in considerably fewer steps in the longest linear sequence compared to the synthesis of the A₃B-type silicon phthalocyanine **Li-Cor-A** known from US Patent 7,005,518 or compared even to *nonbioconjugating* A₃B-type silicon phthalocyanines known from prior art.

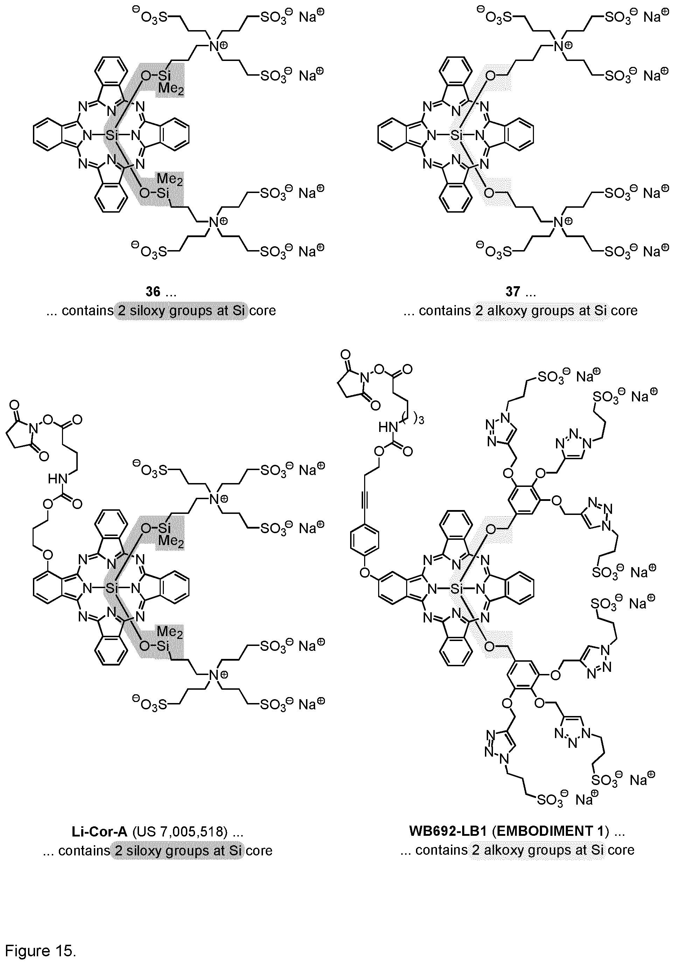

Very little has been established yet in terms of structure/activity relationships in essence because silicon phthalocyanine dyes with an ability to bioconjugate are very rare and hence almost exotic compounds. The most fundamental structure change in the A₃B-type silicon phthalocyanine **Li-Cor-A** from US Patent 7,005,518, which can bioconjugate, compared to the silicon phthalocyanines according to the present invention, which can also bioconjugate, concerns different choices for each dye's pair of axial OR groups: In **Li-Cor-A**, both OR are silyloxy, whereas in the silicon phthalocyanines according to the present invention, both OR are alkoxy or aryloxy or acyloxy or alkylcarbonato or alkylcarbonato. The *same* kind of structure changes differentiates the A₄-type silicon phthalocyanine **36** of the prior art (formula: Figure 15), which cannot bioconjugate, from the A₄-type silicon phthalocyanine **37** of the prior art (formula: *ibid*.), which also cannot bioconjugate. A recent PIT study of these compounds led to the conclusion that compound **36** should cause more cell damage than compound **37** (H. Takakura, S. Matsuhiro, M. Kobayashi, Y. Goto, M. Harada, T. Taketsugu, M. Ogawa, "Axial-ligand-cleavable silicon phthalocyanines triggered by near-infrared light toward design of photosensitizers for photoimmunotherapy", J. Photochem. Photobiol. 2022, 426, 113749).

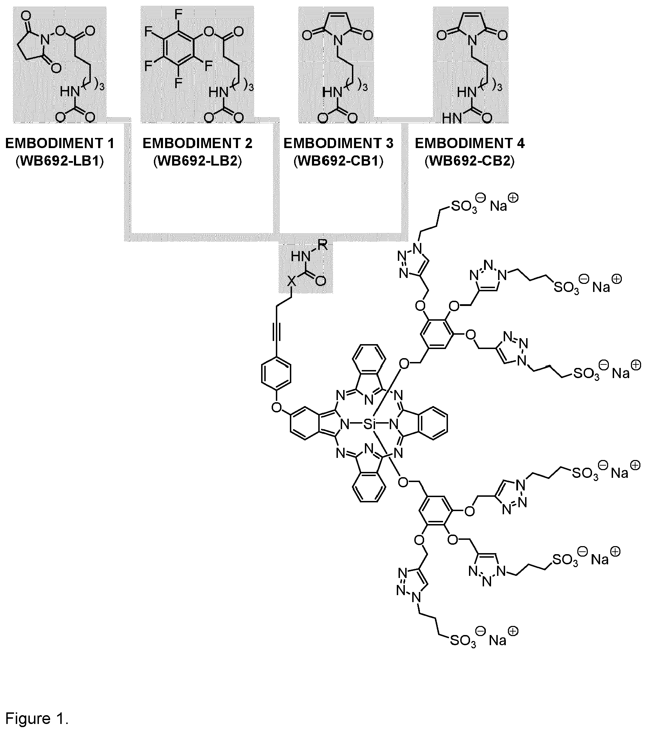

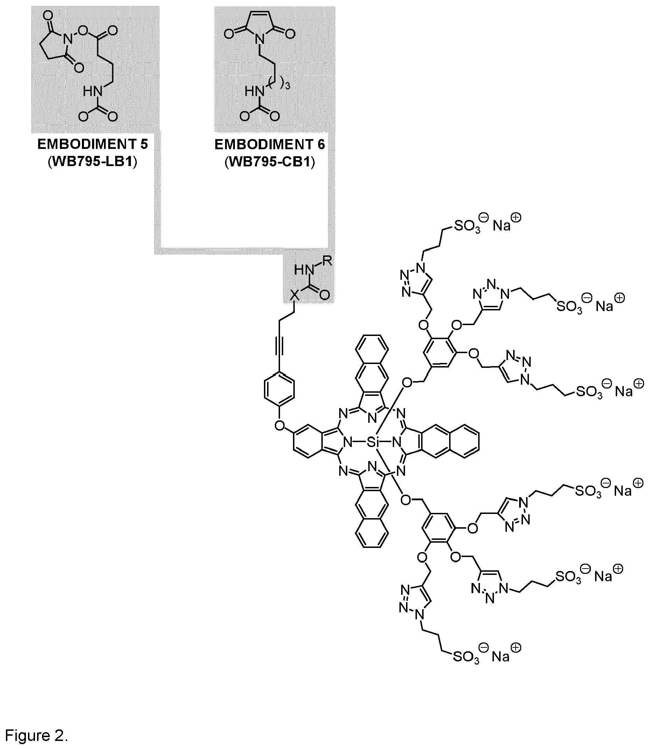

The absorption maxima in the UV/VIS spectra of the representative silicon phthalocyanine **EMBODIMENT 1** (= "**WB692-LB1**"; formula: Figure 1), **EMBODIMENT 2** (= "**WB692-LB2**"; formula: Figure 1), **EMBODIMENT 3** (= **"WB692-CB1"**; formula: Figure 1), and **EMBODIMENT 4** (= "**WB692-CB2**"; formula: Figure 1), all representatives of the silicon phthalocyanines **B** according to Formula I according to the present invention, occur at λₘₐₓ = 692 nm; this amounts to a bathochromic shift of 12 nm compared to the absorption maximum of the silicon phthalocyanine **Li-Cor-A** disclosed in US Patent 7,005,518 which occurs at λₘₐₓ = 680 nm. The absorption maxima in the UV/VIS spectra of the representative silicon phthalocyanine **EMBODIMENT 5** (= "**WB795-LB1**"; formula: Figure 2) and **EMBODIMENT 6** (= "**WB795-CB1**"; formula: Figure 2), both representatives of the silicon phthalocyanines C according to Formula I according to the present invention, occur at λₘₐₓ = 795 nm; this amounts to a bathochromic shift of 115 nm compared to the absorption maximum of the silicon phthalocyanine **Li-Cor-A** of the prior art disclosed in US Patent 7,005,518.

The structures of 6 representative particularly preferred embodiments of the silicon phthalocyanines according to the present invention are shown in Figures 1-2, and the synthetic sequences leading to them are detailed in Figures 3-12.

One of the most preferred application areas of the silicon phthalocyanine dyes according to the present invention is TPDT. In the following some important terms are explained in more detail.

### PDT (Photodynamic Therapy)

PDT is a clinical treatment modality for various diseases, including cancer. It involves the topical or systemic administration of a dye followed by selective irradiation of the target lesion with a specific wavelength of non-ionizing light, which activates the dye to trigger death of the target cells. Due to this two-step therapeutic process, PDT is a safe and minimally-invasive therapy. Nevertheless, classical non-targeted dyes lack sufficient tumor selectivity and are taken up into neighboring normal tissues, resulting in adverse side effects.

PDT employs nontoxic dyes and harmless visible light in combination with oxygen to produce cytotoxic reactive oxygen species that destroy malignant cells and tissues by multifactorial mechanisms including a direct cell-killing, destruction of vasculature to deprive the malignant tissues of oxygen and nutrient and the induction of acute inflammation that attracts leukocytes.

### TPDT (Targeted Photodynamic Therapy)

PDT is therefore being investigated as a target specific treatment, also called TPDT. For this, the dyes are conjugated to carrier molecules, which specifically bind to antigens of the target cells. Dyes are preferred with high absorption in the red or NIR light spectrum, which offers deepest tissue penetration.

### PIT (Photoimmunotherapy)

When molecules concerning the immune system, e.g. antibodies or antibody fragments, are used as binding moieties, TPDT is also called photoimmunotherapy (PIT).

### Site-specific conjugation of dyes

To generate homogeneous products with predictable and often also improved characteristics, site-specific conjugation of diagnostic or therapeutic drugs to carrier molecules, is the preferred method. One method is to mutate amino acids in the carrier molecule to cysteines or to add cysteines for the conjugation of a dye via maleimide linkers.

In an IgG antibody molecule, cysteines form disulfide bridges for the inter- and intra-chain connections of the heavy and light chains. When the dyes include maleimide linkers for the conjugation to cysteine residues, selective reduction of disulfide bonds in the hinge region or between the heavy and light chains of the IgG antibodies is necessary to generate free SH groups for conjugation. With such procedures, however, heterogeneous conjugates consisting of heavy chain-light chain molecules with only monovalent binding, or single heavy or light chains that can no longer bind to the target antigen, are produced.

Therefore, a preferred method for TPDT is to conjugate dyes to engineered cysteines in the binding moieties that do not influence their folding or binding for avoidance of heterogeneous conjugates with undefined binding properties.

### • Modifications for stable linkage

Antibodies with engineered cysteines can be used for the site-specific conjugation of cytotoxic drugs having e.g. maleimide linkers and are also called thiomabs. In principle, all amino acids of an antibody molecule can be mutated to cysteines. The site-specific location of the conjugation site, however, can impact the stability of the linkage between a drug and an IgG antibody. In the following some preferred modifications are shown, whereby, however, variants thereof are within the knowledge of the person skilled in the art.

The preferred amino acids for stable linkage of an antibody with drugs via maleimide linkers in the C_{H}2 and C_{H}3 domains comprise (EU numbering; abbreviations of the amino acids, see Table 1):
V262, I336, S337, R344, E388, N421, S424, E345, Q438, L443,

The preferred amino acids in the C_{L} domain are:
A118, Q124, K126, T129, R142, K149, Q155, S171, E195, L201, V205, T206

### • Modifications to modulate antibody effector functions

The structure of an IgG antibody is comprised of two antigen-binding Fab arms linked to a Fc domain via the hinge region. This structural arrangement allows antibodies to link bound antigens with humoral and cellular components of the immune system. Engagement of the humoral immune response is governed, in large part, by interactions of the antibody with the protein C1q and the initiation of a series of proteolytic events known as the complement cascade. Cytotoxicity initiated via the complement cascade is also termed complement dependent cytotoxicity (CDC).

The cellular immune response occurs mostly due to the interactions between the antibody and Fc gamma receptors (FcγRs). Intracellular signaling through the activating receptors is modulated through the phosphorylation of immunoreceptor tyrosine-based activation motifs (ITAMs), which leads to effector functions such as antibody-dependent cell-mediated cytotoxicity (ADCC), antibody-dependent cellular phagocytosis (ADCP), and inflammation via the induction of cytokine secretion.

Moreover, half-life of IgG antibodies is attributed, in large part, to their ability to bind the neonatal Fc receptor (FcRn) through the Fc domain.

Preferred single amino acids for direct C1q interactions are (EU numbering):
D270, K322, P329, P331
- Preferred key sequence motifs for FcyR interactions are
   P232-S239, D265-D270, Y296-T299, N325-I332
- Preferred key sequence motifs for FcRn interactions are
   L251-S254, L309-Q311, N434-Y436

Various studies have shown that mutations of specific amino acids in an antibody, which are inside or outside the key sequence motifs for C1q, FcyR or FcRn, can influence effector functions. These amino acids should be used preferentially for the cysteine mutations to simultaneously enhance the efficacy and/or safety of a TPDT.

In the case that increased effector function is desired (e.g. target cells outside the light focus are to be damaged during TPCT, like metastases that also express the target antigen), the preferred amino acids in the antibody molecule to be mutated to cysteines are for enhanced CDC (12):
S267, H268, S324, K326, E333, E345, E430, S440
   - for enhanced ADCC:
L234, L235, G236, S239, F243, H268, D270, R292, S298, Y300, V305, K326, A330, I332, E333, K334, P396
   - for enhanced ADCP:
G236, S239, I332

In the case that reduced effector functions are desired (e.g. for the reduction of off-target toxicities or to reduce the induction of pro-inflammatory cytokines), the preferred amino acids to be mutated to cysteines are:
- L234, L235, G236, P238, N297, L328, A330, P331(for IgG1)
- V234, G237, P238, H268, V309, A330, P331 (for IgG2)
- S228, F234, L235, L236, G237, E318 (for IgG4)

In the case that a prolonged serum half-life is desired (e.g. for lowering the therapeutic dose and/or frequency of administration), the preferred amino acids for cysteine mutations are:
- T250, M252, S254, T256, V259, T307, V308, E380, M428, N434

Table 1 shows the meaning of the mutated amino acids in the one letter code.

**Table 1**

| **Amino acid name** | **one letter abbreviation** |
|---|---|
| Alanine | A |
| Arginine | R |
| Asparagine | N |
| Aspartic acid | D |
| Cysteine | C |
| Glutamine | Q |
| Glutamic acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

### Induction of immunogenic cell death

It was found that PDT and TPDT/PIT resulted not only in functional damage of cell membranes of the targeted cells, but also to a rapid induction of an immunogenic cell death (ICD). Following IR700-based PIT, ICD was marked by activation of stress markers including heat shock proteins 70 and 90, dying signals such as calreticulin, ATP and HMGB1, which promote maturation of immature dendritic cells, and initiation of a host immune response against released antigens from dying cancer cells that amplifies the therapeutic effect. Therefore, combination therapies that further enhance tumor-cell-selective systemic hostimmunity are under investigation, e.g. use of T-cell activating type 1 cytokines, such as IL-2 and IL-15, immune-checkpoint inhibitors, such as anti-CTLA4 or anti-PD1/PDL1 antibodies, or depletion of immune-suppressor cells such as the negative regulatory T-cells (T_{reg}) or the myeloid derived suppressor cells (MDSC). This could lead to responses in distant metastases that are out of the light focus and may also inhibit recurrences due to activation of systemic anticancer immunity and long-term immune memory without the systemic autoimmune adverse effects often associated with immune checkpoint inhibitors.

The silicon phthalocyanine dyes according to the present invention can be detected or activated with red/NIR light that can penetrate deeper into tissue than light with a shorter wavelength. This makes diagnosis and therapy based on such dyes more effective, e.g. the diagnosis and therapy of skin diseases or superficial tumors(e.g. melanomas, oesophageal , head and neck colon or urothelial tumors). Moreover, when bioconjugates, consisting of the dyes of the present invention linked to a molecule targeting tumor cells, are used e.g. in the operation field after surgical excision of a tumor it is of utmost importance that the NIR light can penetrate the tissue as deeply as possible in order to eliminate specifically remaining tumor cells that were not removed by surgery and that were targeted by the bioconjugate for complete removal of the tumor cells, avoidance of tumor recurrence and improved therapeutic outcome.

Moreover, the silicon phthalocyanine dyes according to the present invention having a maximal absorption wavelength of 795 nm might be preferred for specific clinical use. For example, light of about 800 nm wavelength has been shown to have maximum penetration through the cranium and into the brain, so that dyes with an appropriate absorption wavelength of about 800 nm are considered optimal for detection and therapy of lesions in the skull as well as in bones or in areas that are covered by bone. This particularly concerns the detection and treatment of brain tumors, bone metastases or lesions associated with neurodegenerative diseases.

The silicon phthalocyanines according to the present invention might also be used in a dual-labeled molecule targeting tumor cells (e.g. antibody or ligand), where the first label is the silicon phthalocyanine and the second label is a radioactive tracer for pre-, intra- and postoperative diagnosis and therapy. E.g. the radioactive tracer can be used for the diagnosis of the tumor, e.g. via positron emission tomography (PET), followed by PIT/PDT of the tumor. Alternatively, the silicon phthalocyanines according to the present invention in a dual-labeled molecule might be used for tumor diagnosis and the radioactive tracer for the following targeted radiotherapy.

| *List of frequently used abbreviations:* |
|---|
| 3:1 naphthalo-/phthalocyanine hybrid = tribenzophthalocyanine |
| APCI = atmospheric pressure chemical ionization |
| ESI = electrospray ionization |
| HRMS = high-resolution mass spectrum |
| IR = infrared |
| NIR = near-infrared |
| NMR = nuclear magnetic resonance |
| PDT = photodynamic therapy |
| PIT = photoimmunotherapy |
| PS = photosensitizer |
| PSMA = prostate-specific membrane antigen |
| silicon phthalocyanines according to the invention = silicon phthalocyanines including 3:1 naphthalo-/phthalocyanine hybrids according to the invention |
| silicon phthalocyanine dyes according to the invention = silicon phthalocyanine dyes including 3:1 naphthalo-/phthalocyanine hybrid dyes according to the invention |
| siloxy = silyloxy |
| TPDT = targeted photodynamic therapy |
| US Patent 7,005,518 = X. Peng, D. R. Draney, J. Chen, Li-Cor Inc., Lincoln, Nebraska, *Phthalocyanine Dyes*, US-Patent 7,005,518 B2, February 26th, 2006**.** |
| WO 2021/207691 = J. Betancourt, L. Makings, T. Wiemann, R. Hoey, Rakuten Medical Inc., San Mateo, California, *Phthalocyanine Dye Compounds, Conjugates and Methods of Use Thereof*, WO Application number 2021/207691 A1, October 14th, 2021**.** |
| UV/VIS = ultraviolet and visible light |
| WB692-LB1 (for Wolf-Brückner silicon pthalocyanine dye with λₘₐₓ = 692 nm, lysine-binding variant #1) = **EMBODIMENT 1** (formula: Figure 1) |
| WB692-LB2 (for Wolf-Brückner silicon pthalocyanine dye with λₘₐₓ = 692 nm, lysine-binding variant #2) = **EMBODIMENT 2** (formula: Figure 1) |
| WB692-CB1 (for Wolf-Brückner silicon pthalocyanine dye with λₘₐₓ = 692 nm, cysteine-binding variant #1) = **EMBODIMENT 3** (formula: Figure 1) |
| WB692-CB2 (for Wolf-Brückner silicon pthalocyanine dye with λₘₐₓ = 692 nm, cysteine-binding variant #2) = **EMBODIMENT 4** (formula: Figure 1) |
| WB795-LB1 (for Wolf-Brückner 3:1 naphthalo-/phthalocyanine hybrid dye with λₘₐₓ = 795 nm, lysine-binding variant #1) = **EMBODIMENT 5** (formula: Figure 2) |
| WB795-CB1 (for Wolf-Brückner 3:1 naphthalo-/phthalocyanine hybrid dyes with λₘₐₓ = 795 nm, cysteine-binding variant #1) = **EMBODIMENT** 6 (formula: Figure 2) |

### Examples

The present invention is further illustrated by the following Examples and the Figures which represent preferred embodiments of the present invention:

### I. Preparative Examples and Experimental Methods

### 1.1 Chemicals

Under a dry atmosphere of N₂, tetrahydrofuran (THF) was distilled over potassium, n-hexane likewise, and diethyl ether (Et₂O) over Na/K alloy. Dichloromethane (CH₂Cl₂) and acetonitrile (MeCN) were distilled over CaH₂ under a dry atmosphere of N₂. Cyclohexane (C₆H₁₂) and ethyl acetate (EtOAc) for flash chromatography and workup were distilled using a rotary evaporator. Chloroform (CHCl₃), dichloromethane (CH₂Cl₂), diethyl ether (Et₂O), methanol (MeOH), all for workup and/or flash chromatography, were purchased as "p.a. grade" and used without further purification.

### 1.2 Laboratory hardware

Reactions were carried out in oven-dried (80°C) glassware. Chemicals were transferred under N₂atmosphere in plastic syringes, which were connected to oven-dried or packaged sterile steel cannulas. All apparatuses were used with a connection to a Schlenk line to prevent contact with water or air.

### 1.3 Thin-layer chromatography

Thin-layer chromatography on Merck silica plates (TLC Silica gel 60 F₂₅₄; supporting material = glass) served for monitoring reactions and assessing purification procedures. If possible, chromatograms were marked in UV light at 254 nm and subsequently stained using Ce(ll)stain [CeSO₄ · 4 H₂O with molybdophosphoric acid (6.25 g) and conc. H₂SO₄ (15 mL) in H₂O (230 mL)]. For ionic compounds Merck C18 reversed-phase silica gel plates were used.

### 1.4 Flash chromatography

Covalent compounds were purified by column chromatography in glass columns filled with silica gel as the solid phase, using compressed air for accelerating elution. Purification conditions are documented in the following style: (Ø [cm], h [cm], F [mL], solv1:solv2 a:b, Fx-Fy); this means: A column with an inner diameter of QJ was packed with silica gel to a height of h. Fractions of the size F were collected. The product was eluted with the solvent solv1 and solv2 in a ratio of a:b or, when a gradient of solvent mixtures was employed (solv1:solv2 a:b → a':b'), in ratios which are mentioned in the respective experiment. The fractions containing the product are specified as Fx-Fy.

Ionic compounds were purified in a Büchi flash machine consisting out of two pump modules C-605, a pump manager C-615, a UV photometer C-635, and a fraction collector C-660. The machine was charged with polypropylene cartridges prepacked either by Macherey-Nagel with reversed-phase silica gel (surface-modified with *n*-H₃₇C₁₈-SiCl₃; "C₁₈ reversed-phase silica gel") or by Interchim with reversed-phase silica gel (surface-modified with *n*-H₉C₄-SiCl₃; "C₄ reversed phase silica gel"). Purification conditions are documented in the same style as detailed for purifications by flash chromatography on silica gel (*cf. supra*)*.*

### 1.5 Nuclear magnetic resonance spectroscopy

Nuclear magnetic resonance (from here "NMR") experiments were performed at the Institute for Organic Chemistry of the Albert-Ludwigs-Universität Freiburg on a Bruker DRX 500 MHz (¹H, ¹³C), a Bruker Avance II 400 MHz (¹H, ¹³C) or a Bruker Avance III 300 MHz (¹H, ¹³C). In the ¹H-NMR spectra the internal standard was residual protio-chloroform (CHCl₃: *δ* = 7.26 ppm). In ¹³C-NMR spectra CDCl₃ was used as an internal standard (middle peak of the 3-line resonance has *δ* = 77.10 ppm). Other NMR solvents and the residual hydrogen equivalents therein, which served as the respective internal standard, were CD₃OD (¹H [3.31 ppm], ¹³C [49.00 ppm]), D₂O (¹H [4.79 ppm]), C₆D₆(¹H [7.15 ppm], ¹³C [128.00 ppm]), and DMSO-d₆ (¹H [2.50 ppm], ¹³C [39.52 ppm]). All NMR spectra were processed and analyzed by Bruker's program TopSpin. ¹H-NMR data are reported as follows: chemical shift (*δ* in ppm), multiplicity (s for singlet; d for doublet; t for triplet; m for multiplet; m_{c} for symmetrical multiplet; br for broad signal), coupling constant(s) (in Hz; ³*J* couplings implied unless otherwise noted), integral (always complying with the assignment), and nucleus-specific assignment (referring to the numbers in the accompanying drawing; those may differ from the numbering which underlies the respective compound's IUPAC name). All resonances - except AB signals - were interpreted as being due to first-order splittings; in AB signals, the high-field proton is denoted "A" and the low-field proton "B". Integrals over the ¹H-NMR signals were consistent with our assignments. ¹³C NMR data are reported in terms of chemical shift (*δ* in ppm) and nucleus-specific assignment (again, the numbering refers to that of the accompanying drawing and may differ from the numbering which underlies the respective compound's IUPAC name).

### 1.6 Mass spectrometry

Mass spectrometry: High resolution mass spectra (from here "HRMSs") were recorded by C. Warth on a Thermo Scientific Exactive mass spectrometer equipped with an orbitrap (ion trap) analyzer. Ionization method: electrospray ionization (from here "ESI"; spray voltage: 2.5-4.0 kV) or atmospheric pressure chemical ionization (from here "APCI"; spray current: 5 µA).

### 1.7 Syntheses of EMBODIMENT 1 (= silicon phthalocyanine WB692-LB1; the corresponding reactions are visualized in Figures 3-6)

### Preparation 1: 4-(4-lodophenoxy)phthalodinitrile (1)

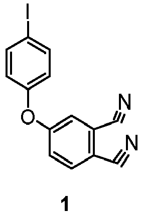

4-Nitrophthalodinitrile (**3**; 15.0 g, 86.6 mmol), K₂CO₃ (89.80 g, 649.8 mmol, 7.5 equiv.) and 4-iodophenol (28.6 g, 130 mmol, 1.5 equiv.) were dissolved in DMF (420 mL). The resulting mixture was stirred for 32 h. Saturated aqueous NH₄Cl (200 mL) and H₂O (500 mL) were added. Extraction with Et₂O (5 × 250 mL) followed. The combined organic phases were washed with aq. NaOH (2 M, 1 × 100 mL, 2 × 50 mL) and dried over Na₂SO₄. All volatiles were removed under reduced pressure. This gave the title compound (**1**; 28.3 g, 81.8 mmol, 94%) as a colorless solid.

### Preparation 2: 5-(4-lodophenoxy)isoindoline-1,3-diimine (5)

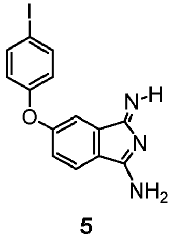

4-(4-lodophenoxy)phthalodinitrile (**1**; 14.94 g, 43.16 mmol) was dissolved in MeOH (100 mL). NaOMe was added (2.09 g, 38.9 mmol, 0.9 equiv.). The resulting mixture was heated at 50°C. A saturated solution of NH₃ in MeOH (150 mL) was added. The resulting mixture was refluxed for 2 h. All volatiles were removed under reduced pressure. H₂O (100 mL) was added. The precipitate was filtered off. It was dried over KOH in vacuo. This delivered the title compound (**5**) as a slightly greenish solid (15.18 g, 41.79 mmol, 96%).

### Preparation 3: Isoindoline-1,3-diimine (6)

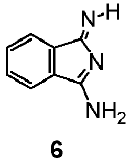

NaOMe (15 g, 27 mmol, 0.70 equiv.) was dissolved in MeOH (400 mL). The resulting mixture was heated at 50°C. Phthalodinitrile (**4**; 50.0 g, 390 mmol) was added. The resulting mixture was heated under reflux. A solution of NH₃ in MeOH (200 mL, 7nn, 1.40 mol, 3.6 equiv.) was added. Heating under reflux was continued for 2.5 h. All volatiles were removed under reduced pressure. H₂O (200 mL) was added. A precipitate formed. It was filtered off 30 min later. It was washed with H₂O (150 mL) and dried over KOH in vacuo for 2d. The title compound (**6**) resulted as a slightly blue solid (38.96 g, 268.4 mmol, 69%). There was no need for further purification.

### Preparation 4: Methyl 3,4,5-Tris[(prop-2-yn-1-yl)oxyjbenzoate (10)

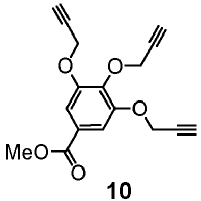

Methyl gallate (**7**; 1.513 g, 8.216 mmol) and K₂CO₃ (5.64 g, 40.8 mmol, 4.96 equiv.) were dissolved in DMF (22 mL). Propargyl bromide (80% in toluene, 6.0 mL, 6.408 g pure, 53.86 mmol) was added dropwise. The resulting mixture was stirred for 5 d. Half-saturated aqueous NH₄Cl (50 mL) and Et₂O (50 mL) were added. The layers were separated. The aqueous layer was extracted with Et₂O (4 × 50 mL). The combined organic layers were washed with brine (2 × 20 mL) and dried over Na₂SO₄. All volatiles were removed under reduced pressure and the residue was purified by flash chromatography on silica gel [QJ = 3.5 cm, h = 14 cm, F = 50 mL, c-C₆H₁₂:EtOAc 90:10, from F15 80:20] . F25-36 delivered the title compound (**10**) as a colorless solid (2.399 g, 8.046 mmol, 97%). Purification of larger quantities of **10** can be realized by crystallization from c-C₆H₁₂:EtOAc or MeOH, which resulted in slightly lower yields (86-89%).

### Preparation 5: (3,4,5-Tris[{prop-2-yn-1-yl}oxy]phenyl)methanol (9)

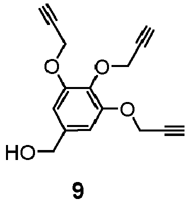

The benzoate **10** (8.00 g, 26.82 mmol) was dissolved in THF (280 mL). LiAlH₄ (3.70 g, 97.5 mmol, 3.64-fold molar amount) was suspended in THF (150 mL). As a first portion, 50 mL of this suspension was added dropwise to the solution of **10**. The reaction mixture was cooled to 0°C. The remainder of the suspension of the reductant was added. An extra portion of LiAlH₄ (4.50 g,119 mmol, 4.42-fold molar amount) was suspended in THF (200 mL) and added as well. The reaction mixture was warmed to room temp. and stirred for 7 h. It was cooled to 0°C. H₂O (8 mL) was added carefully followed by an aqueous solution of NaOH (15%, 8 mL) and pure H₂O (32 mL). MgSO₄ was added MgSO₄ for taking up the excess water. The resulting mixture was filtered. The filter cake was triturated with Et₂O (300 mL). The combined filtrates were liberated from volatile material under reduced pressure. This delivered the title compound (**9**) as a colorless solid (6.528 g, 24.15 mmol, 90%).

### Preparation 6: Sodium 3-azidopropane-1-sulfonate (12)

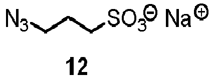

Propanesultone (2.82 g, 23.1 mmol) and NaN₃ (3.00 g, 46.2 mmol, 2.0 equiv.) were dissolved in 1,4-dioxane and H₂O (25 mL, v:v = 1:1). The resulting mixture was stirred at room temp. for 3 d. All volatiles were removed under reduced pressure. The residue was suspended in a mixture of EtOH (25 mL) and H₂O (1 mL). The resulting mixture was stirred under reflux. A suspension resulted which was filtered hot. The filtrate was evaporated under reduced pressure. This gave the title compound (**12**) as a colorless solid (3.74 g, 19.9 mmol, 86%).

### Preparation 7: 29,31-(Dichlorosilanediyl)-2-(4-iodophenoxy)-29H,31H-silicon phthalocyanine (8) and 29,31-Bis{[3,4,5-tris(prop-2-yn- 1-yloxy)phenyl]methoxy}silanediyl-2-(4-iodophenoxy)-29H,31H-silicon phthalocyanine (11)

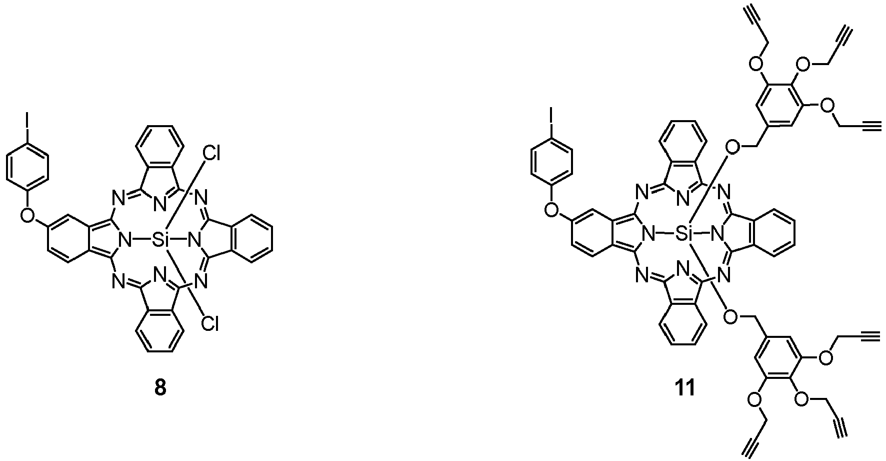

The diiminoisoindolines **5** (1.00 g, 2.75 mmol) and **6** (3.20 g, 22.0 mmol, 8.0 equiv.) were suspended in quinoline (40 mL). Under an atmosphere of N₂, SiCl₄ (2.80 mL, 4.14 g, 24.4 mmol, 8.86 equiv.) was added dropwise. The resulting mixture was heated under reflux for 1.5 h. The reaction mixture was cooled to 120°C and then filtered. The residue was washed with CH₂Cl₂ (2 × 25 mL). The filtrate was diluted with toluene (250 mL), refluxed, and filtered hot. The residue was washed with acetone (20 mL) to deliver a dark blue solid (1.66 g); it contained the expected mixture of the monosubstituted, i. e. A₃B-type silicon phthalocyanine **8** (desired) and its unsubstituted analog 29,31-Bis{[3,4,5-tris(prop-2-yn-1-yloxy)phenyl]methoxy}silanediyl-29*H*,31*H*-silicon phthalocyanine, i. e. the A₄-type parentsilicon phthalocyanine (undesired, not depicted).

Such a "crude silicon phthalocyanine **8**" (1.60 g, ≤1.93 mmol), K₂CO₃ (1.653 g, 11.96 mmol, ≥6.20 equiv.) and the benzyl alcohol **9** (2.69 g, 9.97 mmol, ≥5.17 equiv.) were suspended in toluene (120 mL). The resulting mixture was heated under reflux for 5 h. It was cooled to room temp. and filtered over a pad of silica. The filter cake was triturated with EtOAc (500 mL) and then discarded. All volatiles were removed from the filtrate under reduced pressure. The resulting residue was purified by flash chromatography on silica gel [QJ = 5.5 cm, h = 18 cm, F = 70 mL, toluene:EtOAc 96:4, from F24 94:6]. This delivered (F26-F47) a crude material containing the title silicon phthalocyanine (**11**) admixed with a small amount of the unsubstituted, i. e. A₄-type silicon phthalocyanine. A second purification by flash chromatography on silica gel [QJ = 3.0 cm, h = 21 cm, F = 60 mL, c-C₆H₁₂:EtOAc 85:15, from F30 80:20] delivered (F43-54) the title compound (**11**) as a dark green solid (0.4925 g, 0.3797 mmol, 13%).

### Preparation 8: 2-(4-lodophenoxy)-29,31-bis[(3,4,5-tris{[1-(sodium 3-sulfonatopropyl)-1,2,3-triazol-4-yl]methoxy}phenyl)methoxy]silanediyl-29H,31H-silicon phthalocyanine (13)

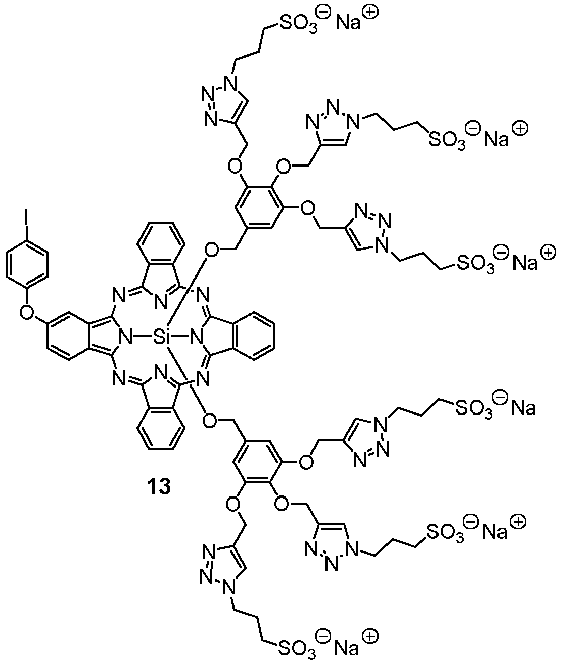

The silicon phthalocyanine **11** (300 mg, 231 *µ*mol), sodium 3-azidopropane-1-sulfonate (**12**; 433 mg, 2.31 mmol, 10 equiv.) and PPh₃ (38.5 mg, 147 *µ*mol, 63 mol-%) were dissolved in DMSO (3 mL). Cul (25.7 mg, 135 *µ*mol, 58 mol-%) was added and then NEt₃ (0.6 mL) was added dropwise. The resulting mixture was stirred for 24 h. It was added dropwise to acetone (14 mL). A precipitate formed. It was concentrated as a pellet by centrifugation. The supernatant liquid was decanted. The remaining pellet was washed with acetone (15 mL) and purified by flash chromatography on "C₁₈ reversed-phase silica gel" [Ø = 2.0 cm, h = 15 cm, F = 20 mL, 25 mL/min, 10 mM HNEt₃^{⊕⊖}OAc (which buffered at pH = 7.0), H₂O:MeCN 85:15 to 70:30 in 15 min]. The collected fractions (F14-18) were concentrated under reduced pressure and the resulting solution was freeze dried. The residue was dissolved in H₂O (4 mL). The resulting solution was added dropwise to a saturated solution of Nal in acetone (15 mL). A precipitate was formed. It was concentrated as a pellet by centrifugation. The supernatant liquid was decanted. The remaining pellet was washed with acetone (10 mL), dissolved in H₂O (10 mL), and freeze-dried to deliver the title compound (13) as a green solid (0.4429 g, 0.1830 mmol, 79%).

### Preparation 9: 2-[4-(4-Hydroxybut-1-ynyl)phenoxy]-29,31-bis[(3,4,5-tris{[1-(sodium 3-sulfonatopropyl)-1,2,3-triazol-4-yl]methoxy}phenyl)methoxy]silanediyl-29H,31H-silicon phthalocyanine (14)

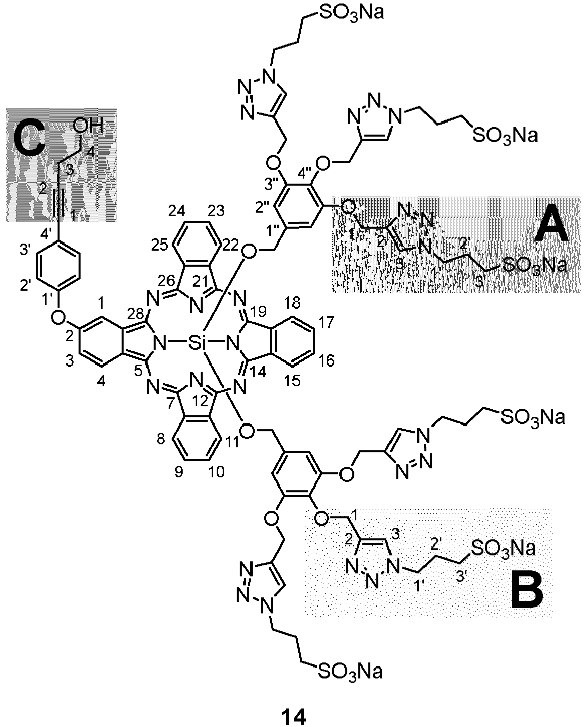

The silicon phthalocyanine 13 (0.100 g, 41.3 *µ*mol) and (*meta*-NaO₃S-H₄C₆)₃P (6.00 mg, 10.3 *µ*mol, 25 mol-%) were dissolved in degassed H₂O (3 mL). Pd(OAc)₂ (10 mM in MeCN, 0.2 mL, 2 *µ*mol, 5 mol-%), Cul (10 mM in MeCN, 0.4 mL, 4 *µ*mol, 10 mol-%), and NEt₃ (0.6 mL) were added. But-3-yn-1-ol (0.5 M in degassed H₂O, 0.82 mL, 41 mmol, 10 equiv.) was added dropwise during 5 h. The resulting mixture was stirred at room temp. for 16 h. Nal (0.3 g) was added. The resulting mixture was added dropwise to acetone (15 mL). A precipitate formed. It was concentrated as a pellet by centrifugation. The supernatant liquid was decanted. The remaining pellet was purified by flash chromatography on "C₁₈ reversed-phase silica gel" [Ø = 2.0 cm, h = 15 cm, F = 20 mL, 25 mL/min, 10 mM HNEt₃^{⊕⊖}OAc (which buffered at pH = 7.0), H₂O:MeCN 85:15 → 75:25 in 20 min]. The collected fractions (F15-25) were concentrated under reduced pressure. The resulting solution was freeze-dried. The resulting residue was dissolved in aqueous Nal (10 weight-%, 1 mL). The resulting solution was added dropwise to acetone (15 mL). A precipitate formed. It was centrifuged, washed with acetone (15 mL) and dissolved in H₂O (6 mL). The solution was freeze-dried to deliver the title compound (**14**) as a green solid (0.76 g, 0.032 mmol, 78%).

**HRMS** (neg. ion mode ESI): [M - 6 x Na^{⊕}] observed as [¹²C₉₂¹H₈₆¹⁴N₂₆¹⁶O₂₈³²Se²⁸Si]^{6⊖} (tantamount to z = 6) with *m*/*z* = 370.5709 which deviates from the calculated *m*/*z* = 370.4039 by -0.21 ppm.

**¹H NMR** (500.42 MHz, DMSO-d₆): *δ* = AB signal (*δ*_{A}, = -0.73, *δ*_{B} = -0.70, ²*J*_{A,B} = 14.3 Hz, 4H, 2 × SiOC-*H*₂R), 1.97-2.07 (tt, ³*J*_{2',3'} = ≈ ³*J*_{2',1'} = 6.8 Hz, 4H, **B**: 2 × 2'-H₂), 2.09-2.21 (m_{c}, 8H, **A**: 4 × 2'-H₂), 2.39-2.44 (m_{c}, 4H, **B**: 2 × 3'-H₂), 2.47-2.50 (m overlapping with DMSO-d₆-Signal, 8H, **B**: 4 × 3'-H₂), 2.65 (t, ³*J*_{3,4} = 6.8 Hz, 2H, **C**: 3-H₂), 3.64 (s overlapping with signal of **C**: 4-H₂, 4H, 4 × 2"-H), 3.67 (t overlapping with signal of 2"-H₂, ³*J*_{4,3} = 6.8 Hz, 2H, **C**: 4-H₂), 3.92 (s, 8H, **A**: 4 × 1-H₂), 4.38 (t, ³*J*_{1',2'} = 6.8 Hz, 4H, **B**: 2 × 1'-H₂), 4.48 (t, ³*J*_{1',2'} = 6.8 Hz, 8H, **A**: 4 × 1'-H₂), 4.58 (s, 4H, **B**: 2 × 1-H₂), br. s (5.01, 1H, C: 4-OH), 7.41 (m_{c}, 2H, 2 × 2'-H), 7.57 (m_{c}, 2H, 2 × 3'-H), 7.76 (s, 2H, **B**: 2 × 3 H), 7.98 (s, 4H, **A**: 4 × 3-H), 8.10 (d, *^{ortho}J*_{3,4} = 8.1 Hz, 1H, 3-H) 8.38-8.51 (m, 6H, 6 × *β*-H = 9-H, 10-H, 16-H, 17-H, 23-H, 24-H), s (9.04, 1H, 1-H), 9.54-9.69 ppm (m, 7H, 7 × *α*-H = 4-H, 8-H, 11-H, 15-H, 18-H, 22-H, 25-H).

**¹³C NMR** (125.85 MHz, DMSO-d₆): *δ* = 23.38 (**C**: C-3), 26.38 (**B**: 2 × C-2'), 26.43 (**A**: 4 × C-2'), 47.96 (**B**: 2 × C-3'), 48.02 (**A**: 4 × C-3'), 48.42 (**B**: 2 × C-1'), 48.55 (**A**: 4 × C-1'), 57.43 (2 × SiOCH₂R), 59.86 (**C**: C-4), 60.85 (**A**: C-1), 65.69 (**B**: C-1), 80.58 (**C**: C-1), 88.50 (**C**: C-2), 101.23 (C-2"), 111.21 (C-1), 119.50 (C-4'), 119.97 (C-2'), 123.34 (C-3), 123.47 (C-X), 123.57 (C-X), 123.67 (C-X), 124.11 (**A**: C-3), 124.14 (**B**: C-3), 125.75 (C-X), 130.23 (C-X), 131.98 (C-X), 132.05 (C-X), 132.11 (C-X), 133.59 (C-3'), 134.04 (C-4"), 134.67 (C-1"), 134.87 (C-X), 134.93 (C-X), 135.02 (C-X), 135.05 (C-X), 136.98 (C-X), 142.14 (**A**: C-2), 143.52 (**B**: C-2), 148.00 (C-X), 148.57 (C-X), 148.60 (C-X), 148.80 (C-X), 148.97 (C-X), 149.24 (C-X), 149.28 (C-X), 150.19 (C-3"), 155.71 (C-1'), 160.11 ppm (C-X).

Resonances which are attributed to nuclei denoted as "C-X" could not be assigned more specifically based on 1D-NMR data alone. Altogether, compound **14** contains ¹³C nuclei in potentially as many as 57 different chemical environments. We detected (and listed above) 48 different ¹³C-NMR resonances.

### Preparation 10: 2-({4-[(Imidazol-1-yl)carbonyloxy]but-1-ynyl}phenoxy)-29,31-bis[(3,4,5-tris{[1-(sodium 3-sulfonatopropyl)-1,2,3-triazol-4-yl]methoxy}phenyl)methoxy]silanediyl-29ft,31H-silicon phthalocyanine (15)

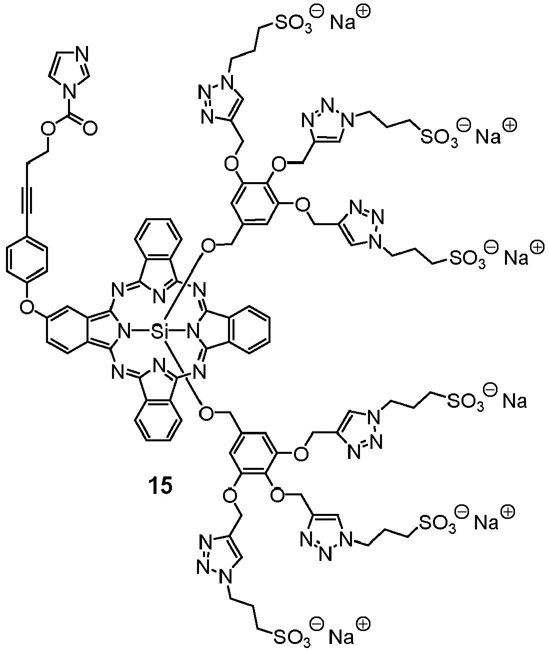

The silicon phthalocyanine **14** (100 mg, 42.3 *µ*mol) was dissolved in DMSO (1.5 mL). Carbonyldiimidazole (68.0 mg, 423 *µ*mol, 10 equiv.) was added. The atmosphere was exchanged with N₂. Pyridine (0.5 mL) was added and the resulting mixture was stirred at 70°C for 5.5 h. It was cooled to room temp. was added dropwise to a saturated Nal-solution (8 mL) in acetone. A precipitate formed. It was concentrated as a pellet by centrifugation. The supernatant liquid was decanted. The remaining pellet was washed with acetone (2 × 10 mL). It was dried in vacuo to deliver the title compound (**15**) as a slightly impure green solid (97.7 mg, 39.8 *µ*mol, 94%).

**HRMS** (neg. ion mode ESI): [M - 6 x Na^{⊕}] observed as [¹²C₉₆¹H₈₈¹⁴N₂₈¹⁶O₂₉³²S₆²⁸Si]^{6⊖} (tantamount to z = 6) with *m*/*z* = 386.0731 which deviates from the calculated *m*/*z* = 386.0733 by -0.64 ppm.

### Preparation 11: 2-[4-({[(5-Carboxylpentyl)amino]carbonyloxy}but-1-ynyl)phenoxy]-29,31-bis[(3,4,5-tris{[1-(sodium 3-sulfonatopropyl)-1,2,3-triazol-4-yl]methoxy}phenyl)methoxy]-silanediyl-29H,31H-silicon phthalocyanine (16)

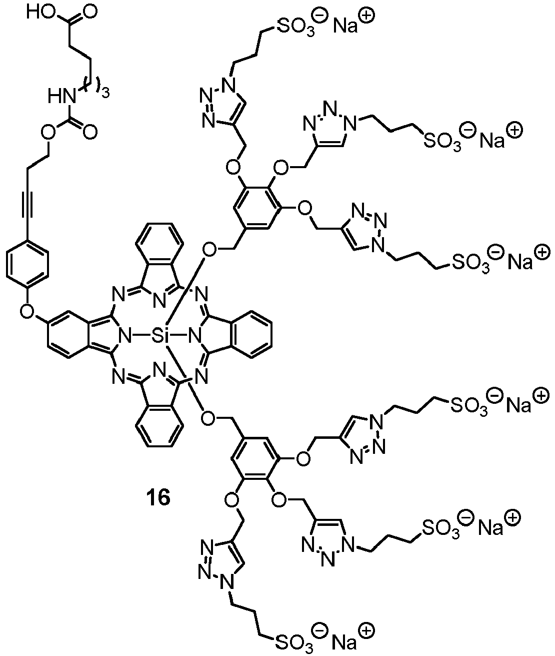

The silicon phthalocyanine **15** (25.0 mg, 10.2 *µ*mol) was dissolved in DMSO (0.2 mL). Pyridine (0.1 mL) and sodium 6-aminohexanoate (0.7 M in H₂O, 60µL, 6.5 mg, 42 *µ*mol, 4.2 equiv.) was added. The resulting mixture was stirred at room temp. for 21 h. It was added to a saturated solution of Nal in acetone (8 mL). A precipitate formed. It was concentrated as a pellet by centrifugation. The supernatant liquid was decanted. The remaining pellet was washed with acetone (10 mL). It was purified by flash chromatography on "C₄ reversed-phase silica gel" [Ø = 2.0 cm, h = 14 cm, F = 20 mL, 25 mL/min, 10 mM HNEt₃^{⊕⊖}OAc (which buffered at pH = 7.0), H₂O:MeCN 90:10 → 70:30 in 28 min]. Fractions 25-27 were concentrated under reduced pressure. The resulting solution was freeze-dried. The solid which resulted was dissolved in H₂O and precipitated by pouring into a saturated solution of Nal in acetone (8 mL). This precipitate was concentrated as a pellet by centrifugation. The supernatant liquid was decanted. The remaining pellet was washed with acetone (8 mL) and thereafter dissolved in H₂O (4 mL) and freeze-dried. This delivered the title compound (16) as a green solid (10 mg, 3.9 *µ*mol, 38%).

**HRMS** (neg. ion mode ESI): [M - 6 x Na^{⊕}] observed as [¹²C₉₉¹H₉₇¹⁴N₂₇¹⁶O₃₁³²S₆²⁸Si]^{6⊖} (tantamount to z = 6) with *m*/*z* = 396.5825 which deviates from the calculated *m*/*z* = 396.5828 by -0.80 ppm.

### Preparation 12: 2-({[({6-[(2,5-Dihydro-1H-2,5-dioxopyrrol-1-yl)oxy]-6-oxohex-1-yl}amino)-carbonyloxy]but-1-ynyl}phenoxy)-29,31-bis[(3,4,5-tris{[1-(sodium 3-sulfonatopropyl)-1,2,3-tri-azol-4-yl]methoxy}phenyl)methoxy]silanediyl-29H,31H-silicon phthalocyanine (EMBODIMENT 1 = WB692-LB1)

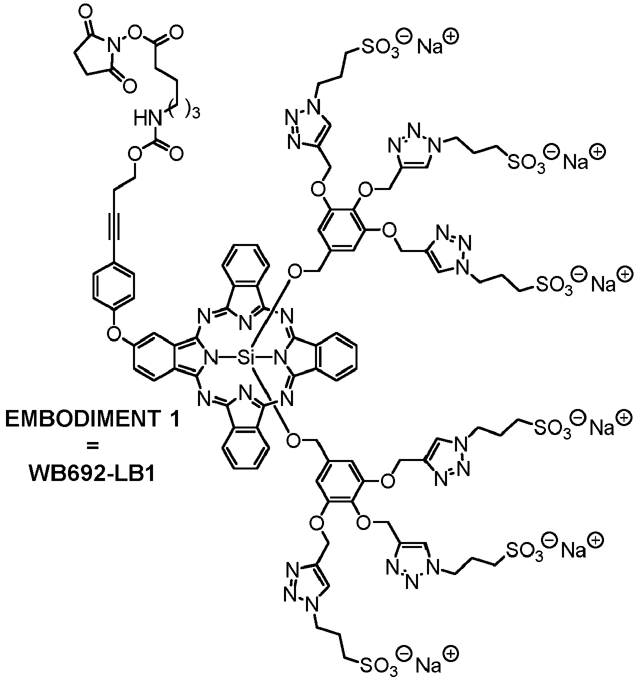

The silicon phthalocyanine **16** (10.0 mg, 3.93 *µ*mol) was dissolved in DMSO (0.2 mL). Disuccinimidyl carbonate (1.8 mg, 7.0 *µ*mol, 1.8 equiv.) was added. The resulting mixture was heated at 60°C for 1.5 h. Additional disuccinimidyl carbonate (3.8 mg,14 *µ*mol, 3.8 equiv.) was added. Stirring at 60°C was continued for 5 min. After cooling to room temp., the reaction mixture was added dropwise to a saturated solution of Nal in acetone. This caused the reaction product to precipitate. It was concentrated as a pellet by centrifugation. The supernatant liquid was decanted. The remaining pellet was washed with acetone (8 mL). All volatiles were removed under reduced pressure to deliver the title compound **(EMBODIMENT 1 = WB692-LB1)** as an impure green solid (12 mg, "116%").

**HRMS** (neg. ion mode ESI): [M - 6 x Na^{⊕}] observed as [¹²C₁₀₃¹H₁₀₀¹⁴N₂₈¹⁶O₃₃³²S₆²⁸Si]^{6⊖} (tantamount to z = 6) with *m*/*z* = 412.7520 which deviates from the calculated *m*/*z* = 412.7522 by -0.60 ppm.

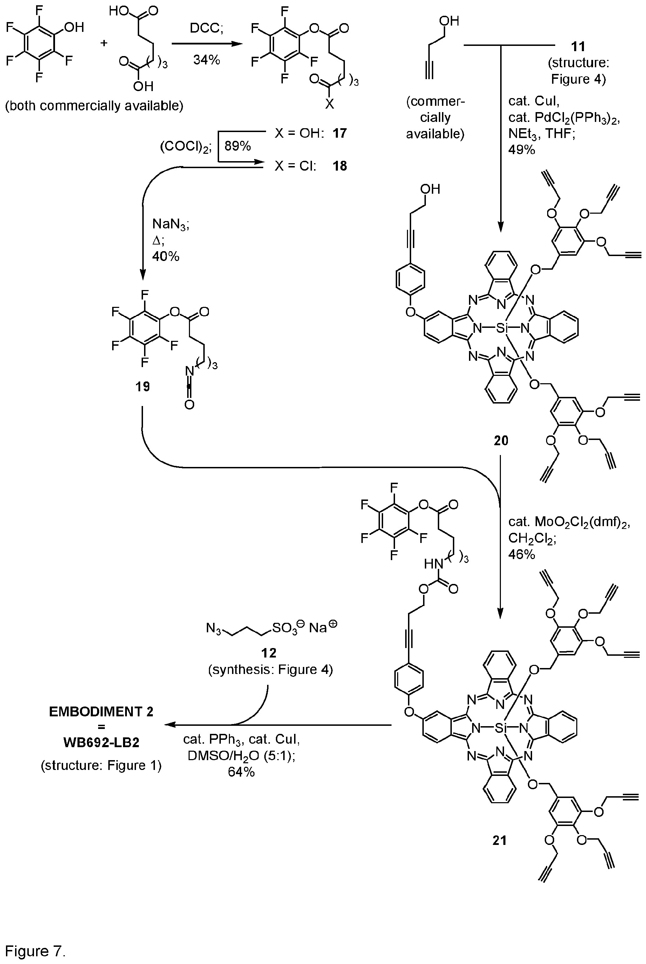

1.8 Synthesis of **EMBODIMENT 2** (= silicon phthalocyanine **WB692-LB2**; the corresponding reactions are visualized - or referred to - in Figure 7)

### Preparation 13: 7-Oxo-7-(perfluorophenoxy)heptanoic acid (17)

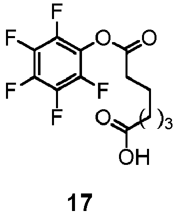

Pentafluorophenol (917 mg, 4.98 mmol) and pimelic acid (814 mg, 5.08 mmol, 1.02 equiv.) were dissolved in EtOAc (15 mL). The resulting solution was cooled to 0°C. N,N`-Dicyclohexylcarbodiimid (1.09 g, 5.28 mmol, 1.06 equiv.) was added in one portion. The resulting mixture was stirred for 1 h at 0°C. It was warmed to room temperature and stirred for 4 h. The reaction mixture was filtrated. The filtrate was evaporated under reduced pressure and the residue was purified by silica column chromatography [QJ = 3 cm, h = 15 cm, F = 50 mL, c-C₆H₁₂:EtOAc → 2:1] which delivered the title compound (**17**; 0.5598 g, 1.716 mmol, 34%, F4-10) as a colorless solid.

### Preparation 14: Perfluorophenyl 7-chloro-7-oxoheptanoate (18)

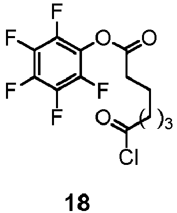

PFP-Monoester **17** (250 mg, 0.766~mmol) was dissolved in CH₂Cl₂ (3 mL). (CO)₂Cl₂ (0.20 mL, 0.30 g, 2.3 mmol, 3.0 equiv.) was added dropwise. The resulting mixture was stirred for 16 h at room temperature. All volatiles were evaporated under reduced pressure to deliver the title compound (**18**; 0.2353 g, 0.6827 mmol, 89%) as a slightly yellow oil.

### Preparation 15: Perfluorophenyl 6-isocyanatohexanoate (19)

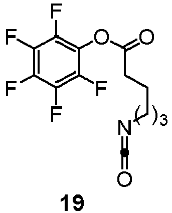

Acid chloride **18** (235.3 mg, 0.6827 mmol) was dissolved in CH₃CN (5 mL). NaN₃ (44.0 mg, 0.683 mmol, 1.0 euqiv.) was added in one portion. The resulting suspension was stirred for 5 h at 60°C. It was cooled to room temperature. The reaction mixture was filtrated over silica. It was rinsed off with CH₂Cl₂ (5 mL). All volatiles were removed under reduced pressure. The residue was purified by silica column chromatography [QJ = 1 cm, h = 14 cm, F = 10 mL, c-C₆H₁₂:EtOAc → 15:1] which delivered the title compound (19; 0.0899 g, 0.2781 mmol, 40%, F4-6).

### Preparation 16: 29,31-Bis{[3,4,5-tris(prop-2-yn- 1-yloxy)phenyl]methoxy}silanediyl-2-[4-{4-hydroxybut-1-yn-1-yl}phenoxy]-29H,31H-silicon phthalocyanine (20)

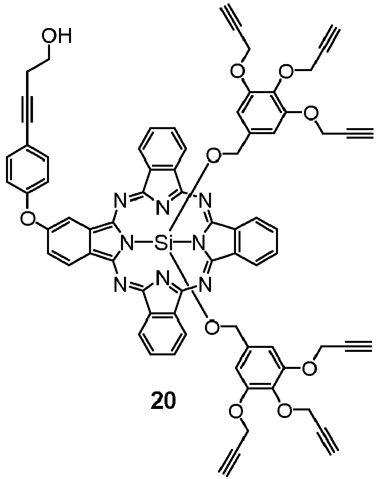

But-3-in-1-ol (2.3 mL, 2.1 g, 30 mmol, 197 equiv.), THF (1 mL) and NEt₃ (0.5 mL) were degassed by freeze-pump method. PdCl₂(P(Ph)₃)₂ (5.4 mg, 7.7 µmol, 5 Mol-%) and Cul (2.9 mg, 15 µmol, 10 Mol-%) were added. A solution of silicon phthalocyanine **13** (200 mg, 154 µmol) in degassed THF (3 mL) was added dropwise over a periode of 45 min. At room temp. it was stirred for 20 h. The mixture was poured on aqueous saturated NH₄Cl (25 mL) and EtOAc (25 mL). The layers were separated and the organic layer was washed with H₂O (6 × 25 mL) dried over Na₂SO₄. All volatiles were removed under reduced pressure and the residue was purified by silica column chromatography [QJ = 2.5 cm, h = 25 cm, F = 20 mL, c-C₆H₁₂:EtOAc → 2:1, at F15 3:2, at F30 1:1] which delivered the title compound (**20**; 93.6-mg, 75.5 µmol, 49%, F42-62) as a dark green solid.

### Preparation 17: 29,31-Bis{[3,4,5-tris(prop-2-yn- 1-yloxy)phenyl]methoxy}silanediyl-2-{4-[4-({[7-oxo-7-(perfluorophenoxy)heptyl]carbamoyl}oxy)but-1-yn-1-yl]phenoxy}-29H,31H-silicon phthalocyanine (21)

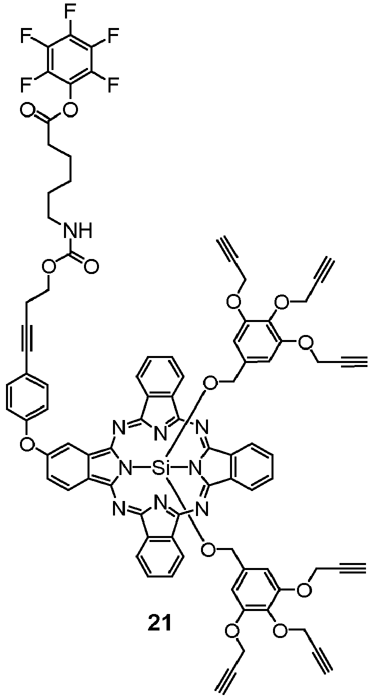

Silicon phthalocyanine **20** (17 mg, 13 µmol) and isocyanate **19** (28 mg, 87 µmol, 6.3 equiv.) were dissolved in CH₂Cl₂ (0.5 mL). MoO₂Cl₂(DMF)₂ (0.47 mg, 1.4 µmol, 10 Mol-%) was added. The resulting mixture was stirred at room temp. for 30 min. EtOAc (20 mL) and aqueous HCl (1M, 10 mL) were added. The layers were separated. The organic layer was washed with H₂O (2 × 10 mL) dried over Na₂SO₄. All volatiles were removed under reduced pressure. The resulting residue was purified by silica column chromatography [QJ = 1 cm, h = 20 cm, F = 8 mL, c-C₆H₁₂:EtOAc → 2:1, at F8 1:1] which delivered the title compound (**21**, 10 mg, 6.4 µmol, 46%, F14-16) as a dark green solid.

### Preparation 18: 2-(4-[4-({[7-oxo-7-(perfluorophenoxy) heptyl]ca rbamoyl} oxy) but-1-yn-1-yl]phenoxy)-29,31-bis[(3,4,5-tris{[1-(sodium 3-sulfonatopropyl)-1,2,3-triazol-4-yl]methoxy}phenyl)methoxy]silanediyl-29H,31H-silicon phthalocyanine (EMBODIMENT 2 = WB692-LB2)

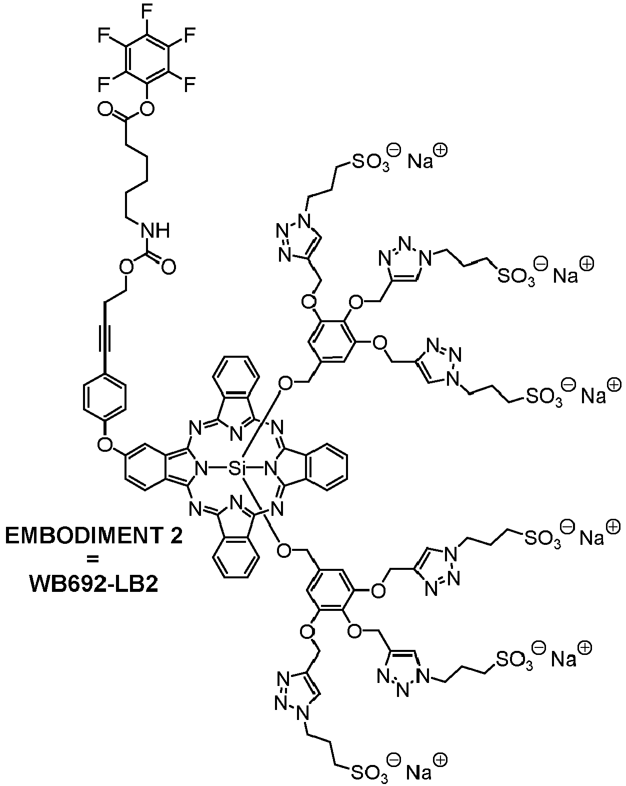

The silicon phthalocyanine **20** (10 mg, 6.4 *µ*mol) and PPh₃ (1.0 mg, 4.1 *µ*mol, 64 mol-%) were dissolved in DMSO (0.06 mL). Sodium 3-azidopropane-1-sulfonate (**12**; 9.5 mg, 6.4 µmol, 8 equiv.), Cul in DMSO (20 µM, 0.2 mL, 60 mol-%) and NEt₃ (0.6 mL) were added. The resulting mixture was stirred for 1 h. It was added dropwise to a saturated solution of Nal in acetone (8 mL). A precipitate formed. It was concentrated as a pellet by centrifugation. The supernatant liquid was decanted. The remaining pellet was washed with acetone (4 × 10 mL). Evaporation of excess acetone under reduced pressure delivered the title compound **(EMBODIMENT 2 = WB692-LB2)** as a green solid (11.1 mg, 4.13 µmol, 64%).

**HRMS** (neg. ion mode ESI): [M - 6 x Na^{⊕}] observed as [¹²C₁₀₅¹H₉₆¹⁴N₂₇¹⁶O₃₁¹⁹F₅³²S₆²⁸Si]^{6⊖} (tantamount to z = 6) with *m*/*z* = 424.2466 which deviates from the calculated *m*/*z* = 424.2469 by -0.6031 ppm.

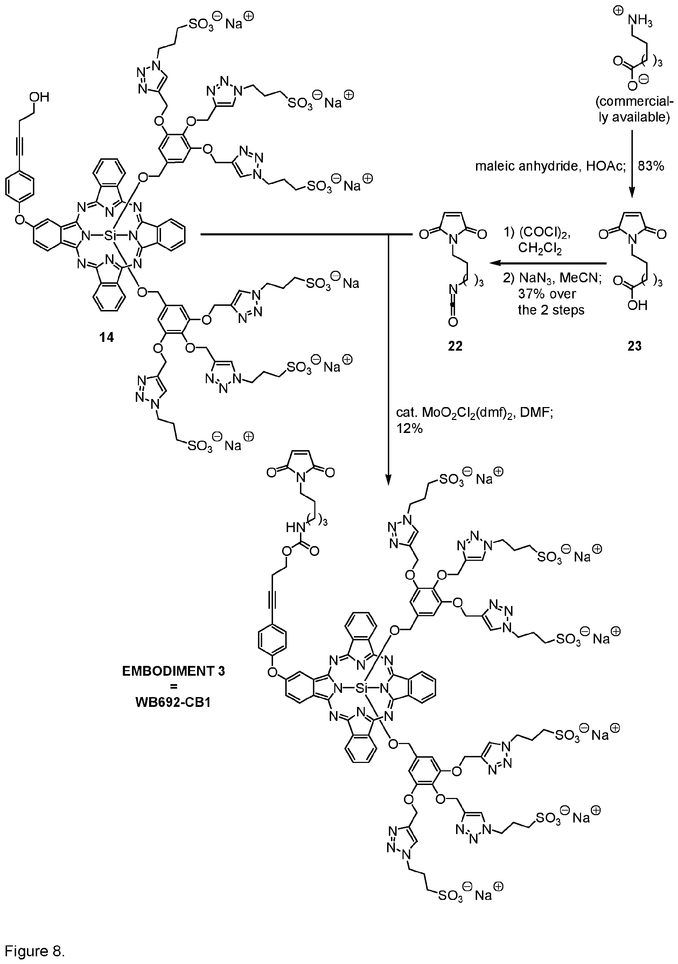

1.9 Synthesis of **EMBODIMENT 3** (= silicon phthalocyanine **WB692-CB1**; the corresponding reactions are visualized - or referred to - in Figure 8)

### Preparation 19: 6-(2,5-Dioxo-2,5-dihydro-1H-pyrrol-1-yl)hexanoic acid (23)

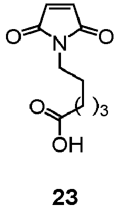

6-Aminohexanoic acid (2.00 g, 15.3 mmol) and maleic anhydride (1.50 g, 15.3 mmol, 1.0 equiv.) were dissolved in AcOH (40 mL). After 30 min a colorless precipitate had formed. The resulting suspension was heated under reflux for 16 h. It was cooled to room temperature. All volatiles were removed under reduced pressure. The resulting residue was dissolved in EtOAc (50 mL) and CH₂Cl₂ (150 mL). It was washed with HCl (4 M, 20 mL) and H₂O (20 mL). The organic layer was dried over Na₂SO₄. All volatiles were removed under reduced pressure. This delivered the title compound (**23**; 2.69 g, 12.7 mmol, 83%) sufficiently pure.

### Preparation 20: 1-(5-Isocyanatopentyl)-1H-pyrrole-2,5-dione (22)

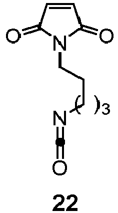

The carboxylic acid **23** (300 mg, 1.42 mmol) was dissolved in CH₂Cl₂ (10 mL). (COCl)₂ (0.38 ml, 0.56 g, 4.4 mmol, 3.1 equiv.) was added dropwise. The resulting mixture was stirred at room temp. for 16 h. All volatiles were removed under reduced pressure. The residue was dissolved in MeCN (5 mL). NaN₃ (92.3 mg, 1.42 mmol, 1.0 equiv.) was added. The reaction mixture was stirred vigorously at room temp. for 2 h and at 70°C for another 2 h. It was cooled to room temp. and filtered over celite and silica. The filter cake was triturated with CH₂Cl₂ (5 mL) and then discarded. All volatiles were removed from the filtrate under reduced pressure, leaving behind the title compound (**22**) as a slightly yellow oil (0.308 g, 1.34 mmol, 94%) almost pure (yet susceptible to decompose when being stored). Purification by flash chromatography on silica gel [QJ = 2 cm, h = 27 cm, F = 20 mL, c-C₆H₁₂:EtOAc 75:25] delivered (F9-13) the title compound (**22**) as a colorless solid (0.122 g, 0.531 mmol, 37%).

### Preparation 21: 2-[({5-[(1H-2,5-Dioxopyrrol-1-yl)pent-1-yl]amino]carbonyloxy)}but-1-yn-yl)phenoxy]-29,31-bis[(3,4,5-tris{[1-(sodium 3-sulfonatopropyl)-1,2,3-triazol-4-yl]methoxy}-phenyl)methoxyjsilanediyl-29H,31H-silicon phthalocyanine (EMBODIMENT 3 = WB692-CB1)

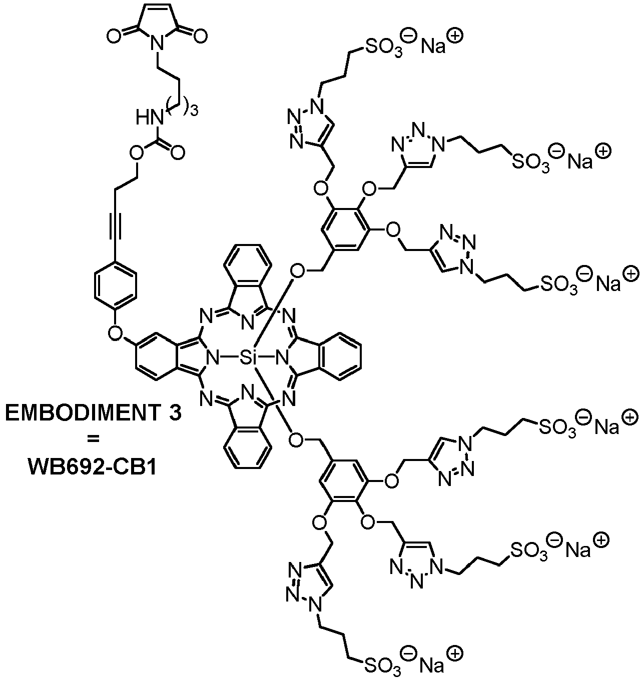

The silicon phthalocyanine **14** (58.7 mg, 24.8 *µ*mol) and the isocyanate **22** (23.0 mg, 110 *µ*mol, 4.46 equiv.) were dissolved in DMF (2.0 mL). MoO₂Cl₂(DMF)₂ (1.0 mg, 2.9 *µ*mol, 12 mol-%,) was added. The resulting mixture was stirred at room temp. for 3 h. Additional amounts of isocyanate **22** (26.0 mg, 125 *µ*mol, 5.04 equiv.) and MoO₂Cl₂(DMF)₂ (3.0 mg, 8.7 *µ*mol, 35 mol-%) were added. Stirring at room temp. was continued for 16 h. An aqueous solution of Nal [10% (w:w), 2 mL] was added. The resulting mixture was added to pure acetone (12 mL) forming a precipitate. It was concentrated as a pellet by centrifugation. The supernatant liquid was decanted. The remaining pellet was washed with acetone (8 mL). It was purified by flash chromatography on "C₁₈ reversed-phase silica gel" [Ø = 2.0 cm, h = 15 cm, F = 20 mL, 25 mL/min, 10 mM HNEt₃^{⊕⊖}OAc (which buffered at pH = 7.0), H₂O:MeCN 80:20 → 70:30 in 17 min]. The fractions (F13-F20) were concentrated under reduced pressure. The resulting solution was freeze-dried. The residue was dissolved in an aqueous solution of Nal [10% (w:w), 1 mL]. The resulting solution formed a precipitate when it was added to pure acetone (12 mL). This precipitate was concentrated as a pellet by centrifugation. The supernatant liquid was decanted. The remaining pellet was washed with acetone. It was dissolved in H₂O whereupon freeze-drying delivered the title compound **(EMBODIMENT 3** = **WB692-CB1)** as a green solid (7.5 mg, 2.9 *µ*mol, 12%).

**HRMS** (neg. ion mode ESI): [M - 6 x Na^{⊕}] observed as [¹²C₁₀₂¹H₉₈¹⁴N₂₈¹⁶O₃₁³²S₆²⁸Si]^{6⊖} (tantamount to z = 6) with *m*/*z* = 405.0848 which deviates from the calculated *m*/*z* = 405.0847 by +0.31 ppm.

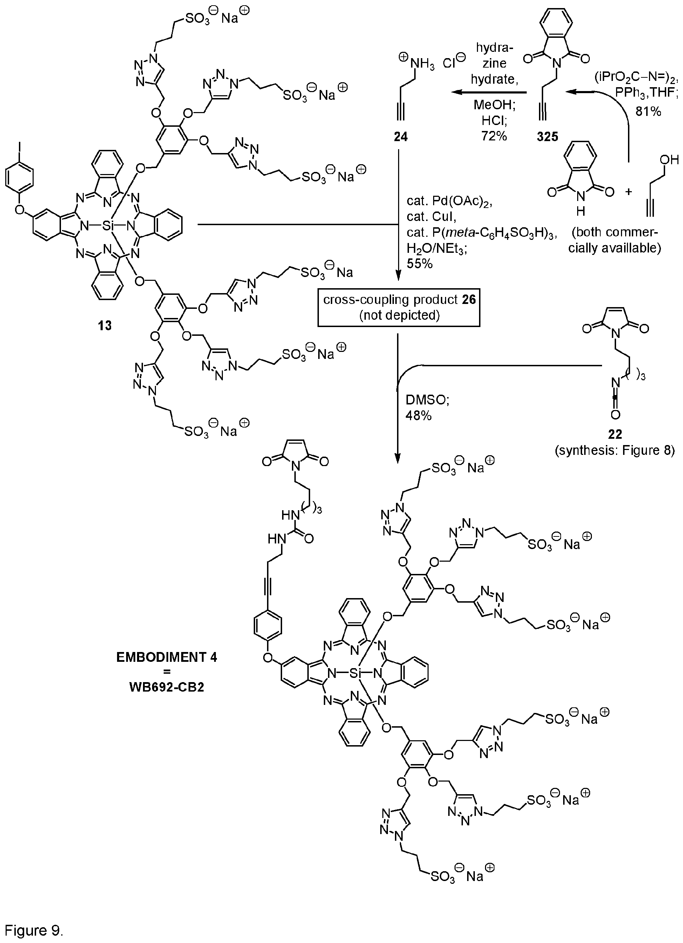

### 1.10 Synthesis of EMBODIMENT 4 (= silicon phthalocyanine WB692-CB2; the corresponding reactions are visualized - or referred to - in Figure 9)

### Preparation 22: 2-(But-3-yn-1-yl)isoindoline-1,3-dione (25)

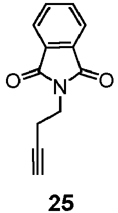

Phthalimide (1.946 g, 13.23 mmol) and PPh₃ (3.47 g, 13.2 mmol, 1.0 equiv.) were dissolved in THF (60 mL). But-3-yn-1-ol (1.00 mL, 0.927 g, 13.2 mmol, 1.0 equiv.) and diisopropyl azodicarboxylate (2.60 mL, 2.67 g, 13.2 mmol, 1.00 equiv.) was added dropwise. The resulting mixture was stirred for 3 d at room temperature. All volatiles were removed under reduced pressure. The solid residue was suspended in CH₂Cl₂ (10 mL). The resulting suspension was applied to flash chromatography on silica gel [QJ = 4.0 cm, h = 20 cm, F = 50 mL, c-C₆H₁₂:EtOAc 17:83, from F18 25:75]. F10-16 delivered the title compound (**25**) as a colorless solid (2.144 g, 10.76 mmol, 81%).

### Preparation 23: But-3-yn-1-amin hydrochloride (24)

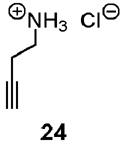

Phthalimid **25** was dissolved in MeOH (12 mL). Hydrazine hydrate (0.16 mL, 0.17 g, 3.3 mmol, 1.3 equiv.) was added dropwise. The resulting mixture was stirred for 24 h. A solid precipitated. It was filtrated and rinsed off with MeOH (8 mL). The filtrate was acidified with 1M HCl until pH = 1 was reached. A solid precipitated. It was collected by centrifugation. The solid was suspended in H₂O (10 mL) and the resulting suspension was filtrated. The filtrate was evaporated under reduced pressure to deliver the title compound (**24**) as a slightly yellow solid (0.192 g, 1.82 mmol, 72%).

### Preparation 24: 2-[4-(4-Aminobut-1-ynyl)phenoxy]-29,31-bis[(3,4,5-tris{[1-(sodium 3-sulfonatopropyl)-1,2,3-triazol-4-yl]methoxy}phenyl)methoxy]silanediyl-29H,31H-silicon phthalocyanine (26)

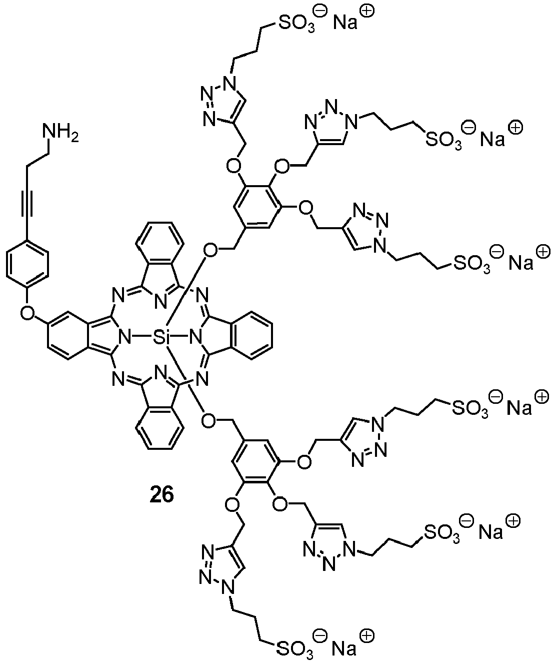

The silicon phthalocyanine **13** (0.080 g, 33.06 *µ*mol) and (*meta*-NaO₃S-H₄C₆)₃P (4.7 mg, 8.3 *µ*mol, 25 mol-%) were dissolved in degassed H₂O (3 mL). Pd(OAc)₂ (16 mM in MeCN, 0.10 mL, 1.7 *µ*mol, 5 mol-%), Cul (16 mM in MeCN, 0.1 mL, 1.7 *µ*mol, 5 mol-%), and NEt₃ (0.4 mL) were added. A solution of But-3-in-1-amin hydrochlorid (34.9 mg, 331 µmol, 10 equiv.) in degassed H₂O (1.0 mL) was added dropwise during 5 h. The resulting mixture was stirred at room temp. for 2.5 h. The resulting mixture was added dropwise to a solution of Nal in acetone (8 mL). A precipitate formed. It was concentrated as a pellet by centrifugation. The supernatant liquid was decanted. The remaining pellet was purified by flash chromatography on "C₁₈ reversed-phase silica gel" [QJ = 2.5 cm, h = 12 cm, F = 20 mL, 25 mL/min, 10 mM HNEt₃^{⊕⊖}OAc (which buffered at pH = 7.0), H₂O:MeCN 90:10 → 85:15 in 2 min, H₂O:MeCN 85:15 → 75:25 in 15 min, H₂O:MeCN 75:25 → 70:30 in 3 min, H₂O:MeCN 70:30 → 0:100 in 2 min]. The collected fractions (F12-14) and (F15-19) were freeze dried. The resulting residue of the collected fractions (F12-14) was dissolved in a minimum amount H₂O and the resulting solution was added to a saturated solution of Nal in acetone (8 mL). A precipitate formed. It was concentrated as a pellet by centrifugation. The supernatant liquid was decanted and the residue washed with acetone (2 × 10 mL). It was dissolved in H₂O and freeze dried to deliver one portion of the title compound (**26**) as a green solid (0.0171 g, 0.00724 mmol, 22%). The residue of the collected fractions (F15-19) was purified again by flash chromatography on "aqueous C₁₈ reversed-phase silica gel" [QJ = 2.5 cm, h = 12 cm, F = 20 mL, 25 mL/min, 10 mnn HNEt₃^{⊕⊖}OAc (which buffered at pH = 7.0), H₂O:MeCN 100:0 → 90:10 in 2 min, H₂O:MeCN 90:10 → 80:20 in 10 min, H₂O:MeCN 80:20 → 70:30 in 5 min, H₂O:MeCN 70:30 → 0:100 in 5 min]. The collected fractions (F9-10) were freeze dried. The resulting residue was dissolved in a minimum amount H₂O and the resulting solution was added dropwise to a saturated solution of Nal in acetone (8 mL). A precipitate formed. It was concentrated as a pellet by centrifugation. The supernatant liquid was decanted and the residue was washed with acetone (2 × 10 mL) and dissolved in H₂O. The aqueous solution was freeze dried to deliver a second portion of the title compound (**26**) as a green solid (0.0258 g, 0.0109 mmol, 33% combined yield: 55%).

### Preparation 25: 2-[({5-[(1H-2,5-Dioxopyrrol-1-yl)pent-1-yl]amino]carbonylamino)}but-1-yn-yl)phenoxy]-29,31-bis[(3,4,5-tris{[1-(sodium 3-sulfonatopropyl)-1,2,3-triazol-4-yl]methoxy}-phenyl)methoxyjsilanediyl-29H,31H-silicon phthalocyanine (EMBODIMENT 3 = WB692-CB2)

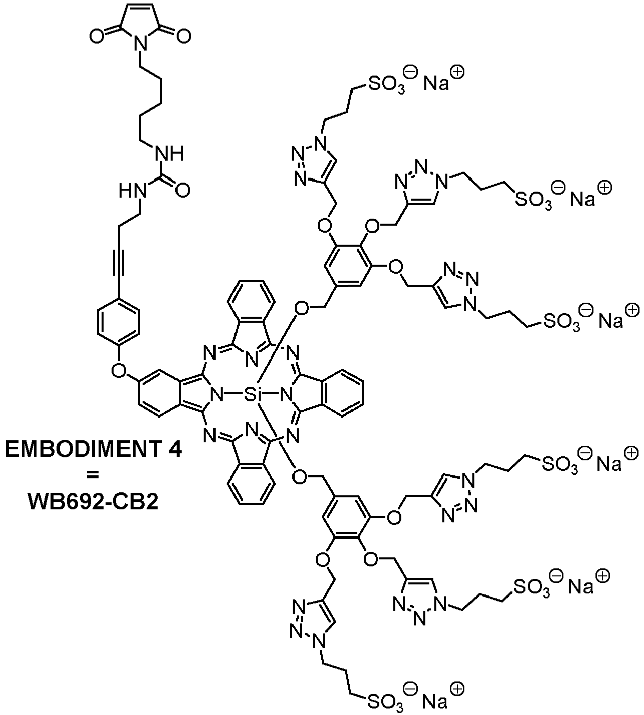

The silicon phthalocyanine **26** (25.0 mg, 10.58 *µ*mol) was dissolved in dimethylsulfoxide (0.4 mL). A solution of isocyanate **22** (34.0 mg, 163 *µ*mol, 15 equiv.) in dimethylsulfoxide (0.2 mL) was added. The resulting mixture was stirred for 9 h at room temperature. The reaction mixture was added dropwise in a saturated solution of Nal in acetone (8 mL). A precipitate formed. Which was collected by centrifugation. The resulting pellet was triturated with a mixture of acetone (10 mL) and H₂O (0.8 mL) and then concentrated again by centrifugation. The supernatant liquid was decanted. The residue was purified by flash chromatography on "aqueous C₁₈ reversed-phase silica gel" [QJ = 2 cm, h = 12 cm, F = 18 mL, 20 mL/min, 10 mnn HNEt₃^{⊕⊖}OAc (which buffered at pH = 7.0), H₂O:MeCN 95:5 → 80:20 in 4 min, H₂O:MeCN 80:20 → 75:25 in 15 min, H₂O:MeCN 75:25 → 70:30 in 5 min, H₂O:MeCN 70:30 → 0:100 in 4 min]. The collected fractions (F12-16) were freeze dried. The resulting residue was dissolved in a minimum amount H₂O. The resulting solution was added dropwise in a saturated solution of NaI in acetone (8 mL). A precipitate formed. It was collected by centrifugation. The pellet was washed with acetone (2 × 10 mL). It was dissolved in H₂O whereupon freeze-drying delivered the title compound **(EMBODIMENT 4 = WB692-CB2)** as a green solid (13 mg, 5.1 *µ*mol, 48%).

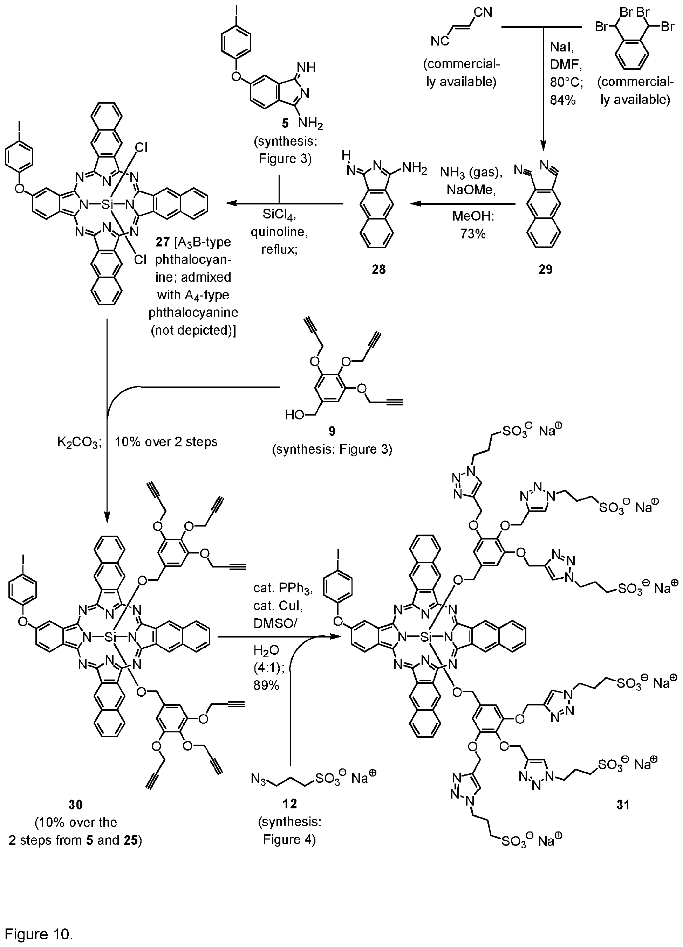

### 1.11 Synthesis of EMBODIMENT 5 (= silicon phthalocyanine WB795-LB1; the corresponding reactions are visualized in Figures 10 - 11)

### Preparation 26: But-3-yn-1-yl 1H-imidazole-1-carboxylate (33)

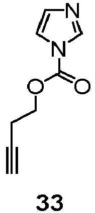

Carbonyl-N,N'-diimidazol (3.216 g, 19.84 mmol, 1.50 equiv.) was dissolved in THF (100 mL). The resulting solution was cooled to 0°C. A solution of But-3-yn-1-ol (1.00 mL, 0.927 g, 13.2 mmol) in CH₂Cl₂ (20 mL) was added over a period of 1 h. The reaction mixture was warmed to room temp. and was stirred for 3 h. All volatiles were removed under reduced pressure. The resulting residue was purified by silica column chromatography [QJ = 4 cm, h = 17 cm, F = 50 mL, c-C₆H₁₂:EtOAc → 75:25] which delivered the title compound (33; 1.963 g, 11.96 mmol, 90%, F9-18) as a colorless solid.

### Preparation 27: Naphthalene-2,3-dicarbonitrile (29)

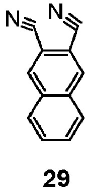

*alpha,alpha,alpha',alpha'*-Tetrabromo-o-xylene (20.00 g, 47.42 mmol), fumaronitrile (7.404 g, 94.84 mmol, 2.0 equiv) and NaI (15.64 g, 104.3 mmol, 2.2 equiv.) were dissolved in DMF (200 mL). The resulting mixture was stirred at 80°C for 21 h. It was cooled to room temperature and poured onto ice water and saturated aqueous Na₂SO₃-solution (≈250 mL, v:v = 1:1). The precipitate was filtrated, washed with EtOAc (3 × 20 ml) and H₂O (100 mL). The residue was dried under vacuum over KOH for 2 d. Which delivered the title compound (**29;** 7.174 g, 40.26 mmol, 84%). The product crystallizes from EtOH:Aceton (v:v, 2:1) as slightly brown needles.

### Preparation 28: 1-Imino-1H-benzo[f]isoindol-3-amine (28)

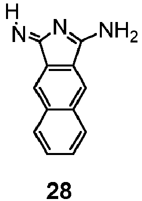

Naphthalodinitrile **29** (3.00 g, 16.8 mmol) was suspended in MeOH (100 mL). NaOMe (0.636 g, 11.8 mmol, 0.7 equiv.) was addded. The resulting mixture was heated under reflux and a solution of NH₃ in MeOH (7nn, 20 mL) was added. The reaction mixture was stirred under reflux for 2.5 h. All volatiles were removed under reduced pressure. H₂O (100 mL) was added. The precipitate was filtrated, washed with H₂O (2 × 20 mL) and acetone (3 × 10 mL). The residue was dried under vaccuum to deliver the title compound (**28;** 2.42 g, 12.4 mmol, 73%) as a slightly brown solid.

### Preparation 29: 29,31-(Dichlorosilanediyl)-2-(4-iodophenoxy)-29H,31H-silicon tribenzophthalocyanine (27) and 29,31-Bis{[3,4,5-tris(prop-2-yn-1-yloxy)phenyl]methoxy}silanediyl-2-(4-iodophenoxy)-29H,31H-silicon tribenzophthalocyanine (30)

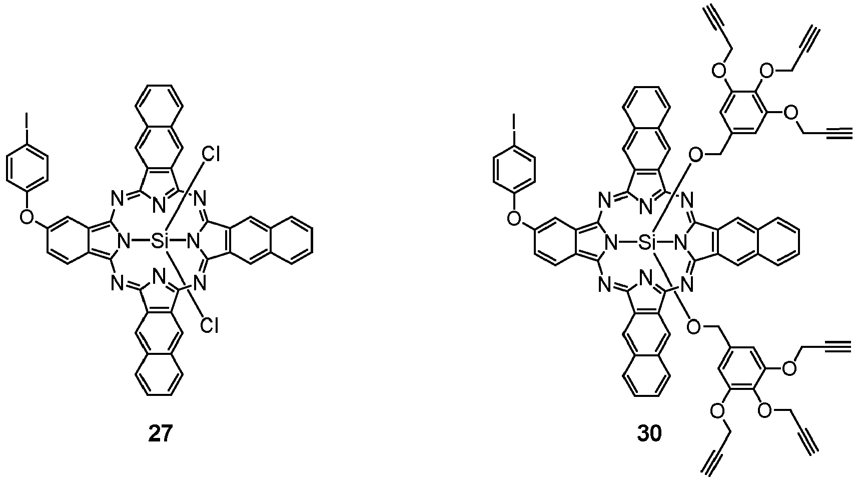

The diiminoisoindolines **5** (0.5 g, 1.38 mmol) and **28** (2.15 g, 11.0 mmol, 8.0 equiv.) were suspended in quinoline (15 mL). Under an atmosphere of N₂, SiCl₄ (1.40 mL, 2.07 g, 12.2 mmol, 8.85 equiv.) was added dropwise. The resulting mixture was heated at 200°C for 1 h. The reaction mixture was cooled to room temp. and acetone (50 mL) was added. The resulting mixture was filtrated. The residue was washed with acetone (20 mL), MeOH (10 mL), CH₂Cl₂ (20 mL) and acetone (3 × 10 mL). This delivered a black solid, which are the raw Dichloro Si-Phthalocyanines as a mixture containing A₃B and A₄ (2.7031 g); it contained the expected mixture of the monosubstituted, i. e. A₃B-type silicon phthalocyanine **27** (desired) and its unsubstituted analog 29,31-Bis{[3,4,5-tris(prop-2-yn-1-yloxy)phenyl]methoxy}silanediyl-29*H*,31*H*-silicon tetrabenzophthalocyanine, i. e. the A₄-type parent-silicon phthalocyanine (undesired, not depicted).

An aliquot of this crude mixture (1.2031 g), K₂CO₃ (2.00 g, 14.47 mmol) and the benzyl alcohol **9** (2.79 g, 10.3 mmol) were suspended in toluene (50 mL). The resulting mixture was heated under reflux for 8 h. It was cooled to room temperature and it was filtrated over silica with c-C₆H₁₂ as solvent. The filter cake was rinsed off with c-C₆H₁₂:EtOAc (v:v = 76:24) until the dark green color disappeared. All volatiles were removed under reduced pressure. The residue was purified by silica column chromatography [QJ = 4 cm, h = 22 cm, F = 50 mL, c-C₆H₁₂:EtOAc → 76:24] which delivered the title compound (**30;** 78.5 mg, 54.2 µmol, 9% yield over 2 steps) as dark green solid.

### Preparation 30: 2-(4-Iodophenoxy)-29,31-bis[(3,4,5-tris{[1-(sodium 3-sulfonatopropyl)-1,2,3-triazol-4-yl]methoxy}phenyl)methoxy]silanediyl-29H,31H-silicon tribenzophthalocyanine (31)

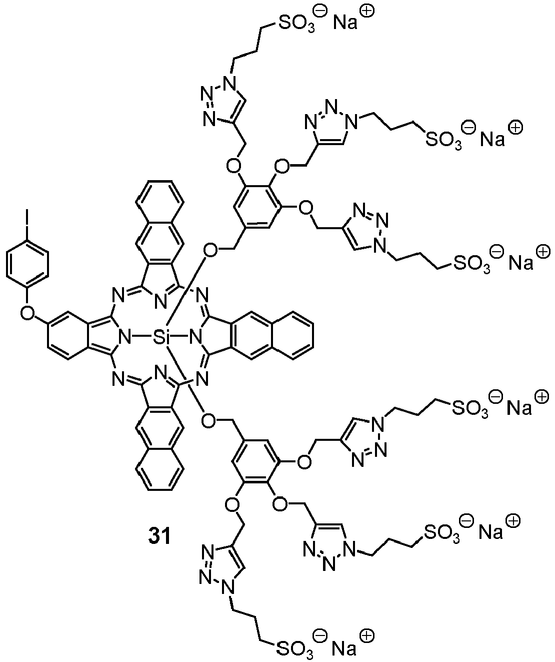

The silicon phthalocyanine **30** (200 mg, 138 µmol), sodium 3-azidopropane-1-sulfonate (**12**) (206.8 mg, 1.105 mmol, 8.0 equiv.), PPh₃ (23.9 mg, 91.2 µmol, 66 mol-%) and Cul (15.8 mg, 82.9 µmol, 60 mol-%) were dissolved in DMSO (2 mL). NEt₃ (0.6 mL) was added. This mixture was stirred for 8 h. The reaction mixture was added dropwise to a saturated solution of NaI in acetone (15 mL). The resulting precipitate was centrifuged, the supernatant liquid was removed. The residue was washed with acetone (2 × 10 mL). The resulting residue was purified by C18 reversed phase flash chromatography [QJ = 2.0 cm, h = 15 cm, F = 20~mL, 25 mL/min, 10 mM HNEt₃^{⊕⊖}OAc (which buffered at pH = 7.0), H₂O:MeCN → 80:20 in 2 min, 80:20 → 70:30 in 20 min, 70:30 → 65:35 in 5 min, 65:35 → 20:80 in 3 min]. The collected fractions (F20-25) were freeze dried. The residue was dissolved in H₂O and the resulting solution was added dropwise to a saturated NaI solution in acetone (15 mL). The resulting precipitate was centrifuged. The supernatant liquid was removed. The residue was washed with acetone (10 mL), dissolved in H₂O (10 mL) and freeze dried to deliver a first portion of the title compound (**31;** 0.2718 g, 0.1057 mmol, 76%) as a dark green solid. The collected fractions (F26-32) were freeze dried and the residue was purified by a second aqueous C4 reversed phase flash chromatography [QJ = 2.0~cm, h = 15 cm, F = 20 mL, 25 mL/min, 10 mM HNEt₃^{⊕⊖}OAc (which buffered at pH = 7.0), H₂O:MeCN = 100:0 → 90:10 in 2 min, 90:10 → 85:15 in 8 min, 85:15 → 80:20 in 10 min, 80:20 → 30:70 in 10 min]. The collected fractions (F39-45) were freeze dried. The residue was dissolved in a minimum amount of H₂O. The resulting solution was added dropwise to a saturated solution of NaI in acetone (8 mL). A precipitate formed. It was concentrated by centrifugation. The supernatant liquid was decanted and the residue washed with acetone (2 × 10 mL). It was dissolved in H₂O and freeze dried to deliver a second portion of the title compound (**31;** 0.0477 g, 18.6 µmol, 13% combined yield 89%).

### Preparation 31: 2-[4-({[(3-Carboxypropyl)amino]carbonyloxy}but-1-ynyl)phenoxy]-29,31-bis[(3,4,5-tris{[1-(sodium 3-sulfonatopropyl)-1,2,3-triazol-4-yl]methoxy}phenyl)methoxy]-silanediyl-29H,31H-silicon phthalocyanine (34)

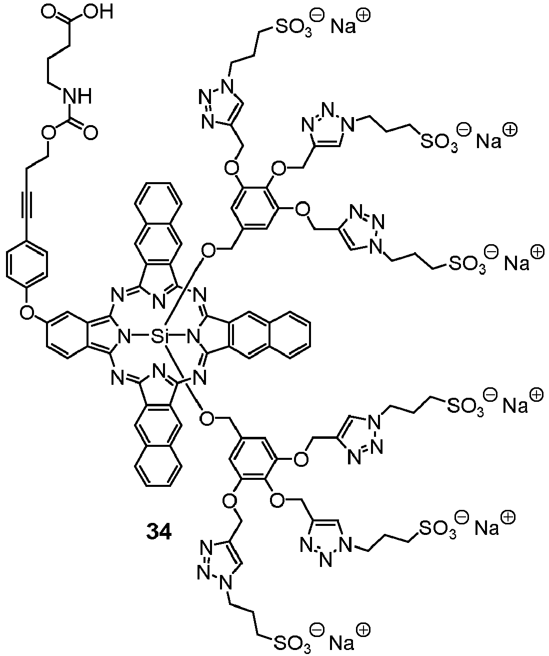

The imidazolide **33** (95.80 mg, 583.6 µmol, 10 equiv.) was dissolved in degassed dimethylsulfoxide (0.5 mL). A solution of 4-aminobutyric acid (66.2 mg, 642 µmol, 11.0 equiv.) and NaOH (25.6 mg, 642 µmol, 11.0 equiv.) in degassed H₂O (0.5 mL) was added. The resulting mixture was stirred for 1 h at room temperature.

The silicon phthalocyanine **31** (0.150 g, 58.4 *µ*mol) and (*meta*-NaO₃S-H₄C₆)₃P (8.29 mg, 14.6 *µ*mol, 25 mol-%) were dissolved in degassed H₂O (3 mL). Pd(OAc)₂ (29 mM in MeCN, 0.1 mL, 2.9 *µ*mol, 5 mol-%), Cul (29 mM in MeCN, 0.1 mL, 2.9 *µ*mol, 5 mol-%), and NEt₃ (1 mL) were added. The before mentioned reaction mixture was added dropwise during 5 h. The resulting mixture was stirred at room temp. for 24 h. The resulting mixture was added dropwise to a saturated solution of NaI in acetone (15 mL). A precipitate formed. It was concentrated as a pellet by centrifugation. The supernatant liquid was decanted. The residue was washed with acetone (1 × 10 mL). The remaining pellet was purified by flash chromatography on "aqueous C₄ reversed-phase silica gel" [Ø = 2.0 cm, h = 15 cm, F = 19 mL, 25 mL/min, 10 mnn HNEt₃^{⊕⊖}OAc (which buffered at pH = 7.0), H₂O:MeCN 100:0 → 90:10 in 2 min, H₂O:MeCN 90:10 → 80:20 in 8 min, H₂O:MeCN 80:20 → 70:30 in 15 min, H₂O:MeCN 70:30 → 20:80 in 10 min]. The collected fractions (F18-28) were freeze dried. The resulting residue was dissolved in a minimum amount of water and added dropwise to a saturated solution of NaI in acetone (15 mL). A precipitate formed. It was centrifuged, washed with acetone (2 × 10 mL) and dissolved in H₂O. The solution was freeze-dried to deliver the title compound (**34;** 0.1217 g, 0.0457 mmol, 78%) as a green solid.

### Preparation 32: 2-({[({4-[(2,5-Dihydro-1H-2,5-dioxopyrrol-1-yl)oxy]-4-oxobut-1-yl}amino)-carbonyloxy]but-1-ynyl}phenoxy)-29,31-bis[(3,4,5-tris{[1-(sodium 3-sulfonatopropyl)-1,2,3-tri-azol-4-yl]methoxy}phenyl)methoxy]silanediyl-29H,31H-silicon tribenzophthalocyanine (EMBODIMENT 5 = WB795-LB1)

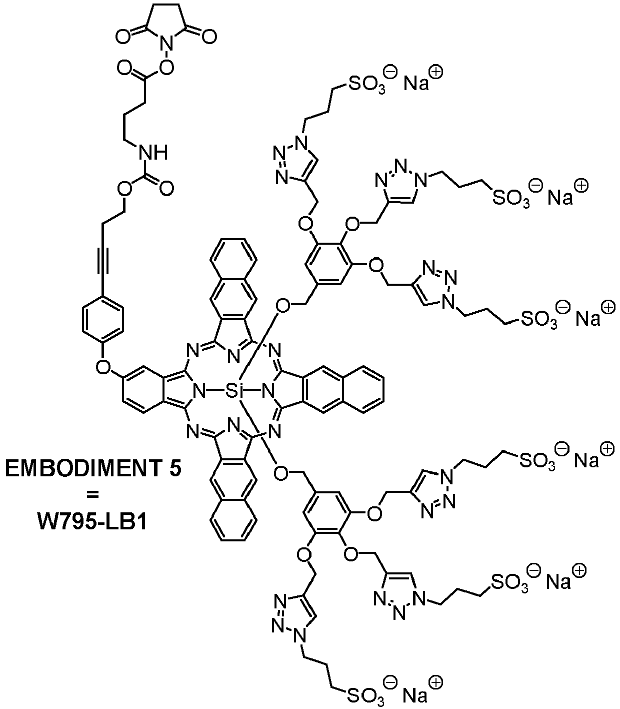

The silicon phthalocyanine **34** (20.0 mg, 7.57 *µ*mol) was dissolved in dimethylsulfoxide (0.4 mL) and pyridine (0.2 mL) was added. The resulting mixture was stirred for 10 min. Disuccinimidyl carbonate (19.4 mg, 75.7 *µ*mol, 10 equiv.) was added. The resulting mixture was heated at 60°C for 6 h. The reaction mixture was cooled to room temp. and added dropwise to a saturated solution of NaI in acetone (8 mL). This caused the reaction product to precipitate. It was concentrated as a pellet by centrifugation. The supernatant liquid was decanted. The remaining pellet was washed with acetone (2 × 10 mL). All volatiles were removed under reduced pressure to deliver the title compound (**EMBODIMENT 5 = WB795-LB1**) as a green solid (20 mg, 7.30 µmol, 96%).

**HRMS** (neg. ion mode ESI): [M - 6 × Na^{⊕}] observed as [¹²C₁₁₃¹H₁₀₂¹⁴N₂₈¹⁶O₃₃³²S₆²⁸Si]^{6⊖} (tantamount to z = 6) with *m*/*z* = 433.2552 which deviates from the calculated *m*/*z* = 433.2561 by -2.2805 ppm.

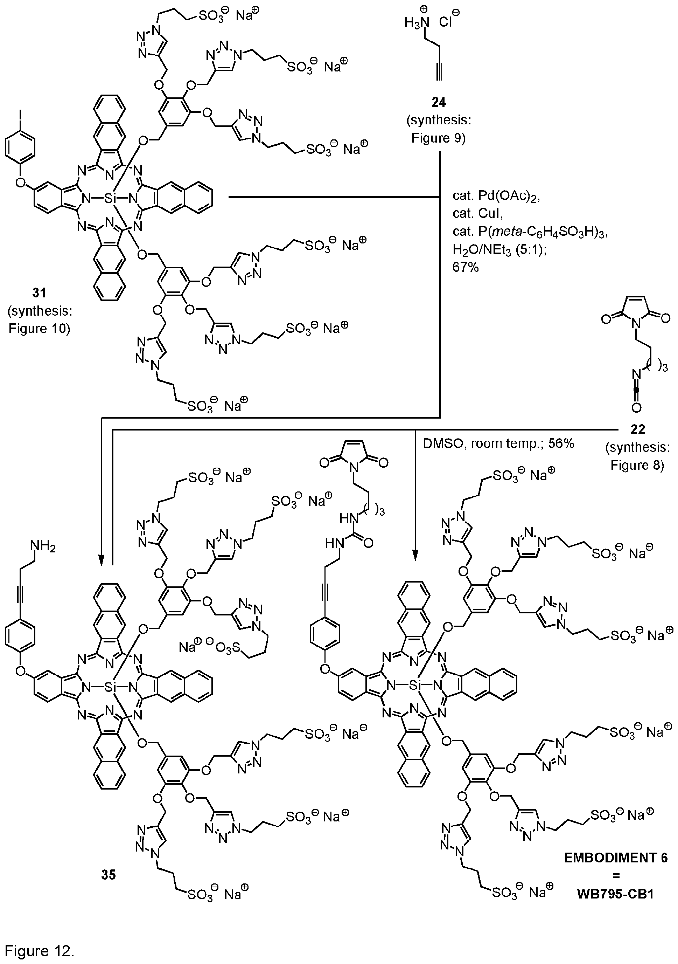

### 1.12 Synthesis of EMBODIMENT 6 (= silicon phthalocyanine WB795-CB1; the corresponding reactions are visualized - or referred to - in Figure 12)

### Preparation 33: 2-[4-(4-Aminobut-1-ynyl)phenoxy]-29,31-bis[(3,4,5-tris{[1-(sodium 3-sulfonatopropyl)-1,2,3-triazol-4-yl]methoxy}phenyl)methoxy]silanediyl-29H,31H-silicon tribenzophthalocyanine (35)

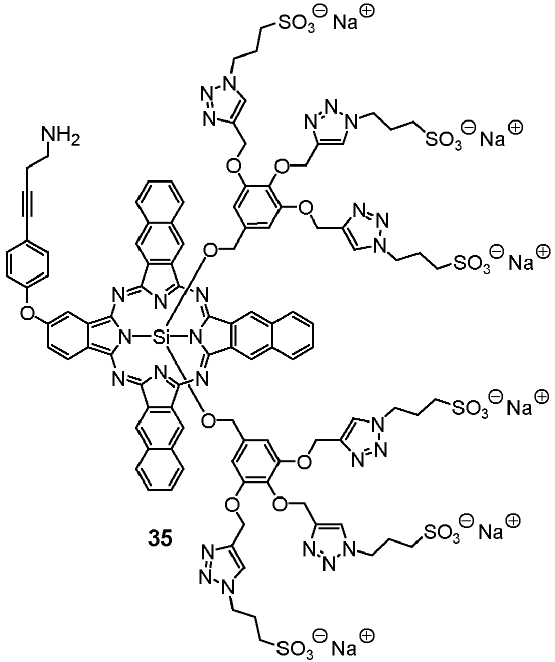

The silicon phthalocyanine (**31;** 80.0 mg, 31.1 µmol) and (*meta*-NaO₃S-H₄C₆)₃P (4.4 mg, 7.7 µmol, 25 mol-%) were dissolved in degassed H₂O (3 mL). Pd(OAc)₂ (16 mM in MeCN, 0.10 mL, 1.7 µmol, 5 mol-%), Cul (16 mM in MeCN, 0.1 mL, 1.7 µmol, 5 mol-%) and NEt₃ (0.4 mL) were added. A solution of But-3-in-1-amin hydrochlorid (32.8 mg, 311 µmol, 10 equiv.) in degassed H₂O (1.0 mL) was added dropwise over a period of 5 h. The resulting mixture was stirred at room temp. for 2.5 h. It was added dropwise to a saturated solution of NaI in acetone (8 mL). A precipitate formed. It was concentrated as a pellet by centrifugation. The supernatant liquid was decanted. The remaining pellet was purified by flash chromatography on "C18 reversed-phase silica gel" [QJ = 2.5 cm, h = 12 cm, F = 20 mL, 25 mL/min, 10 mM HNEt₃^{⊕⊖}OAc (which buffered at pH = 7.0), H2O:MeCN 90:10 → 85:15 in 2 min, H2O:MeCN 85:15 → 75:25 in 15 min, H2O:MeCN 75:25 → 70:30 in 3 min, H2O:MeCN 70:30 → 0:100 in 2 min]. The collected fractions (F17-24) were freeze dried. The resulting residue was dissolved in H₂O and the resulting solution was added to a saturated solution of NaI in acetone (8 mL). A precipitate formed. It was concentrated as a pellet by centrifugation. The supernatant liquid was decanted and the residue washed with acetone (2 × 10 mL). It was dissolved in H₂O and freeze dried to deliver the title compound (**35;** 0.0521 g, 0.0207 mmol, 67%) as a green solid.

### Preparation 34: 2-[({5-[(1H-2,5-Dioxopyrrol-1-yl)pent-1-yl]amino]carbonylamino)}but-1-yn-yl)phenoxy]-29,31-bis[(3,4,5-tris{[1-(sodium 3-sulfonatopropyl)-1,2,3-triazol-4-yl]methoxy}-phenyl)methoxy]silanediyl-29H,31H-silicon tribenzophthalocyanine (EMBODIMENT 6 = WB795-CB1)

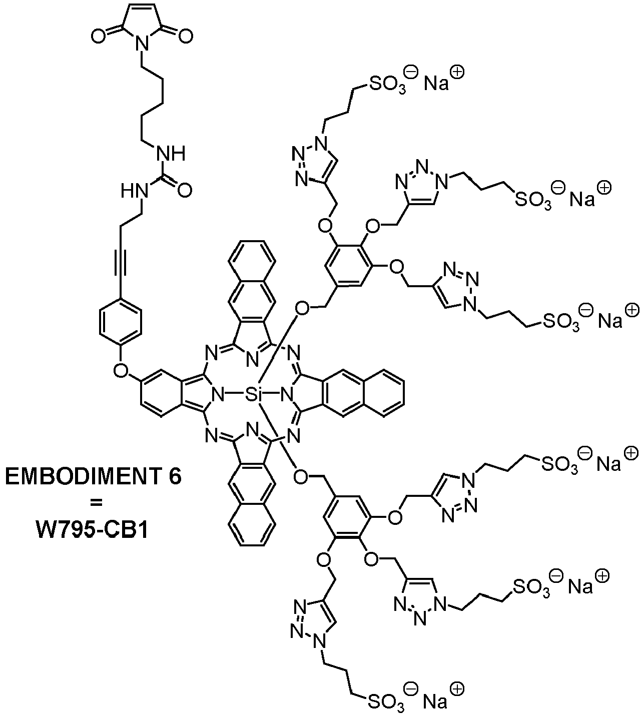

The silicon phthalocyanine **35** (25.0 mg, 9.95 *µ*mol) was dissolved in dimethylsulfoxide (0.4 mL). A solution of isocyanate (**22;** 34.0 mg, 163 *µ*mol, 15 equiv.) in dimethylsulfoxide (0.2 mL) was added. The resulting mixture was stirred for 9 h at room temperature. The reaction mixture was added dropwise in a saturated solution of NaI in acetone (8 mL). A precipitate formed. Which was collected by centrifugation. The resulting pellet was triturated with a mixture of acetone (10 mL) and H₂O (0.8 mL) and then concentrated again by centrifugation. The supernatant liquid was decanted. The residue was purified by flash chromatography on "aqueous C₁₈ reversed-phase silica gel" [QJ = 2 cm, h = 12 cm, F = 18 mL, 20 mL/min, 10 mnn HNEt₃^{⊕⊖}OAc (which buffered at pH = 7.0), H₂O:MeCN 95:5 → 80:20 in 4 min, H₂O:MeCN 80:20 → 75:25 in 15 min, H₂O:MeCN 75:25 → 70:30 in 5 min, H₂O:MeCN 70:30 → 0:100 in 4 min]. The collected fractions (F20-26) were freeze dried. The resulting residue was dissolved in a minimum amount H₂O. The resulting solution was added dropwise in a saturated solution of NaI in acetone (8 mL). A precipitate formed. It was collected by centrifugation. The pellet was washed with acetone (2 × 10 mL). It was dissolved in H₂O whereupon freeze-drying delivered the title compound (**EMBODIMENT 6 = WB795-CB1**) as a green solid (15.1 mg, 5.55 *µ*mol, 56%).

**HRMS** (neg. ion mode ESI): [M - 6 × Na^{⊕}] observed as [¹²C₁₁₀¹H₁₀₆¹⁴N₂₉¹⁶O₃₃³²S₆²⁸Si]^{6⊖} (tantamount to z = 6) with *m*/*z* = 430.0934 which deviates from the calculated *m*/*z* = 430.0939 by -1.2088 ppm.

### 1.13 Cell culture

The human prostate carcinoma cell lines LNCaP, PC3, PC3-PSMA and C4-2 as well as the bladder cancer cell line RT112 were purchased from the American Type Culture Collection (ATCC, Manassas, USA). The colon cancer cell line HCT-116 was purchased from Merk/Sigma Aldrich. The PC3-PSMA cell line was kindly provided by P. Giangrande, University of Iowa, USA. The human breast cancer cell line SK-BR3 was kindly provided by T. Erbes, University Medical Center, University of Freiburg, Germany. Cell line identity was verified by short tandem repeat (STR) analysis (CLS GmbH, Eppelheim, Germany). PC3- and PC3-PSMA cells were routinely propagated in complete F-12 medium (Thermo Fischer Scientific, Waltham, USA), LNCaP and C4-2 cells in RMPI 1640 medium (Thermo Fischer Scientific, Waltham, USA) and SKBR-3 and RT112 cells in DMEM medium (Thermo Fischer Scientific, Waltham, USA), and HCT-116 cells in MSCB medium (Cell systems) all supplemented with 10% fetal bovine serum (Sigma-Aldrich, St. Louis, USA) and 1% penicillin/streptomycin (Sigma-Aldrich, St. Louis, USA). All cells were cultured at 37°C and 5% CO₂.

### 1.14 Western Blot Analysis

Antigen expression was confirmed by Western Blot Analysis. In brief, target cells were lysed in RIPA buffer supplemented with 1X cOmplete^{™} Protease Inhibitor Cocktail (Roche, Basel, Switzerland). Protein concentration was determined by using the QuickStart Bradford Protein Assay (Bio-Rad Laboratories, Inc., Hercules, USA). For detection of PSMA or HER2, the monoclonal mouse anti-PSMA antibody AK7 (17) or the monoclonal rabbit anti-HER2/ErbB2 antibody (Cell Signaling Technology, Danvers, USA) was used, respectively. HRP-conjugated polyclonal rabbit anti-mouse or goat anti-rabbit antibodies (DAKO by Agilent Technologies, Santa Clara, USA) were used as secondary antibodies. β-Actin was used as a loading control and was detected with help of the HRP-conjugated polyclonal rabbit anti-β-Actin antibody (Cell Signaling Technologies, Leiden, Netherlands). Western Blots were developed with an enhanced chemiluminescence (ECL) substrate (Bio-Rad Laboratories, Inc., Hercules, USA) and protein bands were detected using the ChemiDoc MP Imaging System with the Image Lab software (Bio-Rad Laboratories, Inc., Hercules, USA).

### 1.15 Cloning, expression and purification of the recombinant antibodies

For generation of recombinant IgG1 antibodies, the variable domains of the light (VL) and heavy chains (VH) were amplified from existing DNA vectors (provided by collaboration partners or synthesized by GeneArt, Thermo Fischer Scientific, Waltham, USA) by PCR using primers containing the corresponding restriction sites. The amplified VLs and VHs were cloned into the vectors pCSL3k and pCSEH1c, respectively, the latter containing the mutated cysteine residues.

For enzymatic digestion of the heavy and light chain vectors and inserts, the restriction endonucleases BssHll and Nhel from New England Biolabs (Ipswich, USA) and Agel and Xhol from Promega (Madison, USA) were used, respectively. Reaction buffers and incubation temperatures were chosen according to the manufacturer's instructions. For gel extraction, the QIAquick Gel Extraction Kit (Qiagen, Hilden, Germany) was used. Ligation was performed in a molar ratio of 1:3 (vector:insert) using the Rapid DNA Ligation Kit (Roche, Basel, Switzerland). The transformation of the XL1-Blue MRF' Supercompetent bacteria (Agilent Technologies, Santa Clara, USA) was performed as described in the manufacturer's protocol. The bacteria were plated on LB agar plates (Sigma-Aldrich, St. Louis, USA) containing 0.1 mg/ml ampicillin (Sigma-Aldrich, St. Louis, USA) and single clones were transferred into liquid LB medium (Sigma-Aldrich, St. Louis, USA) containing 0.1 mg/ml ampicillin (Sigma-Aldrich, St. Louis, USA). Plasmid DNA was prepared from transformed bacteria using the NucleoSpin Plasmid Kit (Macherey-Nagel, Düren, Germany). DNA was eluted in ddH₂O and concentration was determined by using the NanoDrop Lite Spectrophotometer (Thermo Fischer Scientific, Waltham, USA). Correct sequences of the plasmids were verified by sequencing (Microsynth, Balgach, Switzerland).

EXPI293F cells (Thermo Fischer Scientific, Waltham, USA) were cultured in serum-free FreeStyle F17 medium supplemented with 8 mM glutamine (PAN-Biotech, Aidenbach, Germany) and 0.1% pluronic F68 (PAN-Biotech, Aidenbach, Germany) at 37°C, 110 rpm and 5% CO₂. Two days before transient transfection, EXPI293F cells were seeded at 0.5*10⁶ cells/ml, reaching ~ 1.5-2.0*10⁶ cells/ml (> 90% viability) on the day of transfection. For DNA:PEI complex formation, 1 µg DNA/ml transfection volume and 5 µg of 40 kDa PEI (Polysciences, Warrington, USA) were first diluted separately in 5% transfection volume in supplemented Free-Style F17 media. DNA (1:1 ratio of heavy and light chain vector) and PEI was then mixed and incubated 30 min at room temperature before addition to the cells. 48 h later, the culture volume was doubled by feeding HyClone SFM4Transfx-293 media (Cytiva, Marlborough, USA) supplemented with 8 mM glutamine and 20% HyClone Boost 6 supplement (Cytiva, Marlborough, USA). One week after transfection, supernatant was harvested by centrifugation (15 min, 1500 × g) and antibodies were purified using a protein G affinity chromatography column (Cytiva, Marlborough, USA). Successful purification of the antibodies was verified by Coomassie staining (Protein Ark, Sheffield, UK) and Western Blot using a HRP conjugated polyclonal rabbit anti-human IgG antibody (DAKO by Agilent Technologies, Santa Clara, USA) for detection. Protein concentration was determined by using the Pierce BCA Protein Assay Kit (Thermo Fischer Scientific, Waltham, USA).

### 1.16 Conjugation of recombinant antibodies bearing engineered cysteine residues with thiol reactive dyes

For conjugation, antibodies were first adjusted with 1xPBS containing 1 mM EDTA (pH 7.4) (Serva Electrophoresis GmbH, Heidelberg, Germany) and then reduced with 40 equimolar TCEP (Carl Roth, Karlsruhe, Germany) for 3 hours at 37°C. After dialysis in 1xPBS, 1mM EDTA (pH 7.4) using the Slide-A-Lyzer Dialysis Cassettes (20K MWCO, Thermo Fischer Scientific, Waltham, USA) at 4 °C overnight, the antibodies were re-oxidized with 30 equimolar dehydroascorbic acid (Sigma-Aldrich, St. Louis, USA) for 4h at room temperature. For conjugation, the antibodies were incubated with 10 × equimolar dye at room temperature for 1 hour. To quench the reaction, 25 equimolar N-acetyl-L-cysteine (Sigma-Aldrich, St. Louis, USA) was added (15 min, room temperature), followed by dialysis against 1xPBS (pH 7.4) overnight (4°C). Unbound dye was removed by protein G affinity chromatography (Cytiva, Marlborough, USA) and dialysis against PBS. Successful conjugation and purification of the conjugates was verified by reducing or non-reducing SDS-PAGE using red (λₘₐₓ = 680 nm) or NIR (λmax = 800 nm) fluorescence-based imaging (IVIS Spectrum, In Vivo Imaging System, PerkinElmer, Waltham, USA) and Coomassie staining (Protein Ark, Sheffield, UK). Protein concentration was determined by using the Pierce BCA Protein Assay Kit (Thermo Fischer Scientific, Waltham, USA). The degree of labeling was calculated according to the TechTip#31 protocol "Calculate dye:protein (F/P) molar ratios" (Thermo Fischer Scientific, Waltham, USA).

### 1.17 Conjugation of recombinant antibodies with amine reactive dyes

Recombinant antibodies were incubated with 10 × equimolar dye in 0.1 M Na₂HPO₄ (pH 8.5) at room temperature for 2 h. Unbound dye was removed by protein G affinity chromatography purification (Cytiva, Marlborough, USA) and dialysis. Successful conjugation and purification of the conjugates was verified by reducing or non-reducing SDS-PAGE using red or NIR fluorescence-based imaging (IVIS Spectrum In Vivo Imaging System, PerkinElmer, Waltham, USA) and Coomassie staining (Protein Ark, Sheffield, UK). Protein concentration was determined by using the Pierce BCA Protein Assay Kit (Thermo Fischer Scientific, Waltham, USA). The degree of labeling was calculated according to TechTip#31 "Calculate dye:protein (F/P) molar ratios" (Thermo Fischer Scientific, Waltham, USA).

### 1.18 Fab fragment preparation

Fab fragments were prepared from conjugated antibodies using the Pierce Fab Preparation Kit (Thermo Fischer Scientific, Waltham, USA). Successful conjugation and purification of the conjugates was verified by reducing or non-reducing SDS-PAGE using red or NIR fluorescence-based imaging (IVIS Spectrum In Vivo Imaging System, PerkinElmer, Waltham, USA) and Coomassie staining (Protein Ark, Sheffield, UK). Protein concentration was determined by using the Pierce BCA Protein Assay Kit (Thermo Fischer Scientific, Waltham, USA). The degree of labeling was calculated according to the TechTip#31 protocol "Calculate dye:protein (F/P) molar ratios" (Thermo Fischer Scientific, Waltham, USA).

### 1.19 Flow cytometry

Specific binding of the produced antibodies or conjugates was tested on cells expressing the respective surface antigen. Mean fluorescence values of stained cells were determined with a FACS Calibur Flow Cytometer and the CellQuestPro software (BD Biosciences, Heidelberg, Germany). Binding affinity of the antibodies was calculated with help of the GraphPad Prism 7 software (GraphPad Software Inc., San Diego, USA) and K_{D} values were defined as the antibody or conjugate concentrations leading to half-maximal specific binding.

### 1.20 PIT

2.5*10⁵ cells were seeded per well in 35 mm cell culture dishes (Thermo Fischer Scientific) and cultivated for 24 h in the incubator (37°C, 5% CO₂). On the next day, cell culture medium was removed and fresh medium containing the appropriate treatment (medium, 10 µg/ml antibody, 10 µg/ml conjugate or the equimolar amount of free dye) was administered to the cells. After incubation (37°C, 5% CO₂, 24h), the cells were washed with 1x PBS and irradiated with a red light-emitting diode (LED), which emits light at λₘₐₓ = 680 nm (L690-66-60, Marubeni, Tokyo, Japan) or a LED, which emits light at (λₘₐₓ = 780 nm) (L780-66-60-550) at various mean irradiation doses. Power densities were quantified with an optical power meter (PM 100, Thorlabs, Newton, USA). After 24h, cell viability was analyzed using the vital dye erythrosine b (Logos Biosystems, Gyeonggi-do, South Korea) and the Neubauer Counting Chamber. Significance of the data was calculated with the Student's t test (unpaired, parametric with Welch's correlation) using the GraphPad Prism 7 software (GraphPad Software Inc., San Diego, USA).

In accordance with the above described preparatory examples the person skilled in the art can provide variants of the compounds according to the present invention in analogous manner.

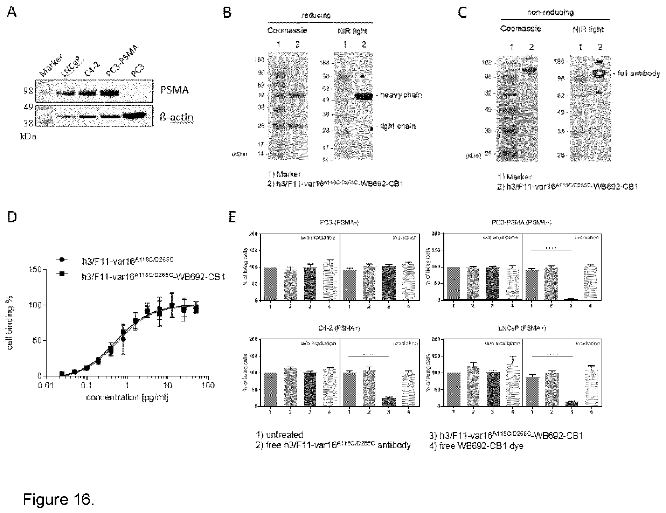

The results of the experiments 1 to 6 are shown in Figures 16-24 whereby the figure legends are given below.

### Experiment 1

### PIT of prostate cancer cells with the humanized anti-PSMA antibody h3/F11var16^{A118C/}^{D265C} linked to the dye WB692-CB1

PSMA expression was confirmed by Western Blotting in the prostate cancer cell lines LNCaP, C4-2, and PC3-PSMA, whereas PC3 cells were shown to be negative (Figure 16A).

The dye WB692-CB1 was conjugated to a humanized version of the anti-PSMA mAb 3/F11 (3/F11) containing two engineered cysteines on positions 118 (A118C) and 265 (D265C). The antibody was named h3/F11-var16^{A118C/D265C}. The antibody-dye conjugate was named h3/F11-var16^{A118C/D265C}-WB692-CB1. SDS gel under reducing conditions confirmed the conjugated dye on the heavy chain (ca. 50 kDa) of the antibody under red light (Figure 16B). SDS gel under non-reducing conditions confirmed that the antibody was still intact after dye conjugation as an about 150 kDa molecule consisting of two heavy and two light chains. This proved that reduction of the engineered cysteines during dye conjugation does not lead to marked reduction of intrachain disulfide bonds as required for the conjugation of dyes to cysteines naturally occurring in the antibody molecule (Figure 16C).

Flow cytometry demonstrated that there was no significant difference in cell binding of the antibody-dye conjugate h3/F11-var16^{A118C/D265C}-WB692-CB1 (K_{D} = 0.5 µg/ml) in comparison to the free antibody h3/F11-var16^{A118C/D265C} (K_{D} = 0.57 µg/ml) (Figure 16D). This confirmed that conjugation of the dye to the cysteines on positions 118 and 265 of the antibody did not impact the antigen binding.

PIT using h3/F11-var16^{A118C/D265C}-WB692-CB1 was tested on PSMA-positive prostate cancer cells of the lines PC3-PSMA, C4-2 and LNCaP. The PSMA-negative cell line PC3 was used as control. PIT using h3/F11-var16^{A118C/D265C}-WB692-CB1 led to death of 96.8 % PC3-PSMA cells, 74.7% (C4-2) and 85.6 % (LNCaP) of PSMA-positive cells after irradiation with 32 J/cm². PSMA-negative PC3 cells remained unaffected (Figure 16E).

Surprisingly, irradiated cells incubated with equimolar concentrations of the free WB692-CB1 dye remained alive (Figure 16E). This means that the dye must be administered with help of a cell-binding molecule to be cytotoxic after red light irradiation. The free dye, on the other hand, is not cytotoxic in therapeutic concentrations, which contributes to the increased safety of our therapeutic approach.

Cells, which were treated with h3/F11-var16^{A118C/D265C}-WB692-CB1, but were not irradiated, also survived. This underlines the high safety of our therapy that cells are only affected, which have bound the antibody dye conjugate and have been irradiated with red light.

### Experiment 2

### PIT of prostate cancer cells with the humanized anti-PSMA antibody h3/F11.19^{T120C/}^{D265C} linked to the dye WB692-CB1

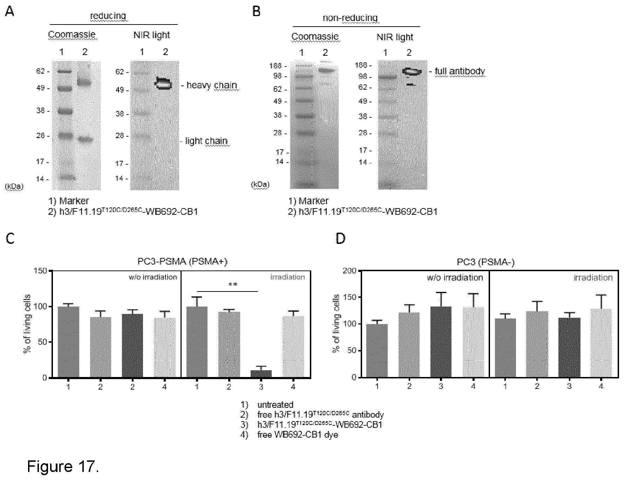

The dye WB692-CB1 was conjugated to another humanized version of the anti-PSMA mAb 3/F11 with two engineered cysteines on positions 120 (T120C) and 265 (D265C), called h3/F11.19^{T120C/D265C}. The antibody-dye conjugate was named h3/F11.19^{T120C/D265C}-WB692-CB1. SDS gel under reducing conditions confirmed the conjugated dye on the heavy chain (ca. 50 kDa) of the antibody under NIR light (Figure 17A). SDS gel under non-reducing conditions showed that the antibody was still intact after dye conjugation as an about 150 kDa molecule consisting of two heavy and two light chains (Figure 17B).

PIT using h3/F11.19^{T120C/D265C}-WB692-CB1 was tested on PSMA-positive prostate cancer cells of the line PC3-PSMA. The PSMA-negative cell line PC3 was used as control. PIT caused death of 89.9 % PC3-PSMA cells (Figure 17C), PSMA-negative PC3 cells remained unaffected by treatment (Figure 17D).

### Experiment 3

### PIT of prostate cancer cells with the humanized anti-PSMA Fab fragments Fab-var16^{A118C} linked to the dye WB692-CB1

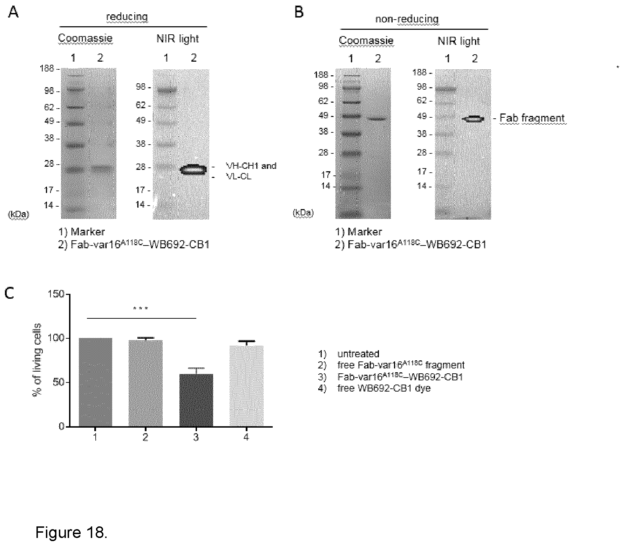

Fab fragments were generated by digest of the antibody h3/F11-var16^{A118C/D265C} with papain. The Fab fragments, containing the cysteine mutation on position 118, were called Fab-var16^{A118C}. The dye WB692-CB1 was successfully conjugated to the Fab (Figure 18A, B) and revealed a specific killing of PSMA-positive LNCaP prostate cancer cells up to 59.9 % by PIT (Figure 18C).

### Experiment 4

### PIT of prostate cancer cells with the murine anti-PSMA antibody 3/F11 linked to the dye WB692-LB1

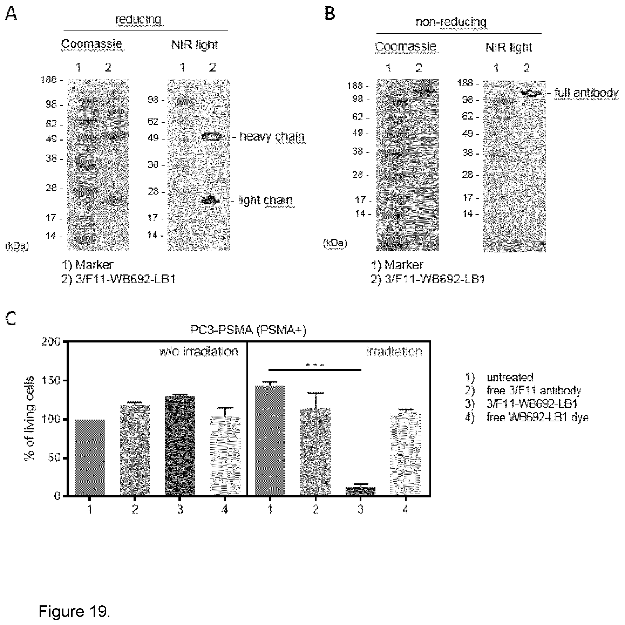

The dye WB692-LB1 was conjugated to the murine anti-PSMA mAb 3/F11. The antibody-dye conjugate was named 3/F11-WB692-LB1. SDS gel under reducing conditions confirmed the conjugated dye on the heavy chain (ca. 50 kDa) of the antibody under red light (Figure 19A). SDS gel under non-reducing conditions showed that the antibody was still intact after dye conjugation as an about 150 kDa molecule consisting of two heavy and two light chains (Figure 19B).

PIT using 3/F11-WB692-LB1 was tested on PSMA-positive PC3-PSMA prostate cancer cells4. The conjugate caused death of 87.6 % PC3-PSMA cells after irradiation, whereas cells remained alive without irradiation (Figure 19C).

### Experiment 5

### PIT of breast cancer cells with an anti-Her2 antibody and the dye WB692-CB1

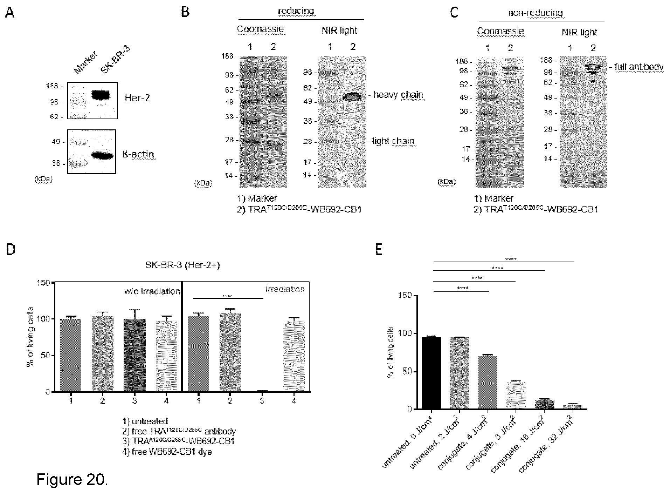

Her-2 expression of SK-BR-3 breast cancer cells was confirmed by Western Blotting (Figure 20A). The dye WB692-CB1 was conjugated to an anti-HER2 antibody variant, derived from Trastuzumab (DBAN: DB00072) containing two cysteine mutations on positions 120 (T120C) and 265 (D265C). The antibody variant was named TRA^{T120C/D265C} and the antibody-dye conjugate was named TRA^{T120C/D265C}-WB692-CB1. SDS gel under red light confirmed the conjugation of the dye on the heavy chain of the antibody (Figure 20B, C). Incubation of Her-2 expressing SK-BR-3 breast cancer cells with the conjugate followed by red light irradiation led to specific death of 98,9 % of the cells (Figure 20D). Treatment of SK-BR-3 cells with TRA^{T120C/D265C}-WB692-CB1 and irradiation with increasing doses of red light induced increasing percentages of cell death. This proved that the cytotoxicity of the conjugate is dependent on red light irradiation dose.

### Experiment 6

### PIT of bladder cancer cells with an anti-EGFR antibody and the dye WB692-CB1

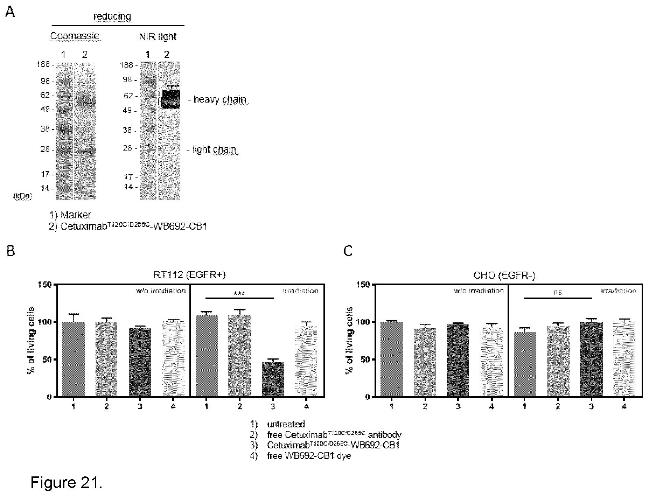

The dye WB692-CB1 was conjugated to an anti-EGFR antibody variant, derived from Cetuximab (DBAN: DB00002) containing two cysteine mutations on positions 120 (T120C) and 265 (D265C). The antibody variant was named Cmb^{T120C/D265C} and the antibody-dye conjugate was named Cetuximab^{T120C/D265C}-WB692-CB1. SDS gel under reducing conditions confirmed the conjugated dye on the heavy chain (ca. 50 kDa) of the antibody under red light (Figure 21A). PIT using Cmb^{T120C/D265C} -WB692-CB1 was tested on EGFR-positive bladder cancer cells of the line RT112. The conjugate caused death of 53 % RT112 cells after irradiation with red light, whereas cells remained alive without irradiation (Figure 21B). EGFR-negative CHO cells remained unaffected by treatment (Figure 21C).

### Experiment 7

### PIT of prostate cancer cells with the humanized anti-PSMA antibody h3/F11.19 and the dye WB795-CB1

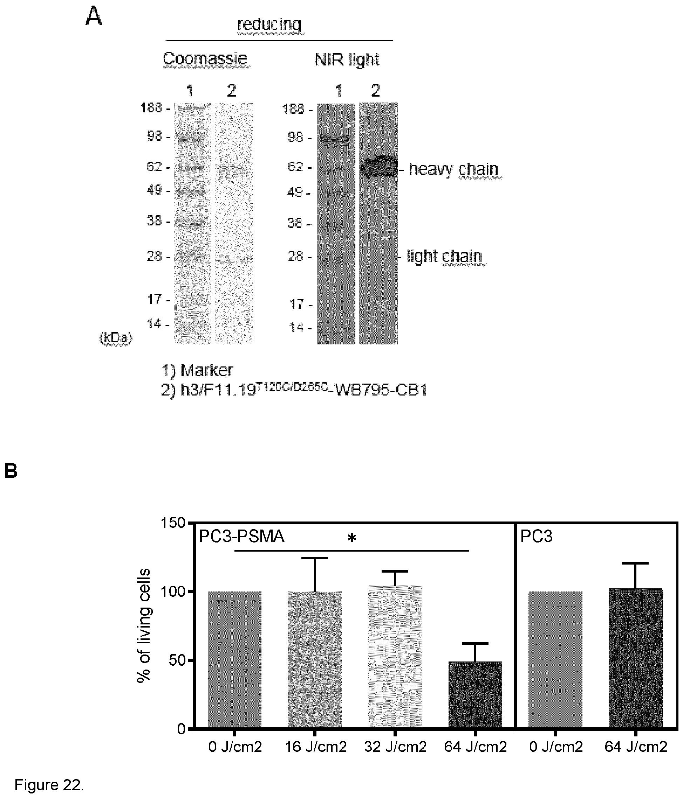

The dye WB795-CB1 was conjugated to the humanized version of the anti-PSMA mAb 3/F11 with two engineered cysteines on positions 120 (T120C) and 265 (D265C), called h3/F11.19^{T120C/D265C}. The antibody-dye conjugate was named h3/F11.19T^{120C/D265C}-WB795-CB1. SDS gel under reducing conditions confirmed the conjugated dye on the heavy chain (ca. 50 kDa) of the antibody under NIR light (Figure 22A). PIT with h3/F11.19T^{120C/D265C}-WB795-CB1 specifically induced death in PC3-PSMA cells after irradiation with NIR light (Figure 22B)

### Experiment 8

### PIT of prostate cancer cells with the humanized anti-PSMA antibody h3/F11.19 and the dye WB692-CB2

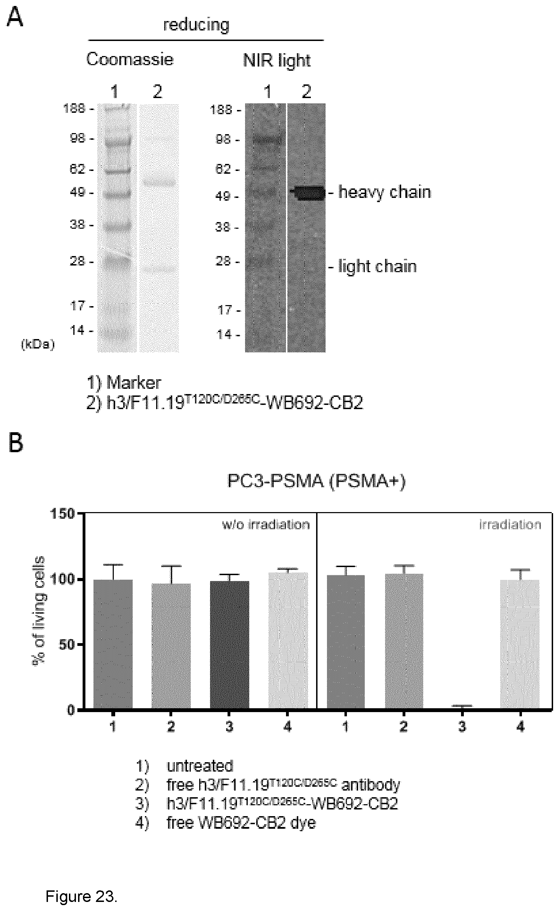

The dye WB692-CB2 was conjugated to the humanized version of the anti-PSMA mAb 3/F11 with two engineered cysteines on positions 120 (T120C) and 265 (D265C), called h3/F11.19^{T120C/D265C}. The antibody-dye conjugate was named h3/F11.19^{T120C/D265C}-WB692-CB2. SDS gel under reducing conditions confirmed the conjugated dye on the heavy chain (ca. 50 kDa) of the antibody under red light (Figure 23A). PIT using h3/F11.19^{T120C/D265C}-WB692-CB2 was tested on PSMA-positive prostate cancer cells of the line PC3-PSMA. PIT caused death of the PC3-PSMA cells (Figure 23B).

### Experiment 9

### PIT of colon cancer cells with the chimeric anti-CD 133 antibody chAC133.1 and the dye WB692-CB1

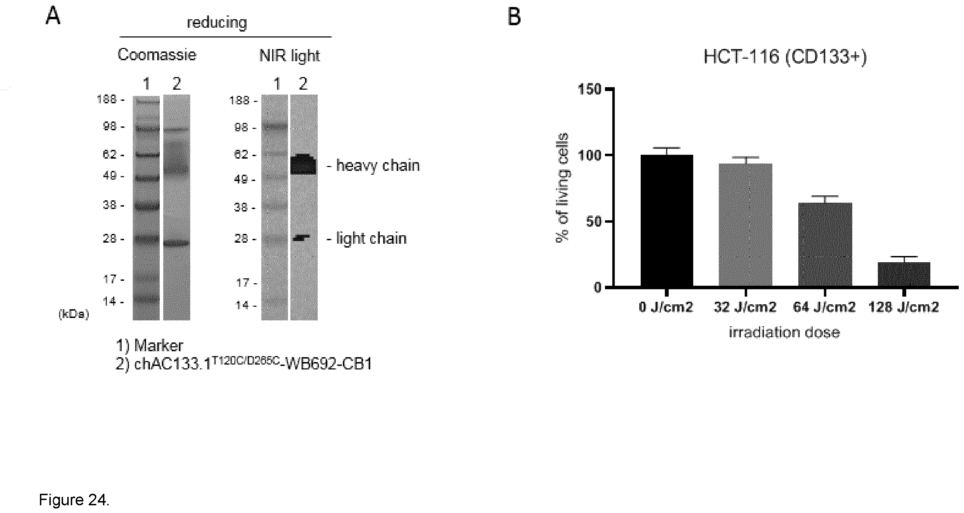

The dye WB692-CB1 was conjugated to the chimeric anti-CD133 antibody chAC133.1 with two engineered cysteines on positions 120 (T120C) and 265 (D265C), called chAC133.1^{T120C/D265C}. The antibody-dye conjugate was named chAC133.1^{T120C/D265C} -WB692-CB1. SDS gel under reducing conditions confirmed the conjugated dye on the heavy chain (ca. 50 kDa) of the antibody under red light (Figure 24A). PIT using chAC133.1^{T120C/D265C} - WB692-CB1was tested on CD133-positive colon cancer cells of the line HCT-116. PIT caused death the colon cancer cells depending on red light irradiation dose (Figure 24B).

### Brief description of the Figures

Figure 1 shows representative particularly preferred embodiments of the silicon phthalocyanines **B** and their nomenclature.
Figure 2 shows representative particularly preferred embodiments of the silicon phthalocyanines **C** and their nomenclature.
Figure 3 shows the first part of the synthesis of the multiply used building blocks **13** and **14,** namely the preparation of the building blocks **8** and **9.**
Figure 4 shows the accomplishment of the multiply used building block **13** in the course of the second part of the synthesis of the multiply used building block **14.** The building block **13** precedes the fully elaborated silicon phthalocyanines **EMBODIMENT 1 (= WB692-LB1), EMBODIMENT 2 (= WB692-LB2), EMBODIMENT 3 (= WB692-CB1),** and **EMBODIMENT 4 (= WB692-CB2B).**
Figure 5 shows the accomplishment of the multiply used building block **14.** It precedes the fully elaborated silicon phthalocyanine **EMBODIMENT 1 (= WB692-LB1), EMBODIMENT 2 (= WB692-LB2),** and **EMBODIMENT 3 (= WB692-CB1).**
Figure 6 shows the fourth part and thereby the end of the synthesis of the fully elaborated AsB-type silicon phthalocyanine **EMBODIMENT 1 = WB692-LB1.**
Figure 7 shows the conversion of the silicon phthalocyanine intermediate **14** (obtained via **13** from **11** as shown in Figures 4 and 5) but carried on without purification) into the fully elaborated AsB-type silicon phthalocyanine **EMBODIMENT 2 = WB692-LB2.**
Figure 8 shows the conversion of the silicon phthalocyanine intermediate **14** into the fully elaborated AsB-type silicon phthalocyanine **EMBODIMENT 3 = WB692-CB1.**
Figure 9 shows the conversion of the silicon phthalocyanine intermediate **13** into the fully elaborated AsB-type silicon phthalocyanine **EMBODIMENT 4 = WB692-CB2.**
Figure 10 shows the synthesis of the common precursor **31** of the fully elaborated A₃B-type silicon phthalocyanines **EMBODIMENT 5 = WB795-LB1** and **EMBODIMENT 6 = WB795-CB1.**
Figure 11 shows the synthesis of the fully elaborated A₃B-type silicon phthalocyanine **EMBODIMENT 5 = WB795-LB1** from the silicon phthalocyanine intermediate **31.**
Figure 12 shows the synthesis of the fully elaborated A₃B-type silicon phthalocyanine **EMBODIMENT 6 = WB795-CB1** from the silicon phthalocyanine intermediate **31.**

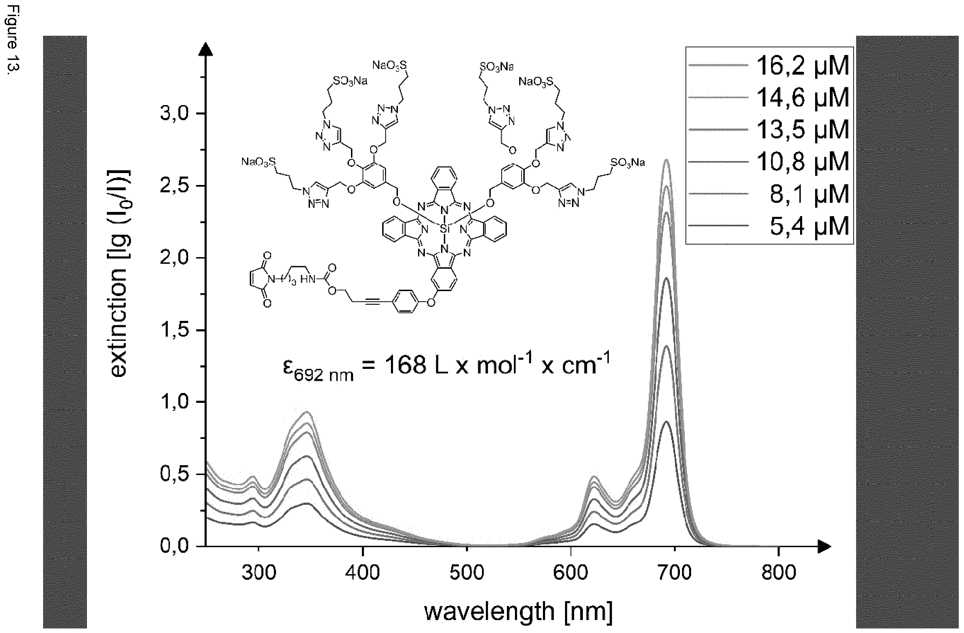

Figure 13 shows the UV/VIS spectrum of the fully elaborated silicon phthalocyanine **WB692-CB1 (= EMBODIMENT 3;** 5 - 16 µM in H₂O; λₘₐₓ = 692 nm; pathlength = 1 cm).

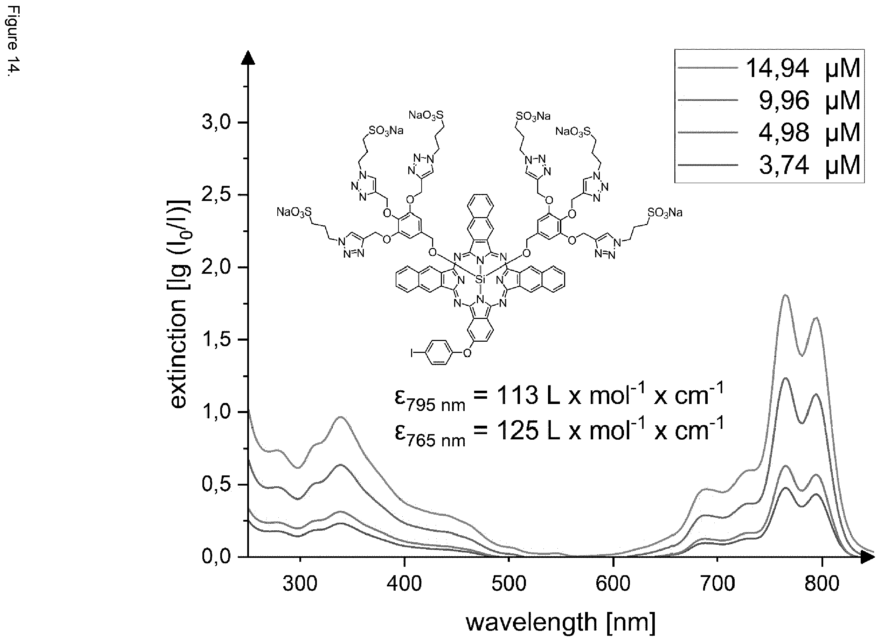

Figure 14 shows the UV/VIS spectrum of the precursor **31** (formula: Figure 10) of the fully elaborated silicon phthalocyanine **WB795-CB1 (= EMBODIMENT 6;** 4 - 15 µM in H₂O; λₘₐₓ = 795 nm, λ_{almost max} = 765 nm; pathlength = 1 cm).
Figure 15 shows that the A₄-type silicon phthalocyanines **36** vs. **37** (both non-bionconjugable and studied by H. Takakura, S. Matsuhiro, M. Kobayashi, Y. Goto, M. Harada, T. Taketsugu, M. Ogawa, J. Photochem. Photobiol. 2022, 426, 113749) differ structurally from one another *similarly* as the AsB-type silicon phthalocyanines **Li-Cor-A** from US Patent 7,005,518 differs from, e. g., compound **WB692-LB1** from this invention (all bioconjugable): In **36** (judged less promising) and in **Li-Cor-A** the octahedrally coordinated silicon atom binds to two O-Si motifs, while in **37** (judged more promising) and, e. g., compound **WB692-LB1** from this invention the octahedrally coordinated silicon atom binds to two O-C motifs.
Figure 16. (A) Western Blot of PSMA expression in prostate cancer cells, β-actin was blotted as loading control. (B) SDS PAGE of the antibody-dye conjugate h3/F11-var16^{A118C/D265C}-WB692-CB1 under reducing conditions and (C) under non-reducing conditions after Coomassie staining and under redlight. (D) Cell binding of h3/F11-var16^{A118C/D265C}-WB692-CB1 to LNCaP cells in comparison to the un-coupled antibody h3/F11^{A118C/D265C}. (E) Viability of prostate cancer cells incubated with h3/F11-var16^{A118C/D265C}-WB692-CB1 or equimolar concentrations of free antibody or free dye 24 h after irradiation with a mean dose of 32 J/cm² red light. ****p<0.0001. The cytotoxicity of the dye on PSMA⁺ cells after irradiation is extremely strong.
Figure 17. (A) SDS PAGE of the antibody dye conjugate h3/F11.19^{T120/D265C}-WB692-CB1 under reducing conditions and (B) under non-reducing conditions after Coomassie staining and under red light. Viability of (C) PSMA-positive PC3-PSMA or (D) PSMA-negative PC3 cells incubated with h3/F11.19^{T120/D265C}-WB692-CB1 or equimolar concentrations of free antibody or free dye 24 h after irradiation with a mean dose of 32 J/cm² red light. ***p<0.001. A comparison of Figure 17 (C) and (D) shows the surprising activity of the construct according to the invention.
Figure 18. (A) SDS PAGE of the Fab-dye conjugate Fab-var16^{A118C}-WB692-CB1 under reducing conditions and (B) under non-reducing conditions after Coomassie staining and under NIR light. (C) Viability of LNCaP prostate cancer cells incubated with Fab-var16^{A118C}-WB692-CB1 24 h after irradiation with a mean dose of 32 J/cm² red light. Controls were left untreated or incubated with Fab fragments alone or the dye alone.***p<0.001.
Figure 19. (A) SDS PAGE of the antibody-dye conjugate 3/F11-WB692-LB1 under reducing conditions and (B) under non-reducing conditions after Coomassie staining and under NIR light. (C) Viability of PC3-PSMA prostate cancer cells incubated with 3/F11-WB692-LB1 24 h after irradiation with a mean dose of 32 J/cm² red light. Controls were left untreated or incubated with free 3/F11 antibody or free WB-692-LB1dye.***p<0.001. Again Figure 19 (C) shows the specific activity of the construct according to the invention after irradiation.
Figure 20. (A) Western Blot of Her-2 expression in SK-BR-3 breast cancer cells, β-actin was blotted as loading control. (B) SDS PAGE of the antibody-dye conjugate TRA^{T120C/D265C}-WB692-CB1 under reducing conditions and (C) under non-reducing conditions after Coomassie staining and under red light. (D) Viability of SK-BR-3 cells incubated with TRA^{T120C/D265C}-WB692-CB1 or equimolar concentrations of free antibody or free dye 24 h after irradiation with a mean dose of 32 J/cm² red light. (E) Viability of SK-BR-3 cells 24 h after treatment with the conjugate TRA^{T120C/D265C}-WB692-CB1 and irradiation with different red light idoses. ****p<0.0001. Figure 20 demonstrates that the invention can be successfully performed with another tumor antigen, namely Her-2.
Figure 21. (A) SDS PAGE of the antibody dye conjugate Cetuximab^{T120C/D265C}-WB692-CB1 under reducing conditions after Coomassie staining and under NIR light. (B) Viability of EGFR-expressing RT112 bladder cancer cells incubated with Cetuximab ^{T120C/D265C}-WB692-CB1 24 h after irradiation with a mean dose of 32 J/cm² red light. (C) Viability of EGFR-negative CHO cells incubated with Cextuximab^{T120C/D265C}-WB692-CB1 24 h after irradiation with 32 J/cm² red light. Controls were left untreated or incubated with free antibody or free WB692-CB1dye.***p<0.001.
Figure 22.(A) SDS PAGE of the antibody dye-conjugate h3/F11.19^{T120C/D265C}-WB795-CB1 under reducing conditions after Coomassie staining and under NIR light. (B) PIT of PC3-PSMA cells after incubation with h3/F11.19^{T120C/D265C}-WB795-CB1 and irradiation with different NIR light doses. PSMA-negative PC3 cells served as control. *p<0.05.
Figure 23. (A) SDS PAGE of the antibody-dye conjugate h3/F11.19^{T120C/D265C}-WB692-CB2 under reducing conditions after Coomassie staining and under red light. (B) Viability of PC3-PSMA prostate cancer cells incubated with h3/F11.19^{T120C/D265C}-WB692-CB2 24 h after irradiation with a mean irradiation dose of 32 J/cm² red light. Controls were left untreated or incubated with free antibody or free dye.
Figure 24. (A) SDS PAGE of the antibody-dye conjugate chAC133.1^{T120C/D265C}-WB692-CB1 under reducing conditions after Coomassie staining and under red light. (B) Viability of HCT-116 colon cancer cells incubated with chAC133.1^{T120C/D265C}-WB692-CB1 24 h after irradiation with increasing irradiation doses of red light.

## Claims

1. Silicon phthalocyanine according to 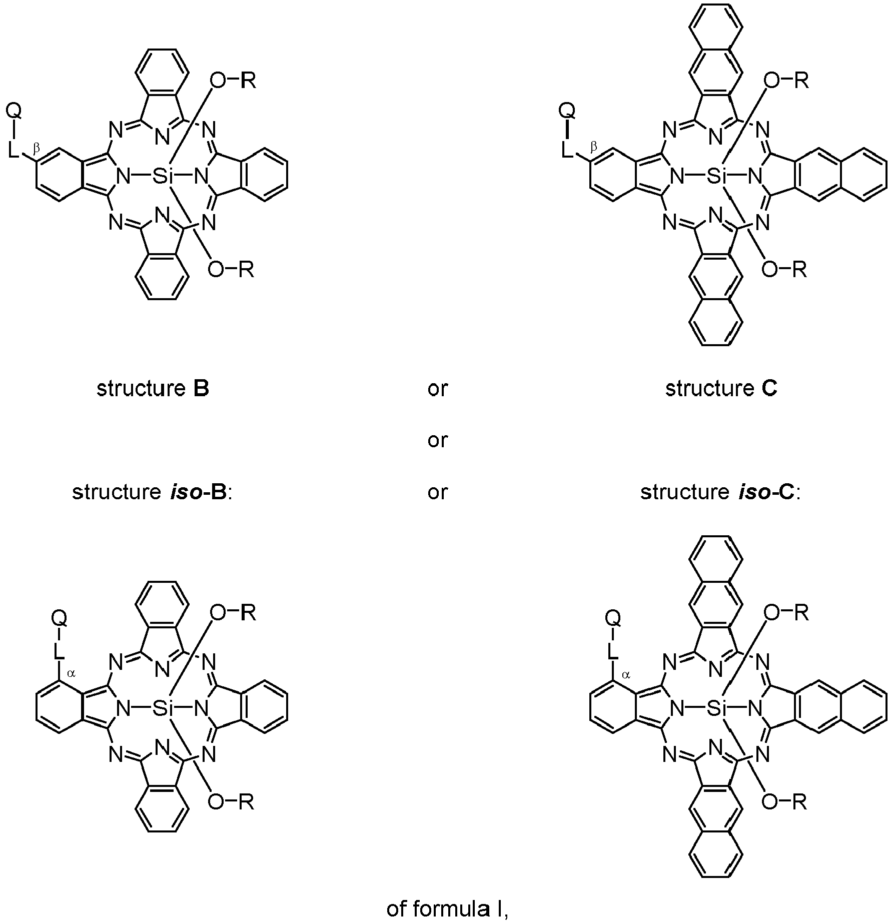 wherein:
-O-R is -O(-R¹)ₚ(-O)_{q}-R²(-R³)ₛ
R¹ is selected from C₁-C₃ alkyl and C(O),
p is 0 or 1,
q is 0 or 1, with the proviso that if p is 0, then q is 0,
R² is selected from alkyl, aryl, arylalkyl, (heteroaryl)alkyl, alkoxyalkyl, and heteroaryl,
R³ is -A¹-A²,
A¹ is selected from (heterocyclyl)alkyl-(O)ᵣ, dihydroheteroaryl)alkyl-(O)ᵣ,
r is 0 or 1
A² is selected from di-, tri- and tetrasaccharides and -C₁-C₆ alkyl, which -C₁-C₆ alkyl is substituted with at least one group selected from sulfonic acid, phosphonic acid, carboxylic acid, and salts thereof,
and
s is 1, 2 or 3,
L is a linker unit -O-L¹-L² being bound via the oxygen either to C^{β} of structures **B** and **C** to form an ether motif C^{β}-O-L¹-L² or to C^{α} of structures ***iso*-B** and ***iso*-C** to form an ether motif C^{α}-O-L¹-L²,))
L¹ is selected from aryl, arylaryl, (arylalkyl)aryl, (arylalkoxy)aryl, [(arylalkyl)amino]aryl, aroylaryl, (arylsulfanyl)aryl, and arylsulfonylaryl
L² is selected from ureido(C₁-C₆ alkyl), ureido(C₂-C₆ alkenyl), ureido(C₂-C₆ alkynyl), carbamoyloxy(C₁-C₆ alkyl), carbamoyloxy(C₂-C₆ alkenyl), carbamoyloxy(C₂-C₆ alkynyl), (alkoxycarbonyl)amino(C₁-C₆ alkyl), (alkoxycarbonyl)amino(C₂-C₆ alkenyl), (alkoxycarbonyl)amino(C₂-C₆ alkynyl), [ureido(C₁-C₆ alkyl)]amino, [carbamoyloxy(C₁-C₆ alkyl)]amino, [(alkoxycarbonyl)amino(C₁-C₆ alkyl)]amino, ureido(C₁-C₆ alkoxy), carbamoyloxy(C₁-C₆ alkoxy), (alkoxycarbonyl)amino(C₁-C₆ alkoxy), [ureido(C₁-C₆ alkyl)]thio, [carbamoyloxy(C₁-C₆ alkyl)]thio, [(alkoxycarbonyl)amino(C₁-C₆ alkyl)]thio, {[ureido(C₁-C₆ alkyl)]amino}carbonyl, {[carbamoyloxy(C₁-C₆ alkyl)]amino}carbonyl, {[(alkoxycarbonyl)amino(C₁-C₆ alkyl)]amino}carbonyl, [ureido(C₁-C₆ alkoxy)]carbonyl, [carbamoyloxy(C₁-C₆ alkoxy)]carbonyl, [(alkoxycarbonyl)amino(C₁-C₆ alkoxy)]carbonyl, [ureido(C₁-C₆ alkyl)]carbonyl, [ureido(C₂-C₆ alkenyl)]carbonyl, [ureido(C₂-C₆ alkynyl)]carbonyl, [carbamoyloxy(C₁-C₆ alkyl)]carbonyl, [carbamoyloxy(C₂-C₆ alkenyl)]carbonyl, [carbamoyloxy(C₂-C₆ alkynyl)]carbonyl, [(alkoxycarbonyl)amino(C₁-C₆ alkyl)]carbonyl, [(alkoxycarbonyl)amino(C₂-C₆ alkenyl)]carbonyl, and [(alkoxycarbonyl)amino(C₂-C₆ alkynyl)]carbonyl
Q is -Q¹(-Q²)ₜ-Q³
Q¹ is a linear or branched, preferably linear, C₁-C₆ alkyl group,
Q² is selected from aryl and alkylaryl,
t is 0 or 1, and
Q³ is selected from the group consisting of
a) a carboxylic acid, a corresponding acyl halide, a corresponding mixed anhydride, preferably aza-analogs resulting from additions to carbodiimides or condensations with uronium salts, a corresponding activated ester,
b) an electrophilic C,C double bonds, preferably α,β-unsaturated esters, α,β-unsaturated lactones (including α-alkylidene lactones), α,β-unsaturated amides, α,β-unsaturated imides (including halogenated ones), α,β-unsaturated nitriles, α,β-unsaturated ketones, α,β-unsaturated sulfones, α,β-unsaturated sulfoxides, alk-1-enylphosphonates, nitroolefins,
c) an alkyl halide, preferably α-haloketones and (haloacet)amides, an alkyl sulfonate,
d) an isocyanate, isothiocyanate,
e) a disulfide, a thiosulfonic acid ester, a trisulfide-*S*,*S*-dioxide,
f) a phosphoramidite and
g) an *N*-aryl-1,2,4-triazolin-3,5-dione.

2. Silicon phthalocyanine according to claim 1, wherein the linker L has a structure selected from the group consisting of any one of

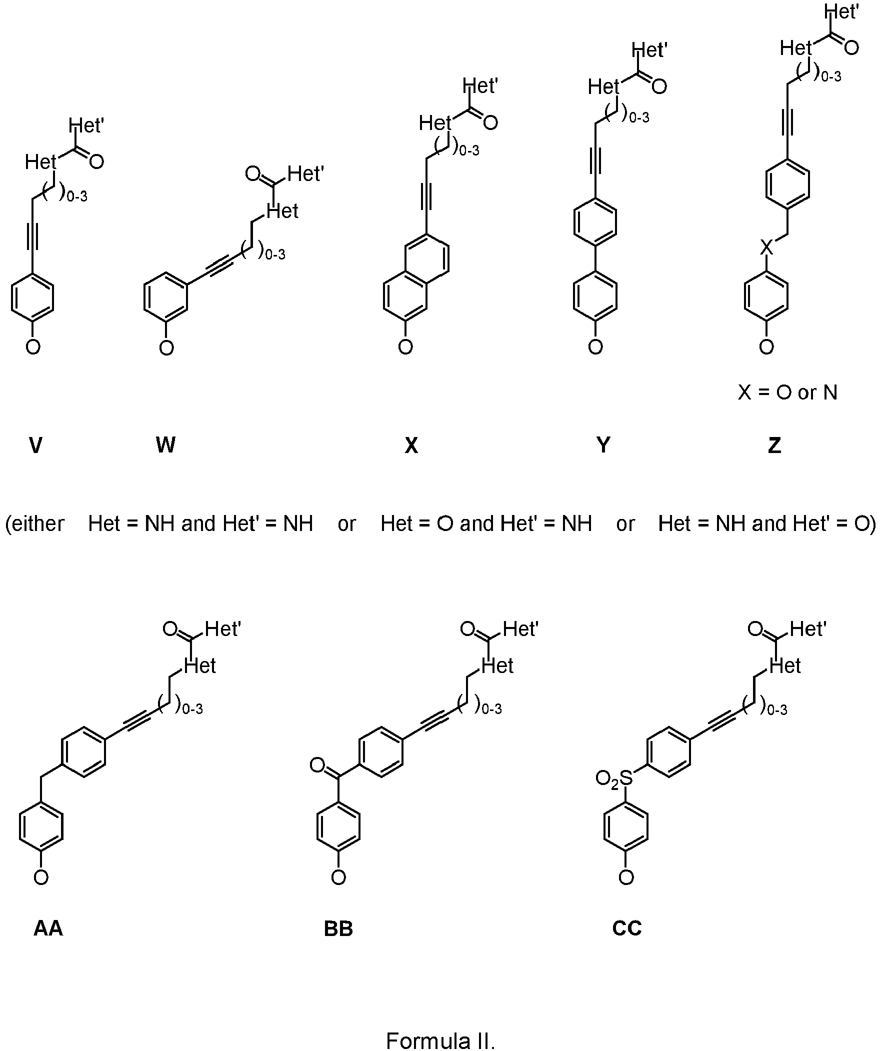

3. Silicon phthalocyanine according to any of claims 1 or 2, **characterized in that** Q has a structure selected from the group consisting of any one of

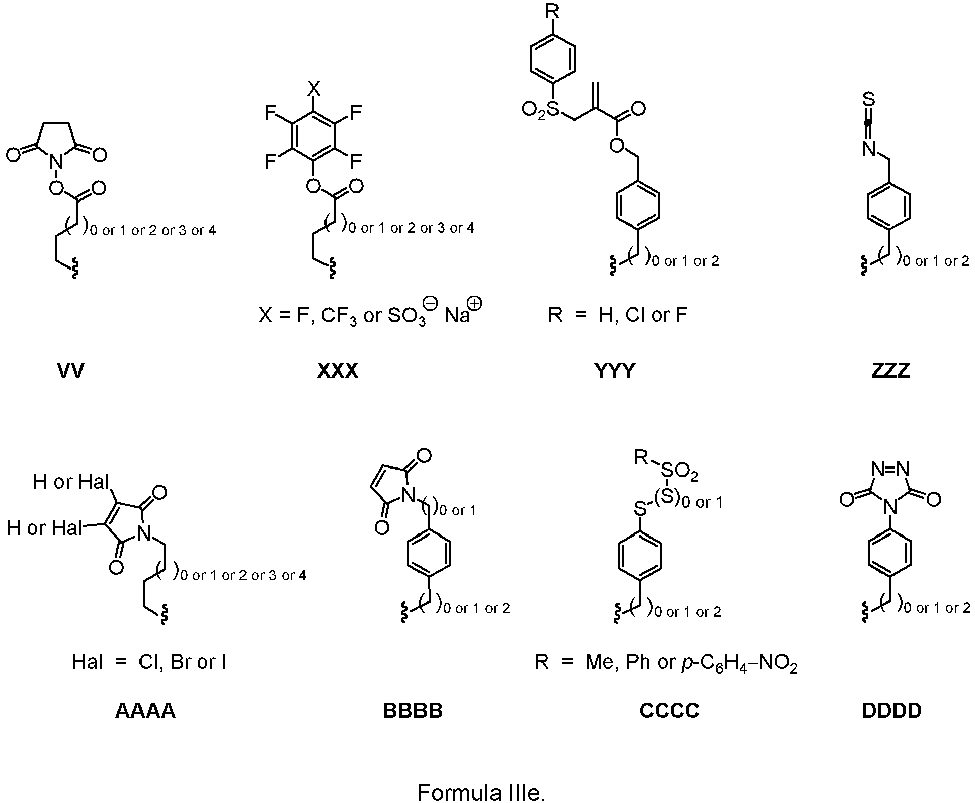

4. Silicon phthalocyanine according to any of claims 1-3, wherein the residue R of the OR groups has a structure as shown in

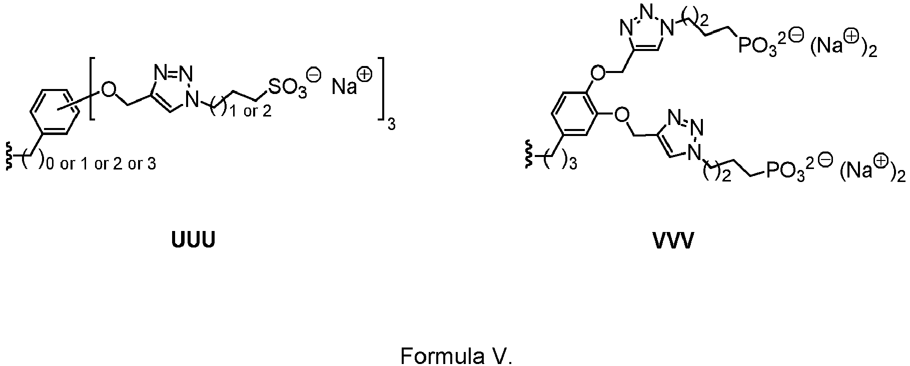

5. Silicon phthalocyanine according to any of claims 1 to 4 **characterized in that** Q³ is a reactive or an activable functional group which is configured to form a covalent bond to a molecule capable of target-directed binding, wherein said molecule is selected from the group consisting of, but not limited to, antibodies, antibody fragments, nanobodies, antigens, ligands, receptors, enzymes, proteins, peptides, small molecules, hormones, inhibitors, aptamers, cells, phages, viruses, DNA, RNA, lipids, carbohydrates, polysaccharides, nanomaterials or chemical drugs.

6. Silicon phthalocyanine according to any of claims 1-5, wherein the residue Q³ is covalently bound to a molecule capable of target-directed binding, wherein said molecule is selected from the group consisting of, but not limited to, antibodies, antibody fragments, nanobodies, antigens, ligands, receptors, enzymes, proteins, peptides, small molecules, hormones, inhibitors, aptamers, cells, phages, viruses, DNA, RNA, lipids, carbohydrates, polysaccharides, nanomaterials or chemical drugs.

7. Silicon phthalocyanine according to claim 5 or 6, wherein said molecule binds to a target structure which is associated with a disease.

8. Silicon phthalocyanine according to any of claims 5-7, **characterized in that** the target structure is selected from the group of, but not limited to tumor associated antigens.

9. Silicon phthalocyanine according to any of claims 1 to 8 for use in therapy of tumor associated diseases.

10. Silicon phthalocyanine according to any of claims 1 to 8 for use in theranostics whereby the dye is activated by a light source in the red / near infrared wavelength for diagnosis and therapy.

11. Silicon phthalocyanine according to any of claims 1 to 8 for use in ex vivo diagnosis.

12. Silicon phthalocyanine according to any of claims 1-8 for use in a dual-labeled molecule, where the first label is the silicon phthalocyanine and the second label is a radioactive tracer for pre-, intra- and post-operative diagnosis and therapy

13. Silicon phthalocyanine according to any of claims 1 to 8 for use in combination with drugs that further enhance tumor-cell-selective systemic host-immunity

14. Use of a compound of formula HO-L¹-Hal in the preparation of a silicon phthalocyanine, wherein L¹ is defined as in claim 1 and Hal is a halogen selected from I, Br, and Cl.

15. Use according to claim 14, wherein the silicon phthalocyanine is according to any of claims 1 to 8 and the compound of formula HO-L¹-Hal is selected from
